# EUROPEAN PATENT APPLICATION

(11) **EP 4 775 569 A1**
(43) Date of publication of application: **15.07.2026**
(21) Application number: 24862001.5
(22) Date of filing: 04.09.2024
(51) Int. Cl.: C07D 401/04, C07D 403/04, A61K 31/513, A61K 31/506, A61P 31/12, A61P 31/14

(54) **3CL PROTEASE INHIBITOR FOR TREATING OR PREVENTING CORONAVIRUS INFECTIONS AND USE THEREOF**

(30) Priority: 08.09.2023 CN 202311156881
(71) Applicant: The Global Health Drug Discovery Institute, Beijing 100192 (CN)
(72) Inventor: LIU, Renhe, Beijing 100192 (CN); HU, Qiping, Beijing 100192 (CN); YAN, Ning, Beijing 100192 (CN); HU, Song, Beijing 100192 (CN); XIA, Yan, Beijing 100192 (CN); DING, Sheng, Beijing 100192 (CN)
(74) Representative: Blumbach · Zinngrebe Patentanwälte PartG mbB
(86) International application number: PCT/CN2024/116911
(87) International publication number: WO 2025/051160

(57) **Abstract**

The present application relates to a 3CL protease inhibitor for treating or preventing coronavirus infections and the use thereof. Specifically, the present application relates to a compound of formula (I) or an isotope-labeled compound thereof, or an optical isomer, geometric isomer, tautomer, or isomer mixture thereof, or a pharmaceutically acceptable salt thereof, or a prodrug thereof, or a metabolite thereof, and the use thereof in the preparation of a drug for treating or preventing coronavirus infections or diseases or symptoms caused by coronavirus.

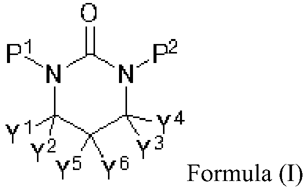

## Description

### CROSS-REFERENCE TO RELATED APPLICATIONS

The present application claims priority to Chinese Patent Application No. 202311156881.1, filed on September 08, 2023, which is incorporated herein by reference in its entirety.

### TECHNICAL FIELD

The present application relates to the field of biomedicine, and specifically, to a 3CL protease inhibitor for treating or preventing coronavirus infections and use thereof.

### BACKGROUND

Severe acute respiratory syndrome coronavirus 2 (SARS-CoV-2) is a positive-sense RNA virus with one of the largest genomes among the presently known RNA viruses. As revealed in studies, SARS-CoV-2 mainly invades cells through the human respiratory mucosal system. After entering cells, the viral gene products are cleaved by proteases to start translation and replication of essential proteins. 3CL proteases identify specific enzyme cleavage sites and cleave a polyprotein precursor into multiple non-structural proteins, and therefore, are vital to life cycles of viruses and are excellent targets for antiviral drugs. Many marketed antiviral drugs target 3CL proteases of viruses such as HIV and HCV. Lopinavir and ritonavir, inhibitors of HIV 3CL protease, specifically interact with a 3CL protease of SARS-CoV-2 in vitro, but have been proved to have no positive effects on patients with SARS-CoV-2 infections in clinical trials. Therefore, potent inhibitors specifically targeting the 3CL protease of SARS-CoV-2 are clinically needed.

### SUMMARY

In a first aspect, the present application provides a compound of formula (I) or an isotope-labeled compound thereof, or an optical isomer, geometric isomer, tautomer, or isomer mixture thereof, or a pharmaceutically acceptable salt thereof, or a prodrug thereof, or a metabolite thereof; 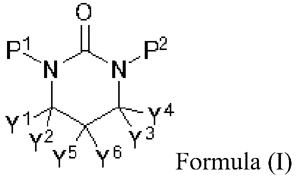 where:
Y¹ and Y² are each independently selected from hydrogen, halogen, a cyano group, an amino group, a C₁-C₆ alkylamino group, or a C₁-C₆ alkyl group optionally substituted with halogen or the cyano group; or Y¹ and Y² jointly form a carbonyl group; or Y¹ and Y², together with a carbon atom to which they are attached, form a C₃-C₆ cycloalkyl group or a 3- to 6-membered heterocycloalkyl group optionally substituted with halogen, a C₁-C₃ alkyl group, a C₁-C₃ haloalkyl group, a C₁-C₃ alkoxy group, a C₁-C₃ haloalkoxy group, the amino group, or a C₁-C₃ alkylamino group;
Y³ and Y⁴ are each independently selected from hydrogen, halogen, a cyano group, an amino group, a C₁-C₆ alkylamino group, or a C₁-C₆ alkyl group optionally substituted with halogen or the cyano group; or Y³ and Y⁴ jointly form a carbonyl group; or Y³ and Y⁴, together with a carbon atom to which they are attached, form a C₃-C₆ cycloalkyl group or a 3- to 6-membered heterocycloalkyl group optionally substituted with halogen, a C₁-C₃ alkyl group, a C₁-C₃ haloalkyl group, a C₁-C₃ alkoxy group, a C₁-C₃ haloalkoxy group, the amino group, or a C₁-C₃ alkylamino group;
Y⁵ and Y⁶ are each independently selected from hydrogen, halogen, a cyano group, an amino group, a C₁-C₆ alkylamino group, or a C₁-C₆ alkyl group optionally substituted with halogen or the cyano group; or Y⁵ and Y⁶, together with a carbon atom to which they are attached, form a C₃-C₆ cycloalkyl group or a 3- to 6-membered heterocycloalkyl group optionally substituted with halogen, a hydroxy group, a keto group, -OMs, a C₁-C₃ alkyl group, a C₁-C₃ haloalkyl group, a C₁-C₃ alkoxy group, a C₁-C₃ haloalkoxy group, the amino group, or a C₁-C₃ alkylamino group; and
optionally, one of Y⁵ and Y⁶, together with one of Y¹, Y², Y³, and Y⁴ and the carbon atom to which they are attached, form a C₃-C₆ cycloalkyl group or a phenyl group optionally substituted with halogen or a C₁-C₃ alkyl group;
further, P¹ is -L¹-Z¹-; P² is -L²-Z²-; L¹ is selected from a bond, -CH₂-, -NH-, or -O-; and L² is selected from a bond or -CH₂-;
Z¹ is 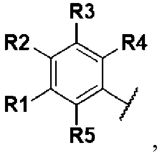 where the ring optionally contains 1 or 2 nitrogen heteroatoms;
Z² is selected from a 5- to 10-membered heteroaryl group, where one or more hydrogen atoms in Z² are each optionally substituted with halogen, a hydroxy group, a cyano group, a C₂-C₆ ester group, a C₁-C₆ alkyl group, a C₁-C₆ haloalkyl group, a C₁-C₆ alkoxy group, or a C₁-C₆haloalkoxy group independently;
R¹, R² and R⁵ are each independently selected from hydrogen, halogen, a hydroxy group, a cyano group, a nitro group, a C₁-C₆ alkyl group or a C₁-C₆ alkoxy group, and optionally, one or more hydrogen atoms in R¹, R² and R⁵ are each independently substituted with halogen, the hydroxy group, the amino group, the C₁-C₆ alkyl group or the C₃-C₆ cycloalkyl group;
one of R³ and R⁴ is H, and the other is selected from hydrogen, halogen, the hydroxy group, the cyano group, the nitro group, the amino group, the amido group, the carboxy group, the C₁-C₆ alkyl group, the C₃-C₆ cycloalkyl group, -O(CH₂)ₙ₁CR⁶R⁷R⁸, -NH(CH₂)ₙ₁R^{10,} -O(CH₂)ₙ₁R¹¹, -(CH₂)ₙ₁NH(CH₂)ₙ₂R¹², -(CH₂)ₙ₁NHCOR¹³, -(CH₂)ₙ₁NHSO₂R¹⁴, or -(CH₂)ₙ₁R¹⁵, and optionally, one or more hydrogen atoms in R³ and R⁴ are each independently substituted with halogen, the hydroxy group, the amino group, the C₁-C₆ alkyl group, the C₃-C₆ cycloalkyl group, the phenyl group or a hydroxy protecting group;
R⁶ and R⁷ are selected from hydrogen, or, together with the carbon atom to which they are attached, form a C₃-C₆ cycloalkyl group or a C₃-C₆ heterocycloalkyl group;
R⁸ is selected from -NH₂, -NHCOR⁹ or -NHSO₂R⁹, where R⁹ is selected from the C₁-C₆ alkyl group or the phenyl group;
R¹⁰, R¹¹, R¹², R¹³ and R¹⁴ are each independently selected from hydrogen, a cyano group, a C₁-C₆ alkyl group, a C₃-C₆ cycloalkyl group, a 3- to 6-membered heterocycloalkyl group, the phenyl group, or the 5- to 6-membered heteroaryl group;
R¹⁵ is selected from a 3- to 7-membered heterocycloalkyl group containing at least one nitrogen atom; and
n₁ and n₂ are selected from 0, 1, 2 or 3.

In a preferred embodiment of the present invention,
Y¹ and Y² jointly form a carbonyl group;
Y³ and Y⁴ are each independently selected from hydrogen, halogen, a cyano group, or a C₁-C₆ alkyl group optionally substituted with halogen or the cyano group; or Y³ and Y⁴, together with a carbon atom to which they are attached, form a C₃-C₆ cycloalkyl group or a 3- to 6-membered heterocycloalkyl group optionally substituted with halogen, a C₁-C₃ alkyl group, a C₁-C₃ haloalkyl group, a C₁-C₃ alkoxy group, a C₁-C₃ haloalkoxy group, the amino group, or a C₁-C₃ alkylamino group;
Y⁵ and Y⁶ are each independently selected from hydrogen, halogen, a cyano group, or a C₁-C₆ alkyl group optionally substituted with halogen or the cyano group; or Y⁵ and Y⁶, together with a carbon atom to which they are attached, form a C₃-C₆ cycloalkyl group or a 3- to 6-membered heterocycloalkyl group optionally substituted with halogen, a C₁-C₃ alkyl group, a C₁-C₃ haloalkyl group, a C₁-C₃ alkoxy group, a C₁-C₃ haloalkoxy group, the amino group, or a C₁-C₃ alkylamino group;
optionally, one of Y⁵ and Y⁶, together with one of Y³ and Y⁴ and the carbon atom to which they are attached, form a C₃-C₆ cycloalkyl group or a phenyl group optionally substituted with halogen or a C₁-C₃ alkyl group; and
preferably, the total amount of rings formed by Y¹, Y², Y³, Y⁴, Y⁵ and Y⁶ is not more than one.

In another preferred embodiment of the present invention, Z² may be selected from 5- to 10-membered heteroaryl groups containing 1 to 3 nitrogen atoms, or preferably, Z² may be selected from a pyridyl group, a pyrimidinyl group, a pyrazinyl group, a pyridazinyl group, a pyrrolyl group, a pyrazolyl group, an imidazolyl group, a triazolyl group or a quinolinyl group, where one or more hydrogen atoms in Z² may be each optionally substituted with halogen, a hydroxy group, a cyano group, a methyl group, an ethyl group, a trifluoromethyl group or a methoxy group.

In another preferred embodiment of the present invention, one of R³ and R⁴ is H, and the other may be selected from any one of the following groups:

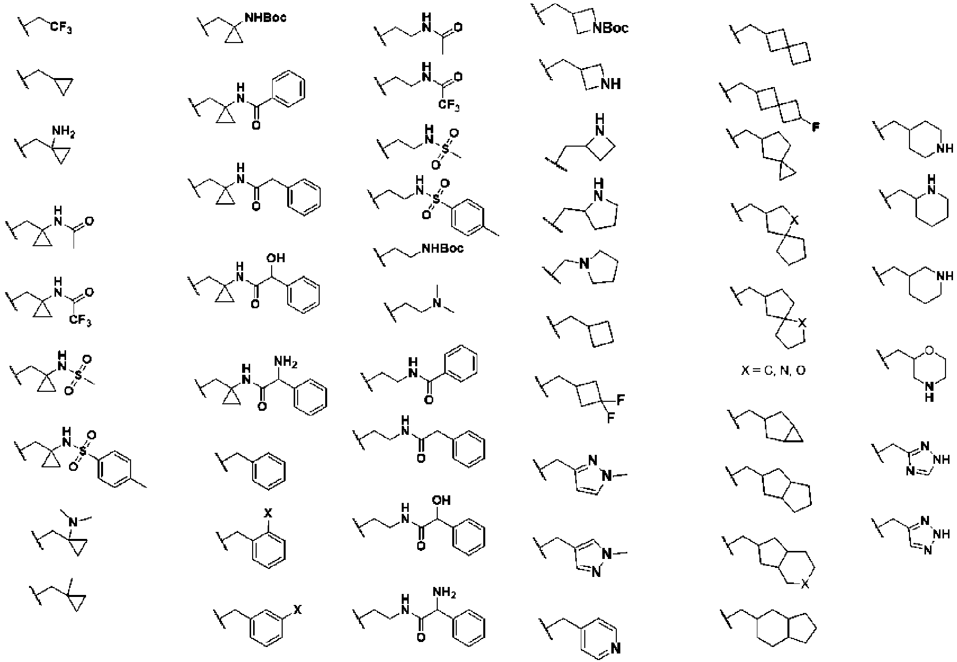

In another preferred embodiment of the present invention, R¹⁵ may be selected from a tetrahydropyrrolinyl group, a hexahydropyridyl group, a morpholinyl group, or a thiamorpholinyl group.

In another preferred embodiment of the present invention, halogen may be selected from F, Cl, or Br; the C₁-C₆ alkyl group may be selected from a methyl group, an ethyl group, a propyl group, or an isopropyl group; the C₁-C₆ haloalkyl group may be selected from a monofluoromethyl group, a difluoromethyl group, a trifluoromethyl group, a trifluoroethyl group, or a trichloromethyl group; the C₁-C₆ alkoxy group may be selected from a methoxy group, an ethoxy group, or a propoxy group; the C₁-C₆ haloalkoxy group may be selected from a trifluoromethoxy group or a trifluoroethoxy group; and/or the C₃-C₆ cycloalkyl group may be selected from a cyclopropyl group, a cyclobutyl group, a cyclopentyl group, or a cyclohexyl group.

In another embodiment of the present invention, the compound of formula (I) or the isotope-labeled compound thereof, or the optical isomer, geometric isomer, tautomer, or isomer mixture thereof, or the pharmaceutically acceptable salt thereof, or the prodrug thereof, or the metabolite thereof may have any one of the following structures:

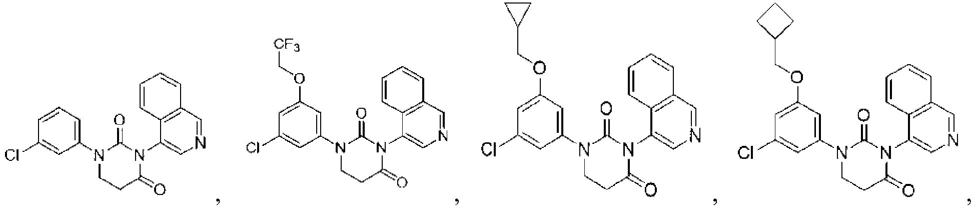

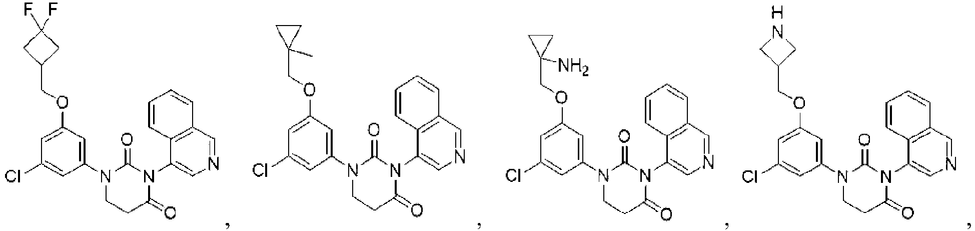

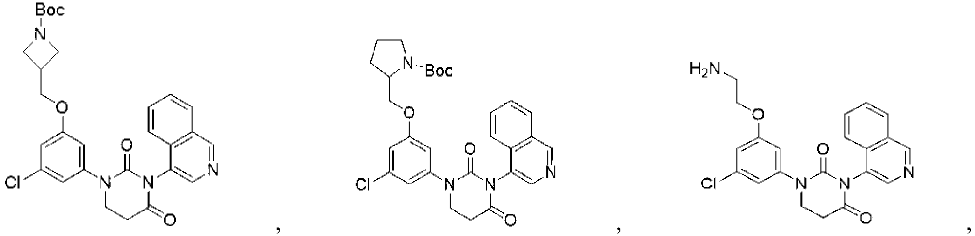

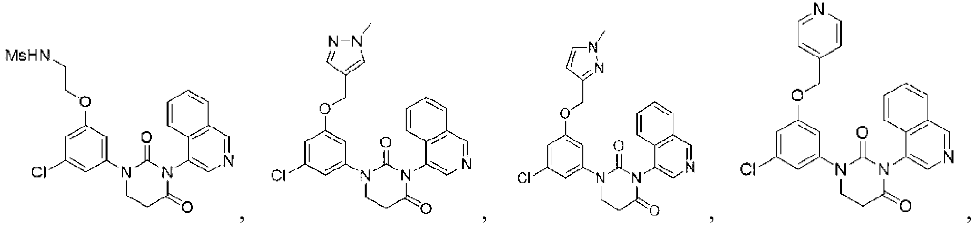

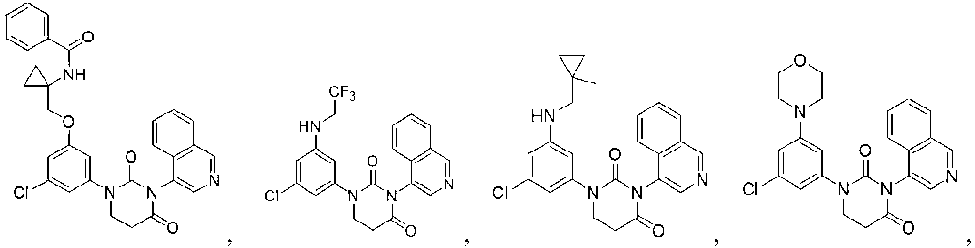

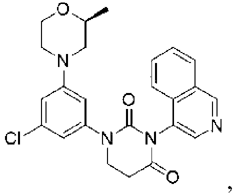

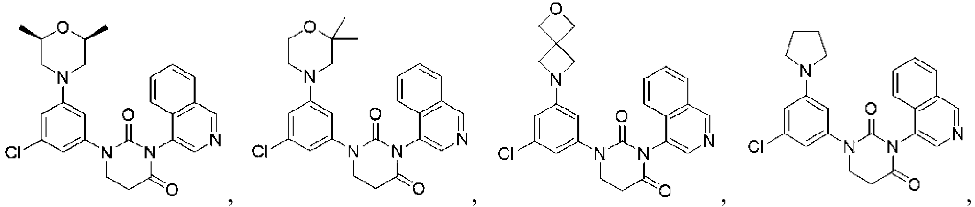

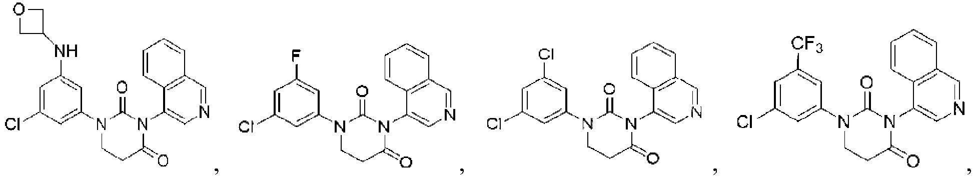

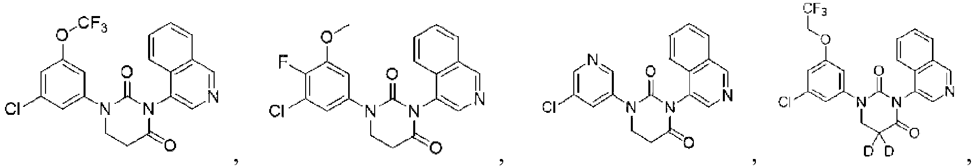

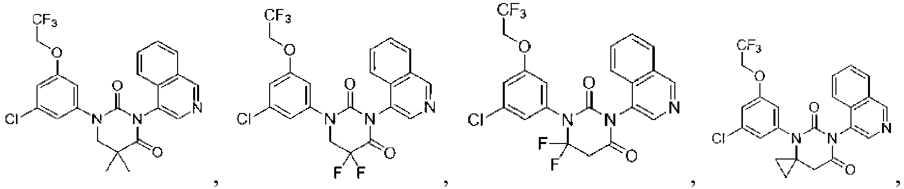

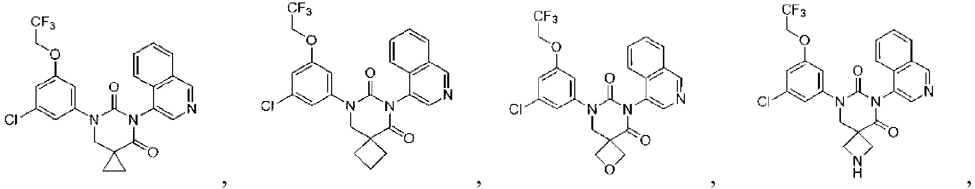

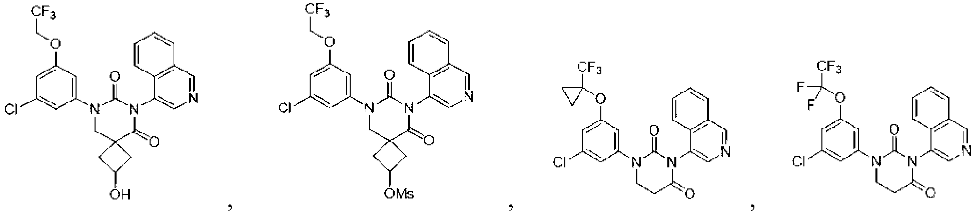

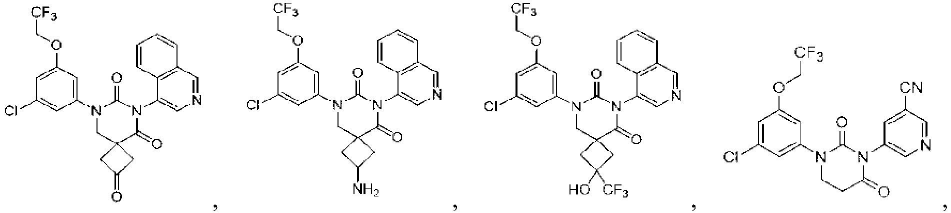

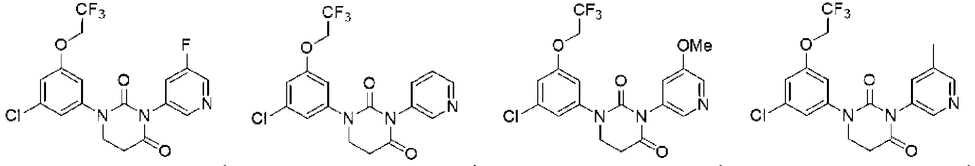

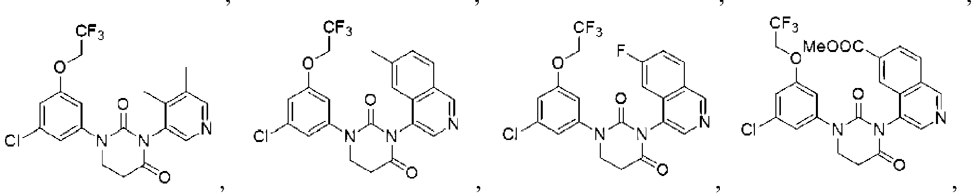

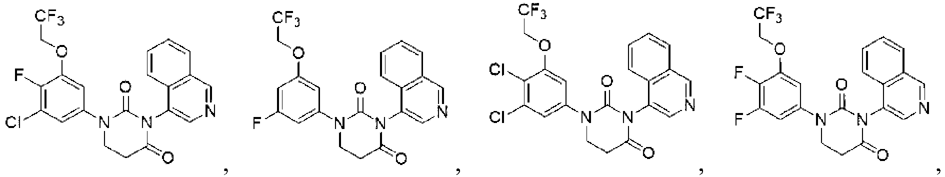

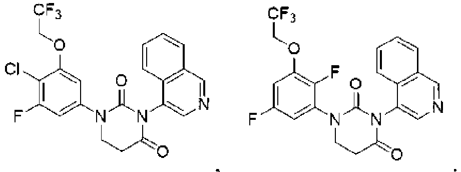

For brevity, the "compound of formula (I)" or the "compound in the present application" described below can also cover any isotope-labeled compound, optical isomer, geometric isomer, tautomer, isomer mixture, pharmaceutically acceptable salt, prodrug or metabolite of the compound of formula (I).

The term "optical isomer" means that when a compound has one or more chiral centers, each chiral center can have an R configuration or an S configuration, and various isomers formed by them are optical isomers. The optical isomers include all diastereoisomers, enantiomers, mesoforms, racemates, or mixtures thereof. For example, the optical isomer can be separated via a chiral chromatographic column or chiral synthesis.

The term "geometric isomer" means that when there is a double bond in the compound, the compound may have a cis isomer, a trans isomer, an E isomer and a Z isomer. The geometric isomer includes a cis isomer, a trans isomer, an E isomer, a Z isomer, or a mixture thereof.

The term "tautomer" refers to an isomer generated due to rapid movement of a specific atom in a molecule between two positions. Persons skilled in the art can understand that tautomers can transform into each other, and may coexist when an equilibrium state is reached in a specific state.

Unless otherwise clarified, the "compound of formula (I)" or the "compound in the present invention" mentioned herein also covers an isotope-labeled compound obtained by substituting any atom in the compound with its isotopic atom. The present invention includes all pharmaceutically acceptable isotope-labeled compounds of the compound of formula (I), where one or more atoms are substituted by an atom having the same atomic number as an atom usually found in nature but a different atomic mass or mass quantity.

Examples of isotopes suitable for inclusion in the compound in the present invention include isotopes of hydrogen, such as ²H(D) and ³H(T); isotopes of carbon, such as ¹¹C, ¹³C, and ¹⁴C; isotopes of chlorine, such as ³⁷Cl; isotopes of fluorine, such as ¹⁸F; isotopes of iodine such as ¹²³I and ¹²⁵I; isotopes of nitrogen such as ¹³N and ¹⁵N; isotopes of oxygen, such as ¹⁵O, ¹⁷O, and ¹⁸O; and isotopes of sulfur such as ³⁵S.

The isotope-labeled compound of formula (I) can generally be prepared by conventional techniques known to persons skilled in the art or by using a suitable isotope-labeled reagent in place of a previously used non-labeled reagent in a method analogous to that described in examples and preparations appended herein.

The compound of formula (I) may exist in a form of pharmaceutically acceptable salt such as acid addition salt and/or base addition salt of the compound of formula (I). Unless otherwise clarified, the "pharmaceutically acceptable salt" used herein includes acid addition salt or base addition salt that may exist in the compound of formula (I).

The pharmaceutically acceptable salt of the compound of formula (I) includes the acid addition salt and base addition salt of the compound of formula (I). A suitable acid addition salt is formed by an acid that forms a non-toxic salt. Examples of the acid addition salt include, but are not limited to: acetate, adipate, aspartate, benzoate, benzenesulfonate, bicarbonate/carbonate, bisulfate/sulfate, borate, camphorsulfonate, citrate, cyclamate, edisylate, formate, fumarate, glucoheptonate, gluconate, glucuronic acid salt, hexafluorophosphate, 2-(4-hydroxybenzyl)benzoate, hydrochloride/chloride, hydrobromide/bromide, hydroiodide/iodide, 2-isethionate, lactate, malate, maleate, malonate, methanesulfonate, methylsulfate, naphthalate, 2-naphthalenesulfonate, nicotinate, nitrate, orotate, oxalate, palmitate, phosphate/hydrogen phosphate/dihydrogen phosphate, pyroglutamate, glucarate, stearate, salicylate, tannate, tartrate, tosylate, and trifluoroacetate. A suitable base addition salt is formed by a base that forms a non-toxic salt. Examples of the base addition salt include, but are not limited to, aluminum, arginine, calcium, choline, diethylamine, diethanolamine, glycine, lysine, magnesium, meglumine, ethanolamine, potassium, sodium, tromethamine, and zinc salt. Half-salts of acids and bases such as hemisulfate and hemicalcium salts may also be formed. For a review of suitable salt, refer to "Handbook of Pharmaceutical Salts: Properties, Selection and Use" (Stahl and Wermuth, Wiley-VCH, 2002). A method for preparing the pharmaceutically acceptable salt of the compound described herein is known to persons skilled in the art.

Some compounds in the present invention can exist in an unsolvated form and a solvated form (including a hydrated form). In general, the compounds of formula (I) fall within the scope of the present invention regardless of whether the compounds exist in the solvated or unsolvated form.

Some compounds in the present invention may exist in different crystalline or amorphous forms, and the compounds of formula (I) fall within the scope of the present invention regardless of which form the compounds are in.

To avoid ambiguity, definitions of terms used herein are provided below. Unless otherwise clarified, the terms used herein have the same meanings as those described below.

The term "pharmaceutically acceptable" means that a corresponding compound, carrier or molecule is suitable for administration to humans. Preferably, the term modifies a product approved by any national regulatory agency such as CFDA (PRC), EMEA (Europe) or FDA (USA) for administration to mammals, preferably, humans.

The term "prodrug" refers to a derivative that is converted into the compound in the present invention via an enzyme-catalyzed reaction such as oxidation, reduction or hydrolysis with an enzyme, gastric acid, or the like under a physiological condition in vivo.

The term "metabolite" refers to all molecules originating from any compound in the present invention in a cell or an organism, preferably, humans.

As used herein, the term "hydroxy group" refers to -OH; the term "cyano group" refers to -CN; the term "amino group" refers to -NH₂; and the term "nitro group" refers to -NO₂. As used herein, the term "hydroxyl protecting group" refers to a protecting group introduced onto a hydroxyl group to avoid side reactions or unnecessary chemical changes of the hydroxyl group during reactions. Common hydroxyl protecting groups include, but are not limited to, Boc, Ms, TMS, and TBS.

The term "substituted" used herein means that one or more (preferably, 1 to 5, or more preferably, 1 to 3) hydrogen atoms in a group are independently replaced by a corresponding number of substituents.

The term "independently" used herein means that when the number of substituents is greater than one, these substituents may be the same or different.

The term "optional" or "optionally" used herein means that an event modified by the term may or may not occur. For example, a group "optionally substituted" means that the group may be unsubstituted or substituted.

The term "heteroatom" used herein represents oxygen (O), nitrogen (N), or S(O)ₘ (m can be 0, 1, or 2, namely, a sulfur atom S, a sulfoxide group SO, or a sulfonyl group S(O)₂).

The term "alkyl group" used herein refers to saturated aliphatic hydrocarbon, including straight and branched chains. In some embodiments, an alkyl group has 1-8, 1-6 or 1-3 carbon atoms. For example, the term "C₁-C₈ alkyl group" refers to a straight or branched chain radical having 1 to 8 carbon atoms. A definition of the term "C₁-C₈ alkyl group" includes terms "C₁-C₆ alkyl group", "C₁-C₃ alkyl group" and "C₁-C₄ alkyl group". Examples of the alkyl group include, but are not limited to, a methyl group, an ethyl group, a propyl group, an isopropyl group, a butyl group, an isobutyl group, a sec-butyl group, a tert-butyl group, a pentyl group, a 2-pentyl group, a 3-pentyl group, an isopentyl group, a neopentyl group, an (R)-2-methylbutyl group, an (S)-2-methylbutyl group, a 3-methylbutyl group, a 2,3-dimethylpropyl group, a 2,3-dimethylbutyl group, and a hexyl group. The alkyl group is optionally substituted with one or more (for example, 1 to 5) suitable substituents.

The term "n-membered heteroaryl group" used herein refers to a heteroaryl group having m carbon atoms forming an aromatic ring and (n-m) heteroatoms forming an aromatic ring, where the heteroatom is selected from O, S and N. For example, five- to seven-membered heteroaryl groups include, but are not limited to, pyrazine, pyrazole, pyrrole, furan, thiophene, thiazole, and pyridine. The heteroaryl group is optionally substituted with one or more suitable substituents.

The term "haloalkyl group" used herein refers to an alkyl group having one or more halogen substituents (at most a perhaloalkyl group, that is, each hydrogen atom of the alkyl group is substituted by a halogen atom). For example, the term "C₁-C₆ haloalkyl group" refers to a C₁-C₆ alkyl group having one or more halogen substituents (at most a perhaloalkyl group, that is, each hydrogen atom of the alkyl group is substituted by a halogen atom). For another example, the term "C₁-C₄ haloalkyl group" refers to a C₁-C₄ alkyl group (up to a perhaloalkyl group, that is, each hydrogen atom of the alkyl group is substituted with a halogen atom) having one or more halogen substituents; the term "C₁-C₃ haloalkyl group" refers to a C₁-C₃ alkyl group (up to the perhaloalkyl group, that is, each hydrogen atom of the alkyl group is substituted with a halogen atom) having one or more halogen substituents; and the term "C₁-C₂ haloalkyl group" refers to a C₁-C₂ alkyl group (that is, the methyl group or the ethyl group) (up to the perhaloalkyl group, that is, each hydrogen atom of the alkyl group is substituted with a halogen atom) having one or more halogen substituents. For another example, the term "C₁ haloalkyl group" refers to a methyl group having 1, 2, or 3 halogen substituents. Examples of the haloalkyl group include CF₃, C₂F₅, CHF₂, CH₂F, CH₂CF₃, CH₂Cl, and the like.

Herein, number ranges related to the number of substituents, the number of carbon atoms, and the number of ring atoms represent enumerations of all integers within the range, and the range is only used as a simplified representation. For example, "1-4 substituents" represent 1, 2, 3, or 4 substituents; and "3-8 ring atoms" represent 3, 4, 5, 6, 7, or 8 ring atoms. Therefore, the number ranges related to the number of substituents, the number of carbon atoms, and the number of ring atoms also cover any one of its sub-ranges, and each sub-range is also construed as being disclosed herein.

The compound in the present application can be prepared in various ways known to persons skilled in the field of organic synthesis. With reference to a synthesis route of a specific compound in a specific embodiment of the present application, persons skilled in the art can make appropriate adjustments to reactant raw materials and reaction conditions to obtain a synthesis method of another compound.

In a second aspect, the present application provides a pharmaceutical composition, containing the compound of formula (I) or the isotope-labeled compound, the optical isomer, the geometric isomer, the tautomer, the isomer mixture, the pharmaceutically acceptable salt, the prodrug, or the metabolite thereof, and a pharmaceutically acceptable carrier thereof.

The pharmaceutically acceptable carrier can be an organic or inorganic inert carrier material. For example, appropriate carriers include water, gelatin, gum arabic, lactose, starch, magnesium stearate, talc, vegetable oil, polyalkylene glycol, petrolatum, mannitol, cellulose, a cellulose derivative, sodium saccharin, glucose, sucrose, magnesium carbonate, saline, glycerin and ethanol. In addition, the pharmaceutical composition may also contain another drug additive, for example, a flavoring agent, a preservative, a stabilizer, an emulsifier, a buffering agent, a diluent, a binder, a wetting agent, a disintegrant, a lubricant or a glidant.

A dosage form of the pharmaceutical composition in the present application may be a liquid dosage form, a solid dosage form or a semisolid dosage form. The liquid dosage form can be a solution (including a true solution and a colloid solution), an emulsion (including o/w type, w/o type and double emulsions), a suspension, an injection (including a water injection, a powder injection and an infusion), eye drops, nasal drops, a lotion, a liniments, and the like; the solid dosage form can be a tablet (including an ordinary tablet, an enteric-coated tablet, a buccal tablet, a dispersible tablet, a chewable tablet, an effervescent tablet and an orally disintegrating tablet), a capsule (including a hard capsule, a soft capsule and an enteric-coated capsule), granules, powder, pills, a suppository, films, patches, an aerosol, a spray, and the like; and the semisolid dosage form can be an ointment, a gel, a paste, and the like. The pharmaceutical composition in the present application can be prepared into a common formulation or a sustained-release formulation, a controlled-release formulation, a targeted formulation and various particle delivery systems.

In some embodiments, the dosage form of the pharmaceutical composition includes tablet, granule, powder, syrup, inhalation and injection.

The solid dosage form administered orally may include a capsule, a tablet, pills, powder and granules. In such solid dosage form, the active compound is mixed with at least one inert excipient (or carrier) (for example, sodium citrate or dicalcium phosphate), which may also include: (a) a filler or a blending agent (for example, starch, lactose, sucrose, glucose, mannitol and silicic acid); (b) a binder (for example, carboxymethylcellulose, alginate, a gel, polyvinylpyrrolidone, sucrose and gum arabic); (c) a humectant (for example, glycerol); (d) a disintegrant (for example, agar-agar, calcium carbonate, potato or tapioca starch, alginic acid, some synthetic silicates and sodium carbonate); (e) a solution blocker (for example, paraffin); (f) an absorption enhancer (for example, a quaternary ammonium compound); (g) a wetting agent (for example, cetyl alcohol and glyceryl monostearate); (h) an adsorbent (for example, kaolin and bentonite) and (i) a lubricant (for example, talc, calcium stearate, magnesium stearate, polyethylene glycol solid and sodium lauryl sulfate) or a mixture thereof.

Formulations suitable for parenteral administration such as injections may include aqueous and nonaqueous isotonic sterile solutions suitable for injection, and aqueous and nonaqueous sterile suspensions. The parenterally administered formulations provided herein are optionally accommodated in sealed unit-dose or multi-dose containers, such as ampoules, and may be stored in freeze-dried (lyophilized) conditions that require only addition of a sterile liquid carrier (such as water for injection) immediately before use. Examples of suitable diluents for reconstitution of the pharmaceutical composition (for example, before injection) include bacteriostatic water for injection, 5% dextrose in water, phosphate buffered saline, Ringer's (Ringer's) solution, saline, sterile water, deionized water and a combination thereof.

The spray can contain excipients such as lactose, talc, silicic acid, aluminum hydroxide, calcium silicate and polyamide powder, or a mixture of these substances. The spray can additionally contain customary propellants, for example, chlorofluorocarbons and volatile unsubstituted hydrocarbons, for example, butane and propane. The inhalant can contain an excipient such as lactose, or an aqueous solution containing, for example, polyethylene oxide-9-lauryl ether, glycocholate and deoxycholate, or an oily solution administered in a form of nasal drops, spray, or gel.

Content of the compound in the present application in the pharmaceutical composition thereof can be adjusted based on an actual need (for example, a dosage form, an administration method and an administered object), for example, 0.1wt%-95wt%, for example, 1wt%-95wt%, 5wt%-90wt%, or 10wt%-80wt%.

Specifically, the pharmaceutical composition in the present application may particularly include 0.01-10 g (for example, 0.05 g, 0.1 g, 0.5 g, 1 g or 5 g) of the compound in the present application.

In a third aspect, the present application provides use of the compound of formula (I) or the isotope-labeled compound, the optical isomer, the geometric isomer, the tautomer, the isomer mixture, the pharmaceutically acceptable salt, the prodrug, or the metabolite thereof in preparation of a drug for treating or preventing a coronavirus infection or a disease or symptom caused by a coronavirus in a subject in need. The compound of formula (I) or the isotope-labeled compound, the optical isomer, the geometric isomer, the tautomer, the isomer mixture, the pharmaceutically acceptable salt, the prodrug, or the metabolite thereof may be used to treat or prevent a coronavirus infection or a disease or symptom caused by a coronavirus in a subject in need.

The term "subject" used in the present application refers to any human or non-human organism that could potentially benefit from treatment with the compound of formula (I). Exemplarily, a subject includes a human or a mammal at any age. Preferably, the subject is a human.

The term "treatment" used herein includes treatment of a disease or a symptom in the mammal, especially the human, and includes: (a) inhibition of an infection, a disease or a symptom, that is, inhibition or delaying of development of the infection, the disease or the symptom; (b) relief of the infection, the disease or the symptom, that is, improvement of the disease or the symptom; and/or (c) cure of the infection, the disease or the symptom.

The term "prevention" used herein includes a prophylactic therapy in the mammal, especially the human, to reduce occurrence possibility of the infection, the disease or the symptom. A patient can be selected for the prophylactic therapy based on a factor of an increased risk of infection or having a disease or a symptom compared to the general population. The "prevention" can include treatment of a subject who has not yet exhibited an infection or a clinical condition, and prevention of a second occurrence of the same or similar infection or clinical condition.

The inventor of the present application have found that the compound in the present application can inhibit the coronavirus infection. For example, the compound can act as a reversible covalent small-molecule inhibitor against 3CL protease of SARS-CoV-2 (namely, a 3CL protease inhibitor), thereby inhibiting replication of the SARS-CoV-2. Therefore, the compound in the present application may be used to prevent or treat the coronavirus infection or the disease or the symptom caused by the coronavirus.

In some embodiments, the coronavirus is selected from severe acute respiratory syndrome coronavirus (SARS-CoV), severe acute respiratory syndrome coronavirus 2 (SARS-CoV-2), Middle East respiratory syndrome coronavirus (MERS-CoV), human coronavirus OC43 (HCoV-OC43), murine hepatitis coronavirus (MHV), and a coronavirus that shares more than 85% homology with any one of the foregoing coronaviruses and that has viral activity, related to mixed infections caused by more than one coronavirus subtype. In some embodiments, the coronavirus is the severe acute respiratory syndrome coronavirus 2 (SARS-CoV-2).

In some embodiments, the disease or symptom caused by a coronavirus includes a respiratory infection, severe acute respiratory syndrome (SARS), pneumonia (including severe pneumonia), gastroenteritis (including acute gastroenteritis), cough, fever, shivering, vomiting, headache, chill, shortness of breath, cytokine storm and the like caused by the virus.

In a fourth aspect, the present application provides a method for treating or preventing a coronavirus infection or a disease or symptom caused by a coronavirus, where the method includes giving a therapeutically effective dose of a compound of formula (I) or an isotope-labeled compound, an optical isomer, a geometric isomer, a tautomer, an isomer mixture, pharmaceutically acceptable salt, a prodrug, or a metabolite thereof to a subject in need.

In some embodiments, the compound in the present invention can be administered orally, parenterally, intravenously, intramuscularly, subcutaneously, nasally, buccally, or ocularly, or via lung, respiratory tract or vagina, or rectally, intraperitoneally, intralesionally, around the lesion, or via another route.

The "therapeutically effective dose" refers to the dose of the compound in the present application that is effective in treating or preventing the coronavirus infection or the disease or symptom caused by the coronavirus when administered alone or in combination.

A specific dose administered depends on the route of administration, severity of the disease, age and weight of the patient, and another factor usually considered by an attending physician when determining the most suitable individual regimen and dosage level for a particular patient. For example, a daily dose of the compound in the present application may be specifically 0.001-150 mg/kg body weight (for example, 0.1 mg/kg body weight, 1 mg/kg body weight, 10 mg/kg body weight or 100 mg/kg body weight).

Specific administration frequency can be determined by persons skilled in the related art, for example, once a day, once in 2 days, once in 3 days, once in 4 days, once in 5 days, once in 6 days, twice a day, or thrice a day.

Persons skilled in the art can understand that a definition and a preferred item described in one aspect of the present application are also applicable to other aspects. Persons skilled in the art can understand that the embodiments of various aspects of the present application can be combined in various manners without departing from the subject and concept of the present application, and these combinations also fall within the scope of the present application.

### DETAILED DESCRIPTION

A compound of formula (I) in the present application can be synthesized in various methods known to persons skilled in the field of organic synthesis. The following specific examples provide some exemplary synthetic methods of the compound of formula (I), and these methods are well known in the field of synthetic chemistry. Apparently, with reference to the exemplary solution in this patent, persons skilled in the art can easily design another synthetic route for the compound of formula (I) by appropriately adjusting a reactant, a reaction condition and a protecting group.

The present invention is further described below with reference to examples, but these examples are not intended to limit the scope of the present invention. Unless otherwise stated, all reactants used in the examples are commercially available; and instruments and devices used in synthesis experiments and product analyses and detection are all conventional instruments and devices commonly used in organic syntheses.

### Example 1: Preparation of Compound GDI15-7099

### (1) Synthesis steps of compound 3

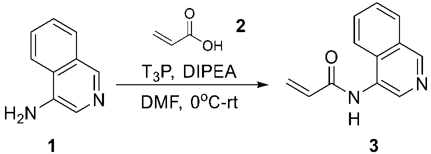

Under nitrogen protection at 0°C, DIPEA (3.59g, 27.8 mmol) and T₃P (8.85g, 27.8 mmol) were slowly added to a solution of Compound 1 (2g, 13.9 mmol) and Compound 2 (1.27g, 14.0 mmol) in DMF (10 mL). The mixture was further allowed to react at room temperature for 1 hour. After the reaction ended, water (100 mL) was added to the mixture, and the mixture was extracted with EtOAc (100 mL × 3). The organic phases were combined and washed with saturated brine, dried over anhydrous sodium sulfate, filtered, and evaporated to dryness. The residue was purified by column chromatography (MeOH/DCM, 5% to 10%) to afford compound 3 (2g, 72.66% yield) as a yellow solid. ESI-MS: [M+H] ⁺, 198.9.

### (2) Synthesis steps of compound 5

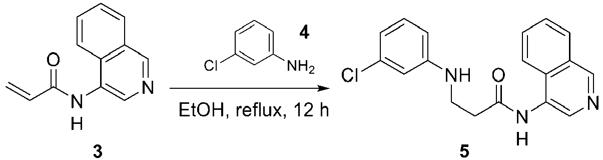

Compound 4 (2.6g, 20.18 mmol) was added to a solution of compound 3 (2.0g, 10.09 mmol) in EtOH (5 mL), and the mixture was allowed to react at 80°C for 12 hours. After the reaction ended, water (50 mL) was added to the mixture, and the mixture was extracted with DCM (50 mL × 3). The organic phases were combined and washed with saturated brine, dried over anhydrous sodium sulfate, filtered, and evaporated to dryness. The residue was purified by column chromatography (MeOH/DCM, 9% to 10%) to afford compound 5 (1.6g, 48.67% yield) as a brown oil. ESI-MS: [M+H] ⁺, 326.0.

### (3) Synthesis steps of compound GDI15-7099

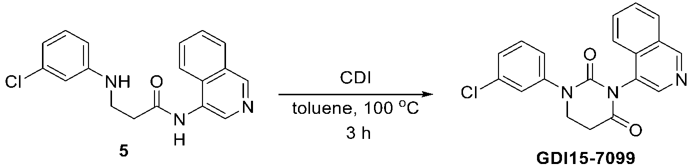

Toluene (5 mL) was added to a mixture of compound 5 (500 mg, 1.53 mmol) and CDI (497.67 mg, 3.07 mmol). The mixture was heated to 100°C under nitrogen atmosphere and was allowed to react for 3 hours. After the reaction ended, the mixture was cooled to room temperature and filtered, and the filtrate was concentrated. Water was then added to the concentrate, and the mixture was extracted with EtOAc. The organic phases were combined and washed with saturated brine, dried over anhydrous sodium sulfate, filtered, and evaporated to dryness. The residue was purified by column chromatography (MeOH/DCM, 4% to 5%) to afford compound GDI15-7099 (38.21 mg) as a white solid. ESI-MS: [M+H] ⁺, 352.0.

**¹H NMR** (400 MHz, DMSO) *δ* 9.41 (s, 1 H), 8.49 (s, 1 H), 8.26 (d, *J* = 8.2 Hz, 1 H), 7.99 (d, *J* = 8.3 Hz, 1 H), 7.86 (t, *J* = 7.6 Hz, 1 H), 7.77 (t, *J =* 7.5 Hz, 1 H), 7.56 (d, *J =* 1.5 Hz, 1 H), 7.43 (dd, *J=* 15.8, 7.1 Hz, 2 H), 7.34 (d, *J =* 7.7 Hz, 1 H), 4.15 (d, *J* = 5.6 Hz, 2 H), 3.26 (dd, *J* = 15.6, 7.5 Hz, 1 H), 3.15 - 3.04 (m, 1 H).

### Example 2: Preparation of Compound GDI15-7245

### (1) Synthesis steps of compound 3

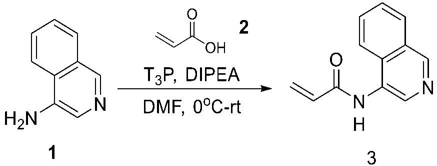

At room temperature under nitrogen protection, compound 2 (2.0g, 27.74 mmol), T₃P (17.65g, 27.74 mmol, 50%wt) and DIPEA (10.76g, 83.23 mmol) were added to a solution of compound 1 (4.0g, 27.74 mmol) in DMF (40 mL). The mixture was then further allowed to react at room temperature for 3 hours. After the reaction ended, water (10 mL) was added to the mixture, and the mixture was extracted with EtOAc (30 mL × 3). The organic phases were combined and washed with saturated brine, dried over anhydrous sodium sulfate, filtered, and evaporated to dryness. The residue was purified by column chromatography (PE/EtOAc, 10% to 20%) to afford compound 3 (2.2g, 40.0% yield) as a white solid. ESI-MS: [M+H] ⁺, 198.95.

### (2) Synthesis steps of compound 4

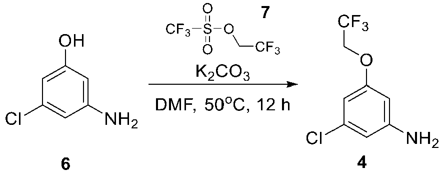

At room temperature under nitrogen protection, compound 7 (1.63g, 6.97 mmol) and K₂CO₃ (2.89g, 20.90 mmol) were added to a solution of compound 6 (1.0g, 6.97 mmol) in DMF (10 mL). The mixture was then allowed to react at 50°C for 16 hours. After the reaction ended, water (5 mL) was added to the mixture, and the mixture was extracted with EtOAc (20 mL × 3). The organic phases were combined and washed with saturated brine, dried over anhydrous sodium sulfate, filtered, and evaporated to dryness. The residue was purified by column chromatography (PE/EtOAc, 10: 1) to afford compound 4 (1.0g, 57.02% yield) as a colorless oil. ESI-MS: [M+H] ⁺, 226.00.

### (3) Synthesis steps of compound 5

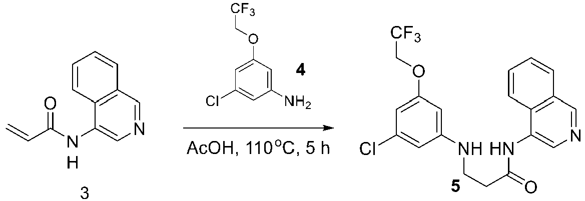

At room temperature under nitrogen protection, compound 3 (699.80mg, 3.53 mmol) was added to a solution of compound 2 (400 mg, 1.77 mmol) in AcOH (4 mL), and the mixture was allowed to react at 110°C for 5 hours. After the reaction ended, water (2 mL) was added to the mixture, and the mixture was extracted with EtOAc (10 mL × 3). The organic phases were combined and washed with saturated brine, dried over anhydrous sodium sulfate, filtered, and evaporated to dryness. The residue was purified by column chromatography (DCM/MeOH, 20: 1) to afford compound 5 (170 mg, 20.40% yield) as a white solid. ESI-MS: [M+H] ⁺, 424.00.

### (3) Synthesis steps of compound GDI15-7245

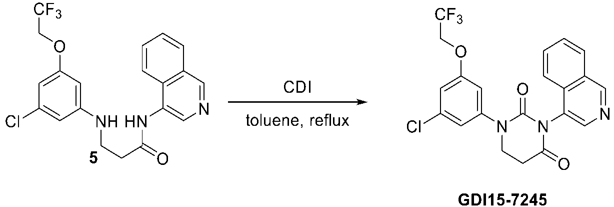

A solution of toluene (2 mL) was added to a mixture of compound 5 (150 mg, 0.35 mmol) and CDI (343.51 mg, 2.12 mmol). The mixture was allowed to react at 110°C for 3 hours. After the reaction ended, the mixture was concentrated in vacuo and further purified by prep-HPLC (Gemini 5 µm C18 column, 150×21.2 mm, eluted with 30% to 90% MeCN/H₂O containing 0.1% NH₄OH) to afford compound GDI15-7245 (30.14 mg, 18.89% yield) as a white solid. ESI-MS: [M+H] ⁺, 450.10.

**¹H NMR** *J* = 8.2 Hz, 1 H), 7.95 (d, *J* = 8.2 Hz, 1 H), 7.84 (t, *J=* 7.5 Hz, 1 H), 7.75 (t, *J* = 7.2 Hz, 1 H), 7.25 (s, 1 H), 7.15 (dd, *J* = 5.9, 1.8 Hz, 2 H), 4.85 (m, 2 H), 4.13 (m, 2 H), 3.25 - 3.21 (m, 1 H), 3.11 - 3.04 (m, 1 H).

### Example 3: Preparation of Compound GDI15-7434

### (1) Synthesis steps of compound 3

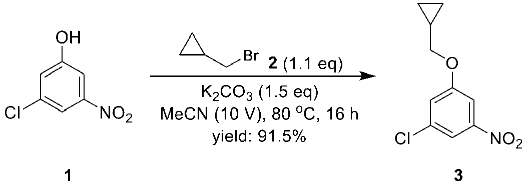

Compound 1 (1g, 5.76 mmol, 1 eq) was dissolved in MeCN (10 mL). K₂CO₃ (1.19g, 8.63 mmol, 1.5 eq) and compound 2 (933 mg, 6.91 mmol, 1.1 eq) were added successively at 20°C. The reaction mixture was allowed to react at 80°C for 16 hours, and the reaction ended as monitored by LCMS. Water (20 mL) was added to the reaction solution, and the resulting mixture was extracted with EtOAc (20 mL × 3). The organic phases were combined and evaporated to dryness to afford the target compound 3 (1.2g, 5.27 mmol, 91.5% yield) as a brown oil. ESI-MS: [M+H]⁺, 228.0.

### (2) Synthesis steps of compound 4

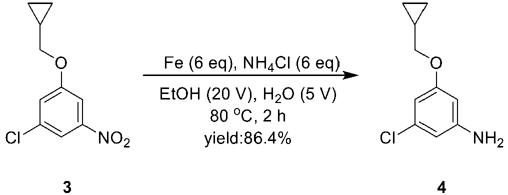

Compound 3 (1.2g, 5.27 mmol, 1 eq) was dissolved in EtOH (24 mL) and H₂O (6 mL). NH₄Cl (1.69g, 31.6 mmol, 6 eq) was added at 20°C, and then the temperature was raised to 80°C. Iron powder (1.77g, 31.6 mmol, 6 eq) was added portionwise at 80°C. The reaction mixture was allowed to react at 80°C for 2 hours, and the reaction ended as monitored by LCMS. The reaction solution was filtered while hot, and a filter cake was rinsed with hot ethanol. The organic phases were combined and evaporated to dryness to afford the target compound 4 (0.9g, 4.55 mmol, 86.4% yield) as a brown oil. ESI-MS: [M+H] ⁺, 198.1.

### (3) Synthesis steps of compound 6

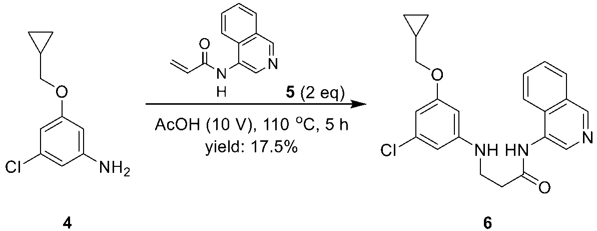

Compound 4 (0.4g, 2.02 mmol, 1 eq) was dissolved in AcOH (4 mL). Compound 5 (802 mg, 4.05 mmol, 2 eq) was added at 20°C. The reaction mixture was allowed to react at 110°C for 5 hours under nitrogen atmosphere, and the reaction ended as monitored by LCMS. Water (10 mL) was added to the reaction solution, and the resulting mixture was extracted with EtOAc (8 mL × 3). The organic phases were combined and evaporated to dryness. The residue was purified by thin-layer chromatography (DCM/MeOH = 20/1) to afford the target compound 6 (140 mg, 354 µmol, 17.5% yield) as a white solid. ESI-MS: [M+H]⁺, 396.1.

### (4) Synthesis steps of compound GDI15-7434

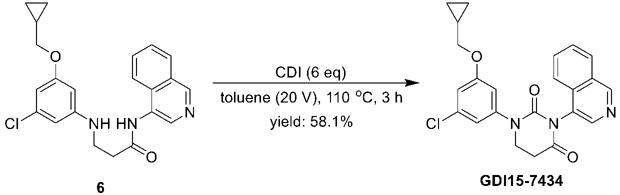

Compound 6 (120 mg, 303 µmol, 1 eq) was dissolved in toluene (2.4 mL). CDI (295.9 mg, 1.82 mmol, 6 eq) was added at 20°C. The reaction mixture was allowed to react at 110°C for 3 hours, and the reaction ended as monitored by LCMS. The reaction solution was evaporated to dryness, and further purified by high performance liquid chromatography (column: Waters Xbridge BEH C18 100*30 mm*10 µm; mobile phase: [H₂O (10 mM NH₄HCO₃)-ACN]; gradient: 45%-75%, 8.0 min) to afford the target compound GDI15-7434 (74.3 mg, 176 µmol, 58.1% yield) as a white solid. ESI-MS: [M+H]⁺, 422.1.

1H NMR (400 MHz, DMSO) δ 9.36 (s, 1 H), 8.45 (s, 1 H), 8.23 (d, *J =* 8.1 Hz, 1 H), 7.94 (d, *J* = 7.9 Hz, 1 H), 7.84 (dt, *J* = 1.1, 7.6 Hz, 1 H), 7.77 - 7.72 (m, 1 H), 7.11 (t, *J* = 1.8 Hz, 1 H), 7.01 (t, *J* = 2.0 Hz, 1 H), 6.93 (t, *J* = 2.0 Hz, 1 H), 4.16 - 4.06 (m, 2 H), 3.86 (d, *J* = 7.0 Hz, 2 H), 3.28 - 3.19 (m, 1 H), 3.14 - 3.03 (m, 1 H), 1.25 - 1.16 (m, 1 H), 0.59 - 0.54 (m, 2 H), 0.34 - 0.30 (m, 2 H).

### Example 4: Preparation of Compound GDI15-7521

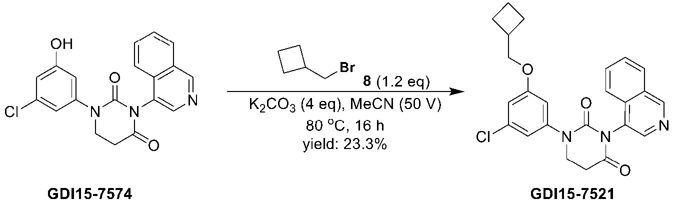

Compound GDI15-7574 (30 mg, 81.6 µmol, 1 eq) and compound 8 (14.6 mg, 97.8 µmol, 11.0 µL, 1.2 eq) were dissolved in MeCN (1.5 mL). K₂CO₃ (45.1 mg, 326 µmol, 4 eq) was added at 25°C. The reaction mixture was allowed to react at 80°C for 16 hours, and the reaction ended as monitored by LCMS. The reaction solution was filtered and the filtrate was purified by high performance liquid chromatography (column: Phenomenex Luna C18 100*30 mm*3 µm; mobile phase: [H₂O (0.2% FA)-ACN]; gradient: 25%-65%, 8.0 min) to afford the target compound GDI15-7521 (8.6 mg, 19.0 µmol, 23.3% yield, 96.4% purity) as a white solid. ESI-MS: [M+H] ⁺, 436.1.

¹H NMR (400 MHz, DMSO) δ 9.43 (s, 1 H), 8.52 (s, 1 H), 8.30 (d, *J* = 8.1 Hz, 1 H), 8.01 (d, *J* = 8.4 Hz, 1 H), 7.90 (dt, *J* = 1.1, 7.7 Hz, 1 H), 7.84 - 7.76 (m, 1 H), 7.18 (t, *J =* 1.7 Hz, 1 H), 7.08 (t, *J* = 1.9 Hz, 1 H), 7.01 (t, *J* = 2.0 Hz, 1 H), 4.22 - 4.13 (m, 2 H), 4.05 (d, *J* = 6.8 Hz, 2 H), 3.34 - 3.24 (m, 1 H), 3.19 - 3.09 (m, 1 H), 2.80 - 2.71 (m, 1 H), 2.16 - 2.07 (m, 2 H), 2.01 - 1.85 (m, 4 H).

### Example 5: Preparation of Compound GDI15-7522

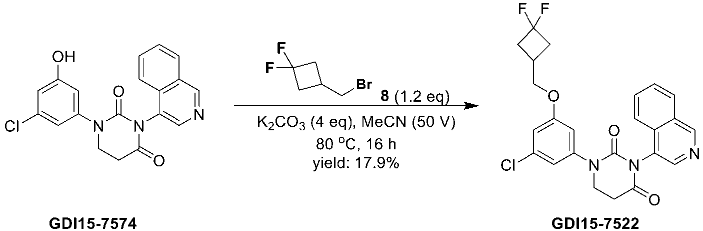

Compound GDI15-7574 (30 mg, 81.6 µmol, 1 eq) and compound 8 (18.1 mg, 97.8 µmol, 1.2 eq) were dissolved in MeCN (1.5 mL). K₂CO₃ (45.1 mg, 326 µmol, 4 eq) was added at 25°C. The reaction mixture was allowed to react at 80°C for 16 hours, and the reaction ended as monitored by LCMS. The reaction solution was filtered and the filtrate was purified by high performance liquid chromatography (column: Phenomenex Luna C18 100*30 mm*3 µm; mobile phase: [H₂O (0.2% FA)-ACN]; gradient: 25%-65%, 8.0 min) to afford the target compound GDI15-7522 (7.0 mg, 14.6 µmol, 17.9% yield, 98.2% purity) as a white solid. ESI-MS: [M+H] ⁺, 472.1.

¹H NMR (400 MHz, DMSO) δ 9.36 (s, 1 H), 8.45 (s, 1 H), 8.23 (d, *J* = 8.1 Hz, 1 H), 7.94 (d, *J* = 8.4 Hz, 1 H), 7.83 (dt, *J* = 1.0, 7.6 Hz, 1 H), 7.77 - 7.69 (m, 1 H), 7.15 (t, *J* = 1.7 Hz, 1 H), 7.03 (t, *J =* 1.9 Hz, 1 H), 6.97 (t, *J =* 2.0 Hz, 1 H), 4.16 - 4.05 (m, 4 H), 3.28 - 3.17 (m, 1 H), 3.12 - 3.03 (m, 1 H), 2.75 - 2.65 (m, 2 H), 2.61 - 2.54 (m, 1 H), 2.44 (s, 1 H).

### Example 6: Preparation of Compound GDI15-7549

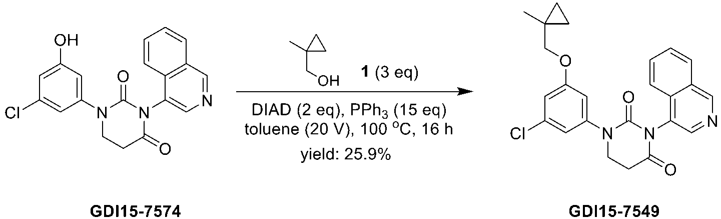

GDI15-7574 (30 mg, 81.6 µmol, 1 eq) and PPh₃ (320 mg, 1.22 mmol, 15 eq) were dissolved in toluene (0.6 mL). Compound 1 (21.08 mg, 245 µmol, 23.4 µL, 3 eq) and DIAD (33.0 mg, 163 µmol, 31.6 µL, 2 eq) were added at 25°C. The reaction mixture was allowed to react at 100°C for 16 hours under nitrogen atmosphere, and the reaction ended as monitored by LCMS. The reaction solution was added into water (5 mL) and extracted with EtOAc (2 mL × 3). The organic phases were combined, dried over anhydrous magnesium sulfate, filtered, and evaporated to dryness. Then the residue was purified by high performance liquid chromatography (column: Waters Xbridge BEH C18 100*30 mm*10 µm; mobile phase: [H₂O (10 mM NH₄HCO₃)-ACN]; gradient: 40%-70%, 8.0 min) to afford the target compound GDI15-7549 (9.2 mg, 21.1 µmol, 25.9% yield) as a white solid. ESI-MS:[M+H]⁺, 435.8.

¹H NMR (400 MHz, DMSO) δ 9.36 (s, 1 H), 8.45 (s, 1 H), 8.23 (d, *J* = 8.0 Hz, 1 H), 7.94 (d, *J* = 8.4 Hz, 1 H), 7.88 - 7.79 (m, 1 H), 7.77 - 7.72 (m, 1 H), 7.11 (t, *J* = 1.8 Hz, 1 H), 7.01 (t, *J* = 2.1 Hz, 1 H), 6.92 (t, *J* = 2.1 Hz, 1 H), 4.18 - 4.06 (m, 2 H), 3.79 (s, 2 H), 3.27 - 3.20 (m, 1 H), 3.15 - 3.03 (m, 1 H), 1.16 (s, 3 H), 0.56 - 0.48 (m, 2 H), 0.44 - 0.35 (m, 2 H).

### Example 7: Preparation of Compound GDI15-7546

### (1) Synthesis steps of compound 2

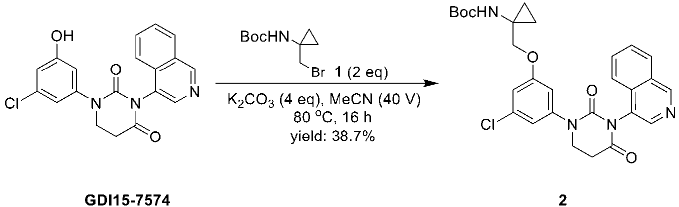

GDI15-7574 (50 mg, 136 µmol, 1 eq) and compound 1 (68.0 mg, 272 µmol, 2 eq) were dissolved in MeCN (2 mL). K₂CO₃ (75.2 mg, 544 µmol, 4 eq) was added at 25°C. The reaction mixture was allowed to react at 80°C for 16 hours, and the reaction ended as monitored by LCMS. The reaction solution was added into water (5 mL) and extracted with EtOAc (2 mL × 3). The organic phases were combined, dried over anhydrous magnesium sulfate, filtered, and evaporated to dryness. The residue was purified by thin-layer chromatography (Petroleum ether/EtOAc = 1:2) to afford the target compound 2 (35 mg, 52.6 µmol, 38.7% yield, 78.4% purity) as a yellow oil. ESI-MS:[M+H]⁺, 536.8.

### (2) Synthesis steps of compound GDI15-7546

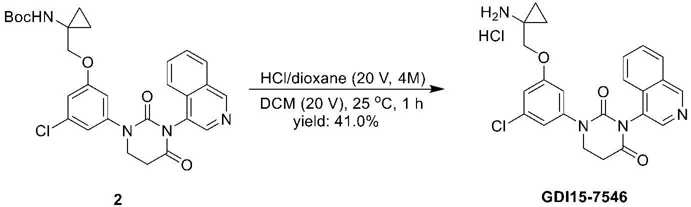

Compound 2 (30 mg, 55.9 µmol, 1 eq) was dissolved in DCM (0.6 mL) and HCl in 1,4-dioxane (0.6 mL, 4 M). The reaction mixture was allowed to react at 25°C for 1 hour, and the reaction ended as monitored by LCMS. The reaction mixture was concentrated under reduced pressure and purified by high performance liquid chromatography (column: Phenomenex Luna C18 75*30 mm*3 µm; mobile phase: [water (0.04% HCl)-ACN]; gradient: 1%-36%, 8.0 min) to afford the target compound GDI15-7546 (10.9 mg, 22.9 µmol, 41.0% yield, 99.5% purity, HCl) as a white solid. ESI-MS: [M+H]⁺, 436.8.

¹H NMR (400 MHz, DMSO) δ 9.43 (s, 1 H), 8.51 (d, *J=* 6.8 Hz, 4 H), 8.28 (d, *J* = 8.4 Hz, 1 H), 7.98 (d, *J =* 8.5 Hz, 1 H), 7.88 (t, *J =* 7.5 Hz, 1 H), 7.80 - 7.74 (m, 1 H), 7.20 (t, *J =* 1.6 Hz, 1 H), 7.08 (t, *J* = 2.1 Hz, 1 H), 6.99 (t, *J =* 1.9 Hz, 1 H), 4.16 - 4.08 (m, 4 H), 3.34 - 3.19 (m, 2 H), 3.15 - 3.04 (m, 1 H), 1.09 - 1.04 (m, 2 H), 0.96 - 0.89 (m, 2 H).

### Example 8: Preparation of Compound GDI15-7547

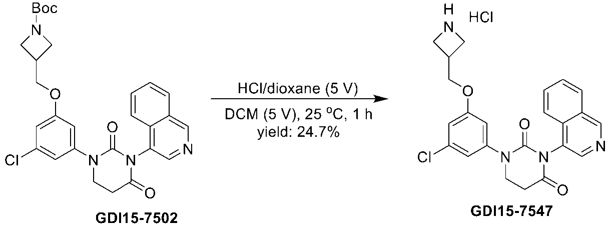

Compound GDI15-7502 (90 mg, 167 µmol, 1 eq) was dissolved in DCM (0.45 mL) and 4M HCl in 1,4-dioxane (0.45 mL). The reaction mixture was allowed to react at 25°C for 1 hour, and the reaction ended as monitored by LCMS. The reaction mixture was concentrated under reduced pressure and purified by high performance liquid chromatography (column: Phenomenex Luna C18 75*30 mm*3 µm; mobile phase: [water (0.04% HCl)-ACN]; gradient: 1%-36%, 8.0 min) to afford the target compound GDI15-7547 (21 mg, 41.4 µmol, 24.7% yield, 93.4% purity, HCl) as a white solid. ESI-MS: [M+H]⁺, 437.2.

1H NMR (400 MHz, DMSO) δ 9.55 (s, 1 H), 9.16 - 8.96 (m, 2 H), 8.59 (s, 1 H), 8.35 (d, *J* = 8.1 Hz, 1 H), 8.06 (d, *J =* 8.4 Hz, 1 H), 7.95 (t, *J =* 7.5 Hz, 1 H), 7.88 - 7.78 (m, 1 H), 7.18 (t, *J =* 1.8 Hz, 1 H), 7.09 (t, *J* = 2.0 Hz, 1 H), 7.03 (t, *J* = 2.0 Hz, 1 H), 4.19 (d, *J* = 5.8 Hz, 2 H), 4.16 - 4.11 (m, 2 H), 4.09 - 4.02 (m, 3 H), 3.80 - 3.75 (m, 2 H), 3.27 (ddd, *J =* 5.8, 8.0, 16.7 Hz, 1 H), 3.21 - 3.13 (m, 1 H), 3.13 - 3.04 (m, 1 H).

### Example 9: Preparation of Compound GDI15-7502

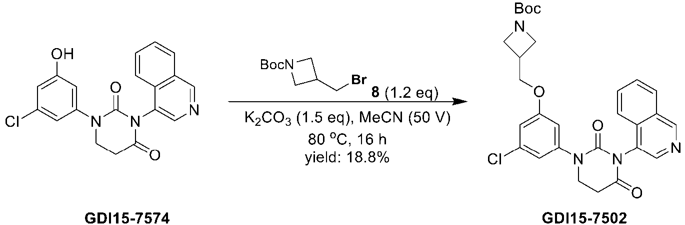

Compound GDI15-7574 (20 mg, 54.3 µmol, 1 eq) and K₂CO₃ (11.2 mg, 81.5 µmol, 1.5 eq) were dissolved in MeCN (1 mL). Compound 8 (16.3 mg, 65.2 µmol, 1.2 eq) was added at 25°C. The reaction mixture was allowed to react at 80°C for 16 hours, and the reaction ended as monitored by LCMS. The reaction solution was filtered and the filtrate was purified by high performance liquid chromatography (column: Waters Xbridge BEH C18 100*30 mm*10 µm; mobile phase: [H₂O (10 mM NH₄HCO₃)-ACN]; gradient: 40%-75%, 8.0 min) to afford the target compound GDI15-7502 (5.5 mg, 10.2 µmol, 18.8% yield) as a white solid. ESI-MS:[M+H]⁺, 537.2.

¹H NMR (400 MHz, CDCl₃) δ 9.36 (s, 1 H), 8.50 (s, 1 H), 8.16 (d, *J =* 8.3 Hz, 1 H), 7.89 - 7.85 (m, 1 H), 7.77 - 7.70 (m, 2 H), 7.27 (s, 7 H), 7.01 (s, 1 H), 6.90 (s, 1 H), 6.83 (s, 1 H), 4.14 (td, *J* = 6.7, 9.1 Hz, 2 H), 4.10 - 4.07 (m, 4 H), 3.77 (dd, *J =* 5.1, 8.7 Hz, 2 H), 3.25 - 3.19 (m, 1 H), 2.97 - 2.91 (m, 1 H), 1.45 (s, 9 H).

### Example 10: Preparation of Compound GDI15-7649

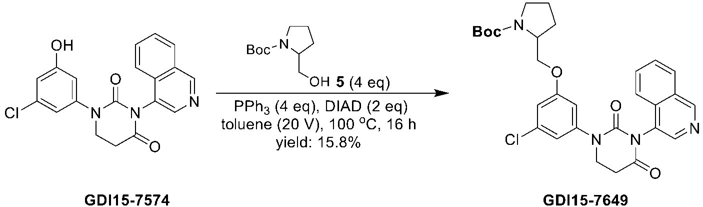

GDI15-7574 (110 mg, 299 µmol, 1 eq) was dissolved in toluene (2.2 mL). PPh₃ (314 mg, 1.20 mmol, 4 eq) and compound 3 (314 mg, 1.20 mmol, 4 eq) were added at 25°C. DIAD (120 mg, 598 µmol, 116 µL, 2 eq) was added dropwise under nitrogen atmosphere. The reaction mixture was allowed to react at 100°C for 16 hours under nitrogen atmosphere, and the reaction ended as monitored by LCMS. The reaction solution was added to water (5 mL) and extracted with EtOAc (2 mL × 3). The organic phases were combined, dried over anhydrous magnesium sulfate, filtered, and evaporated to dryness. The residue was purified by thin-layer chromatography (Petroleum ether/EtOAc = 1:2) to afford GDI15-7649 (80 mg). 30 mg of the compound was taken and purified by high performance liquid chromatography (column: Waters Xbridge BEH C18 100*30 mm*10 µm; mobile phase: [H₂O (10 mM NH₄HCO₃)-ACN]; gradient: 35%-70%, 8.0 min) to afford the target compound GDI15-7649 (15.9 mg, 34.4 µmol, 15.8% yield) as a white solid. ESI-MS:[M+H]⁺, 551.1.

¹H NMR (400 MHz, DMSO) δ 9.36 (s, 1 H), 8.45 (s, 1 H), 8.23 (d, *J* = 8.0 Hz, 1 H), 7.94 (d, *J* = 8.3 Hz, 1 H), 7.83 (t, *J =* 7.4 Hz, 1 H), 7.78 - 7.72 (m, 1 H), 7.14 (s, 1H), 7.05 (d, *J =* 14.4 Hz, 1 H), 6.99 (s, 1 H), 4.16 - 4.03 (m, 3 H), 3.98 (dd, *J* = 6.9, 18.4 Hz, 2 H), 3.29 - 3.20 (m, 3 H), 3.13 - 3.02 (m, 1 H), 2.03 - 1.77 (m, 4 H), 1.40 (s, 9 H).

### Example 11: Preparation of Compound GDI15-7548

### (1) Synthesis steps of compound 2

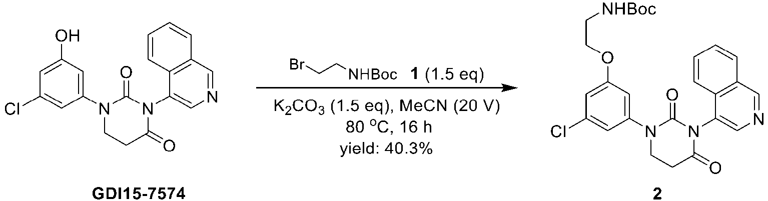

GDI15-7574 (200 mg, 544 µmol, 1 eq) and compound 1 (183 mg, 816 µmol, 1.5 eq) were dissolved in MeCN (4 mL). K₂CO₃ (113 mg, 816 µmol, 1.5 eq) was added at 25°C. The reaction mixture was allowed to react at 80°C for 16 hours, and the reaction ended as monitored by LCMS. The reaction solution was added to water (5 mL) and extracted with EtOAc (2 mL × 3). The organic phases were combined, dried over anhydrous magnesium sulfate, filtered, and evaporated to dryness. The residue was purified by thin-layer chromatography (Petroleum ether/EtOAc = 1:2) to afford the target compound 2 (160 mg, 219 µmol, 40.3% yield, 70% purity) as a yellow solid. ESI-MS: [M+H]⁺, 511.2.

### (2) Synthesis steps of compound GDI15-7548

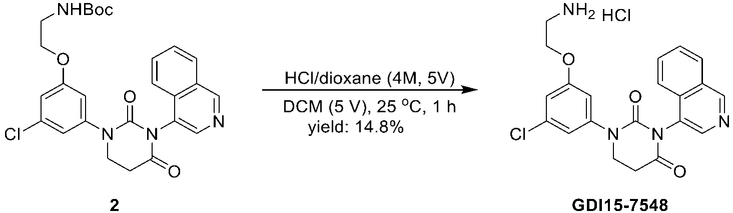

Compound 2 (160 mg, 313 µmol, 1 eq) was dissolved in DCM (0.8 mL) and 4M HCl in 1,4-dioxane (0.8 mL). The reaction mixture was allowed to react at 25°C for 1 hour, and the reaction ended as monitored by LCMS. The reaction mixture was concentrated under reduced pressure to afford the crude product (160 mg). A portion of the crude product GDI15-7548 (60 mg) was taken and purified by high performance liquid chromatography (column: Phenomenex Luna C18 75*30 mm*3 µm; mobile phase: [water (0.04% HCl)-ACN]; gradient: 1%-36%, 8.0 min) to afford the target compound GDI15-7548 (21 mg, 46.4 µmol, 14.8% yield, 98.9% purity, HCl) as a white solid. ESI-MS: [M+H]⁺, 437.2.

¹H NMR (400 MHz, DMSO) δ 9.49 (s, 1 H), 8.55 (s, 1 H), 8.32 (d, *J* = 8.1 Hz, 1 H), 8.13 (s, 3 H), 8.03 (d, *J =* 8.5 Hz, 1 H), 7.96 - 7.89 (m, 1 H), 7.86 - 7.76 (m, 1 H), 7.20 (t, *J =* 1.8 Hz, 1 H), 7.07 (t, *J =* 2.1 Hz, 1 H), 7.00 (t, *J* = 2.0 Hz, 1 H), 4.22 (t, *J =* 5.0 Hz, 2 H), 4.15 - 4.08 (m, 2 H), 3.31 - 3.18 (m, 3 H), 3.12 - 3.00 (m, 1 H).

### Example 12: Preparation of Compound GDI15-7501

### (1) Synthesis steps of compound 3

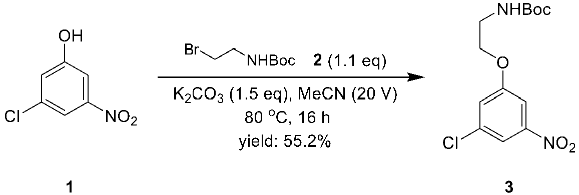

Compound 1 (1g, 5.76 mmol, 1 eq) and K₂CO₃ (1.19g, 8.64 mmol, 1.5 eq) were dissolved in CH₃CN (20 mL). Compound 2 (1.42g, 6.34 mmol, 1.1 eq) was added at 20°C. The reaction mixture was allowed to react at 80°C for 16 hours, and the reaction ended as monitored by LCMS. The reaction solution was directly evaporated to dryness, and purified by column chromatography (SiO₂, Petroleum ether/EtOAc = 10:1 to 5:1) to afford the target compound 3 (1.2g, 3.18 mmol, 55.2% yield, 84% purity) as a yellow solid. ESI-MS: [M+H]⁺, 317.0.

### (2) Synthesis steps of compound 4

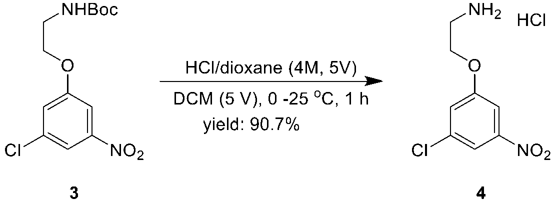

Compound 3 (1.2g, 3.79 mmol, 1 eq) was dissolved in DCM (6 mL), and 4 M HCl in 1,4-dioxane (6 mL) was added at 0°C. The reaction mixture was allowed to react at 25°C for 1 hour, and the reaction ended as monitored by LCMS. The reaction solution was concentrated directly to afford the target compound 4 (0.87g, 3.44 mmol, 90.7% yield, HCl) as a white solid. ESI-MS: [M+H] ⁺, 217.0.

### (3) Synthesis steps of compound 5

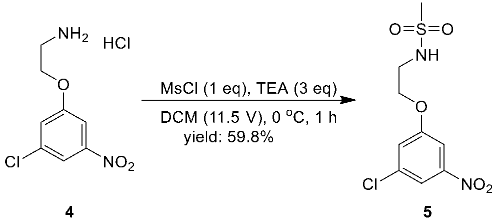

Compound 4 (0.87g, 3.44 mmol, 1 eq, HCl) was dissolved in DCM (10 mL). TEA (1.04g, 10.3 mmol, 1.44 mL, 3 eq) and MsCl (0.390g, 3.40 mmol, 264 µL, 1 eq) were added successively at 0°C. The reaction mixture was allowed to react at 0°C for 1 hour. The reaction ended as monitored by LCMS. The reaction solution was added into ice water (15 mL) and the resulting mixture was extracted with DCM (6 mL × 3). The organic phases were combined, filtered, and concentrated under reduced pressure. The residue was purified by column chromatography (SiO₂, Petroleum ether/EtOAc = 20:1 to 10:1) to afford the target compound 5 (0.64g, 2.06 mmol, 59.8% yield, 94.7% purity) as a white solid. ESI-MS:[M+H]⁺, 295.0.

### (4) Synthesis steps of compound 6

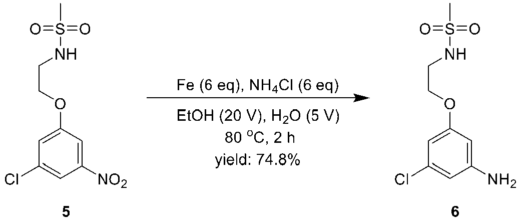

Compound 5 (0.64g, 2.17 mmol, 1 eq) was dissolved in EtOH (13 mL) and H₂O (3.2 mL). NH₄Cl (697 mg, 13.0 mmol, 6 eq) was added at 20°C, and then the mixture was heated to 80°C. Iron powder (728 mg, 13.0 mmol, 6 eq) was added portionwise. The reaction mixture was allowed to react at 80°C for 2 hours, and the reaction ended as monitored by LCMS. The reaction solution was filtered through Celite while hot, and the filter cake was washed with hot EtOH (20 mL). The filtrate was concentrated under reduced pressure and evaporated to dryness, and the residue was purified by thin-layer chromatography (Petroleum ether/EtOAc = 1:1) to afford compound 6 (0.5g, 1.62 mmol, 74.8% yield, 86% purity) as a white solid. ESI-MS:[M+H]⁺, 265.1.

### (5) Synthesis steps of compound 8

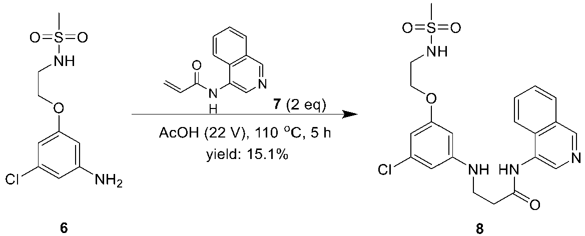

Compound 6 (0.45g, 1.70 mmol, 1 eq) was dissolved in AcOH (1 mL). Compound 7 (674 mg, 3.40 mmol, 2 eq) was added at 25°C. The reaction mixture was allowed to react at 110°C for 5 hours, and the reaction ended as monitored by LCMS. The reaction solution was evaporated to dryness under reduced pressure. Ice water (3 mL) was added to the residue, and the mixture was extracted with EtOAc (1 mL × 3). The organic phases were combined, concentrated under reduced pressure and evaporated to dryness. The residue was purified by thin-layer chromatography (Petroleum ether/EtOAc = 0:1) to afford the target compound 8 (150 mg, 256 µmol, 15.1% yield, 79% purity) as a yellow solid. ESI-MS:[M+H]⁺, 463.1.

### (6) Synthesis steps of compound GDI15-7501

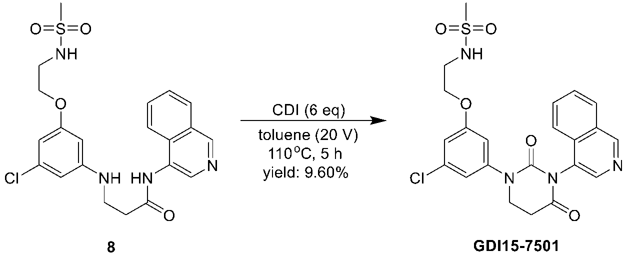

Compound 8 (0.15g, 324 µmol, 1 eq) was dissolved in toluene (3 mL). CDI (315 mg, 1.94 mmol, 6 eq) was added at 25°C. The reaction mixture was allowed to react at 110°C for 5 hours, and the reaction ended as monitored by LCMS. The reaction solution was evaporated to dryness under reduced pressure. Ice water (3 mL) was added to the residue, and the mixture was extracted with EtOAc (1 mL × 3). The organic phases were combined, concentrated under reduced pressure and evaporated to dryness. The residue was purified by thin-layer chromatography (Ethyl acetate/MeOH = 10:1) and high performance liquid chromatography (column: Waters Xbridge BEH C18 100*30 mm*10 µm; mobile phase: [H₂O (10 mM NH₄HCO₃)-ACN]; gradient: 21%-51%, 8.0 min) to afford compound GDI15-7501 (0.016g, 31.1 µmol, 9.60% yield, 95.0% purity) as a white solid. ESI-MS:[M+H]⁺, 488.9.

¹H NMR (400 MHz, DMSO) δ 9.36 (s, 1 H), 8.45 (s, 1 H), 8.23 (d, *J =* 8.1 Hz, 1 H), 7.94 (d, *J = 8.4* Hz, 1 H), 7.83 (dt, *J =* 1.1, 7.6 Hz, 1 H), 7.75 (d, *J =* 7.3 Hz, 1 H), 7.37 - 7.23 (m, 1 H), 7.15 (t, *J* = 1.7 Hz, 1 H), 7.04 (t, *J =* 1.9 Hz, 1 H), 6.97 (t, *J =* 1.9 Hz, 1 H), 4.15 - 4.10 (m, 2 H), 4.07 (t, *J =* 5.4 Hz, 2 H), 3.25 (ddd, *J =* 6.1, 7.9, 16.6 Hz, 3 H), 3.12 - 3.03 (m, 1 H), 2.94 (s, 3 H).

### Example 13: Preparation of Compound GDI15-7611

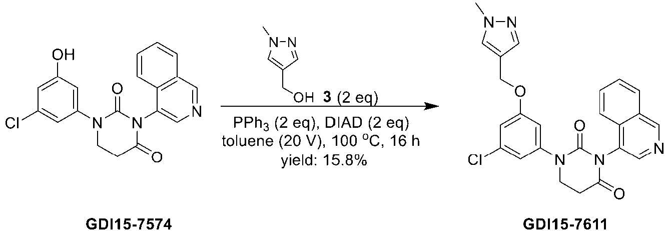

GDI15-7574 (80 mg, 218 µmol, 1 eq) was dissolved in toluene (1.6 mL). PPh₃ (114 mg, 436 µmol, 2 eq) and compound 3 (48.8 mg, 435 µmol, 2 eq) were added at 25°C. DIAD (87.9 mg, 435 µmol, 84.3 µL, 2 eq) was added dropwise under nitrogen atmosphere. The reaction mixture was allowed to react at 100°C for 16 hours under nitrogen atmosphere, and the reaction ended as monitored by LCMS. The reaction solution was added to water (5 mL) and extracted with EtOAc (2 mL × 3). The organic phases were combined, dried over anhydrous magnesium sulfate, filtered, and evaporated to dryness under reduced pressure. The residue was purified by thin-layer chromatography (Petroleum ether/EtOAc = 1:2) and high performance liquid chromatography (column: 3_Phenomenex Luna C18 75*30 mm*3 µm; mobile phase: [H₂O (0.2% FA)-ACN]; gradient: 30%-60%, 8.0 min) to afford the target compound GDI15-7611 (15.9 mg, 34.4 µmol, 15.8% yield) as a white solid. ESI-MS:[M+H]⁺, 462.0.

¹H NMR (400 MHz, DMSO) δ 9.36 (s, 1H), 8.45 (s, 1H), 8.23 (d, *J =* 8.1 Hz, 1H), 7.94 (d, *J* = 8.4 Hz, 1H), 7.83 (dt, *J* = 1.1, 7.7 Hz, 1H), 7.78 (s, 2H), 7.49 (s, 1H), 7.12 (t, *J* = 1.8 Hz, 1H), 7.06 (t, *J* = 2.0 Hz, 1H), 7.01 (t, *J =* 2.1 Hz, 1H), 4.99 (s, 2H), 4.22 - 4.05 (m, 2H), 3.80 (s, 3H), 3.24 - 3.05 (m, 2H).

### Example 14: Preparation of Compound GDI15-7612

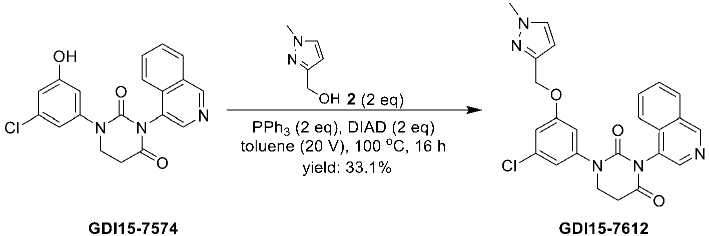

GDI15-7574 (80 mg, 218 µmol, 1 eq) was dissolved in toluene (1.6 mL). PPh₃ (114 mg, 436 µmol, 2 eq) and compound 2 (48.8 mg, 435 µmol, 2 eq) were added sequentially at 25°C. DIAD (87.9 mg, 435 µmol, 84.3 µL, 2 eq) was added dropwise under nitrogen atmosphere. The reaction mixture was allowed to react at 100°C for 16 hours under nitrogen atmosphere, and the reaction ended as monitored by LCMS. The reaction solution was added to water (5 mL) and extracted with EtOAc (2 mL × 3). The organic phases were combined, dried over anhydrous magnesium sulfate, filtered, and evaporated to dryness under reduced pressure. The residue was purified by thin-layer chromatography (Petroleum ether/EtOAc = 1:2) and high performance liquid chromatography (column: 3_Phenomenex Luna C18 75*30 mm*3 µm; mobile phase: [H₂O (0.2% FA)-ACN]; gradient: 30%-60%, 8.0 min) to afford the target compound GDI15-7612 (33.4 mg, 71.9 µmol, 33.1% yield, 99.5% purity) as a white solid. ESI-MS:[M+H]⁺, 462.1.

¹H NMR (400 MHz, DMSO) δ 9.36 (s, 1 H), 8.45 (s, 1 H), 8.23 (d, *J =* 8.1 Hz, 1 H), 7.94 (d, *J* = 8.4 Hz, 1 H), 7.83 (ddd, *J* = 1.2, 7.0, 8.3 Hz, 1 H), 7.74 (dt, *J =* 1.0, 7.5 Hz, 1 H), 7.66 (d, *J* = 2.1 Hz, 1 H), 7.15 - 7.11 (m, 1 H), 7.09 (t, *J* = 2.1 Hz, 1 H), 7.06 - 7.00 (m, 1 H), 6.30 (d, *J* = 2.3 Hz, 1 H), 5.04 (s, 2 H), 4.16 - 4.04 (m, 2 H), 3.81 (s, 3 H), 3.25 - 3.04 (m, 2 H).

### Example 15: Preparation of Compound GDI15-7613

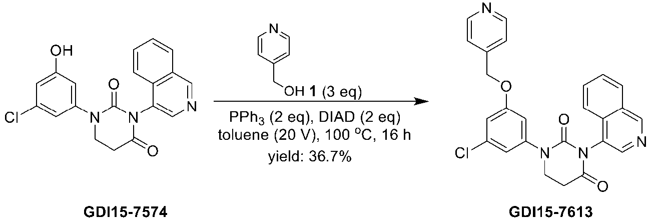

GDI15-7574 (80 mg, 218 µmol, 1 eq) was dissolved in toluene (1.6 mL). PPh₃ (114 mg, 436 µmol, 2 eq) and compound 1 (71.2 mg, 652 µmol, 3 eq) were added sequentially at 25°C. DIAD (87.9 mg, 435 µmol, 84.3 µL, 2 eq) was added dropwise under nitrogen atmosphere. The reaction mixture was allowed to react at 100°C for 16 hours under nitrogen atmosphere, and the reaction ended as monitored by LCMS. The reaction solution was added to water (5 mL) and extracted with EtOAc (2 mL × 3). The organic phases were combined, dried over anhydrous magnesium sulfate, filtered, and evaporated to dryness under reduced pressure. The residue was purified by thin-layer chromatography (Petroleum ether/EtOAc = 1:2) and high performance liquid chromatography (column: 3_Phenomenex Luna C18 75*30 mm*3 µm; mobile phase: [H₂O (0.2% FA)-ACN]; gradient: 30%-60%, 8.0 min) to afford the target compound GDI15-7613 (36.6 mg, 79.8 µmol, 36.7% yield) as a white solid. ESI-MS:[M+H]⁺, 459.1.

¹H NMR (400 MHz, DMSO) δ 9.36 (s, 1 H), 8.59 (d, *J* = 5.8 Hz, 2 H), 8.45 (s, 1 H), 8.23 (d, *J* = 8.1 Hz, 1 H), 7.94 (d, *J* = 8.4 Hz, 1 H), 7.83 (dt, *J* = 1.1, 7.6 Hz, 1 H), 7.79 - 7.69 (m, 1 H), 7.44 (d, *J* = 5.8 Hz, 2 H), 7.20 - 7.16 (m, 1 H), 7.14 (t, *J* = 2.0 Hz, 1 H), 7.06 (t, *J* = 2.0 Hz, 1 H), 5.23 (s, 2 H), 4.21 - 4.02 (m, 2 H), 3.23 - 3.00 (m, 2 H).

### Example 16: Preparation of Compound GDI15-7520

### (1) Synthesis steps of compound 3

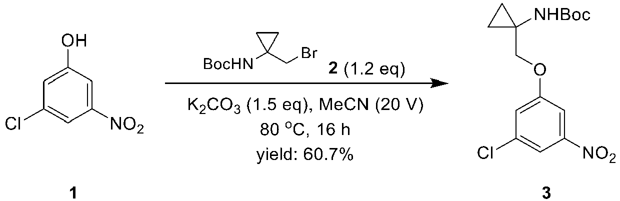

Compound 1 (400 mg, 2.30 mmol, 1 eq) and compound 2 (691 mg, 2.77 mmol, 1.2 eq) were dissolved in MeCN (8 mL). K₂CO₃ (477 mg, 3.46 mmol, 1.5 eq) was added at 25°C. The reaction mixture was allowed to react at 80°C for 16 hours, and the reaction ended as monitored by LCMS. The reaction solution was added into water (50 mL) and extracted with EtOAc (20 mL × 3). The organic phases were combined, dried over anhydrous magnesium sulfate, filtered, and evaporated to dryness under reduced pressure. The residue was purified by thin-layer chromatography (Petroleum ether/EtOAc = 1:1) to afford the target compound 3 (480 mg, 1.40 mmol, 60.7% yield) as a yellow oil. ESI-MS: [M+H] ⁺, 343.2.

### (2) Synthesis steps of compound 5

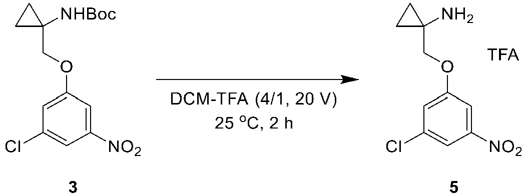

Compound 3 (480 mg, 1.40 mmol, 1 eq) was dissolved in DCM (8 mL) TFA (2 mL). The reaction mixture was allowed to react at 25°C for 2 hours, and the reaction ended as monitored by LCMS. The reaction mixture was concentrated under reduced pressure to afford the target compound 5 (660 mg, TFA, crude) as a yellow oil. ESI-MS: [M+H]⁺, 357.2.

### (3) Synthesis steps of compound 7

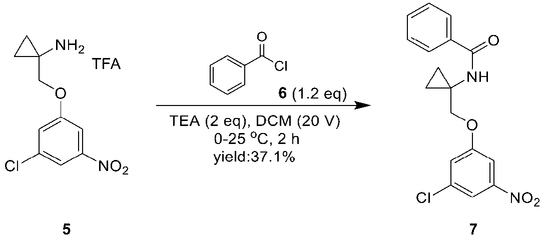

Compound 5 (0.66g, 2.72 mmol, 1 eq) was dissolved in DCM (13 mL). TEA (550 mg, 5.44 mmol, 757 µL, 2 eq) and compound 6 (458 mg, 3.26 mmol, 378 µL, 1.2 eq) were added slowly at 0°C. The reaction mixture was allowed to react at 25°C for 2 hours, and the reaction ended as monitored by LCMS. The reaction solution was added into ice water (30 mL) and extracted with DCM (10 mL × 3). The organic phases were combined, dried over anhydrous magnesium sulfate, filtered, and evaporated to dryness under reduced pressure. The residue was purified by thin-layer chromatography (Petroleum ether/EtOAc = 0:1) to afford the target compound 7 (350 mg, 1.01 mmol, 37.1% yield) as a yellow oil. ESI-MS: [M+H]⁺, 347.2.

### (4) Synthesis steps of compound 8

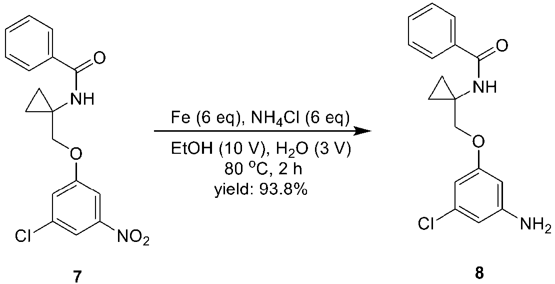

Compound 7 (350 mg, 1.01 mmol, 1 eq) and NH₄Cl (323 mg, 6.06 mmol, 6 eq) were dissolved in EtOH (3.5 mL) and H₂O (1.1 mL). Fe (338 mg, 6.06 mmol, 6 eq) was added portionwise at 80°C. The reaction mixture was stirred and allowed to react at 80°C for 2 hours, and the reaction ended as monitored by LCMS. Celite was added and the resulting mixture was filtered while hot, and then the filter cake was washed with hot ethanol (200 mL). The filtrate was filtered and evaporated to dryness under reduced pressure. The residue was purified by thin-layer chromatography (Petroleum ether/EtOAc = 5:1) to afford the target compound 8 (300 mg, 947 µmol, 93.8% yield) as a yellow oil. ESI-MS: [M+H]⁺, 317.2.

### (5) Synthesis steps of compound 9

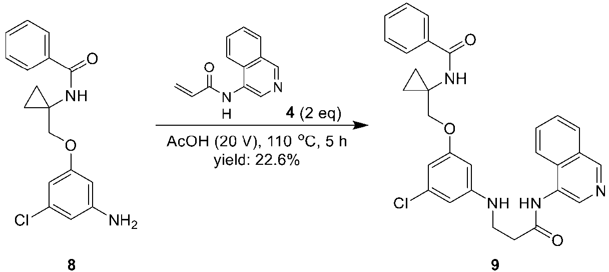

Compound 8 (300 mg, 947 µmol, 1 eq) and compound 4 (375 mg, 1.89 mmol, 2 eq) were dissolved in AcOH (6 mL). The reaction mixture was allowed to react at 110°C for 5 hours, and the reaction ended as monitored by LCMS. The reaction solution was added into ice water (20 mL) and extracted with EtOAc (10 mL × 3). The organic phases were combined, dried over anhydrous magnesium sulfate, filtered, and evaporated to dryness under reduced pressure. The residue was purified by thin-layer chromatography (Petroleum ether/EtOAc = 0:1) to afford the target compound 9 (120 mg, 233 µmol, 22.6% yield) as a yellow oil. ESI-MS:[M+H]⁺, 516.2.

### (6) Synthesis steps of compound GDI15-7520

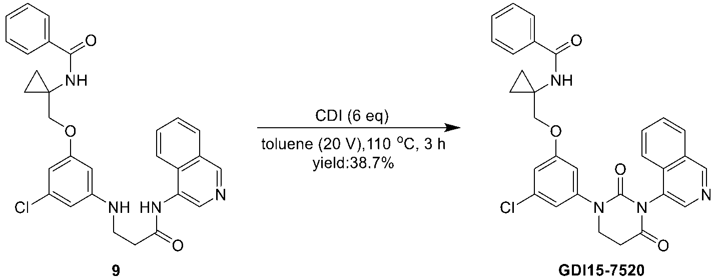

Compound 9 (120 mg, 233 µmol, 1 eq) and CDI (226 mg, 1.40 mmol, 6 eq) were dissolved in toluene (2.4 mL). The mixture was stirred and allowed to react at 110°C for 3 hours, and the reaction ended as monitored by LCMS. The reaction mixture was concentrated under reduced pressure and purified by high performance liquid chromatography (column: Waters Xbridge BEH C18 100*30 mm*10 µm; mobile phase: [H₂O (10 mM NH₄HCO₃)-ACN]; gradient: 35%-65%, 8.0 min) to afford the target compound GDI15-7520 (56 mg, 90.4 µmol, 38.7% yield) as a white solid. ESI-MS: [M+H]⁺, 542.2.

¹H NMR (400 MHz, DMSO) δ 9.36 (s, 1 H), 8.85 (s, 1 H), 8.44 (s, 1 H), 8.23 (d, *J* = 8.3 Hz, 1 H), 7.93 (d, *J* = 8.4 Hz, 1 H), 7.85 - 7.80 (m, 3 H), 7.77 - 7.71 (m, 1 H), 7.53 - 7.46 (m, 1 H), 7.45 - 7.39 (m, 2 H), 7.10 (t, *J* = 1.6 Hz, 1 H), 7.01 (t, *J* = 1.9 Hz, 1 H), 6.92 (t, *J* = 1.9 Hz, 1 H), 4.19 (s, 2 H), 4.12 - 4.02 (m, 2 H), 3.26 - 3.16 (m, 1 H), 3.09 - 2.97 (m, 1 H), 0.95 - 0.87 (m, 4 H).

### Example 17: Preparation of Compound GDI15-7617

### (1) Synthesis steps of compound 2

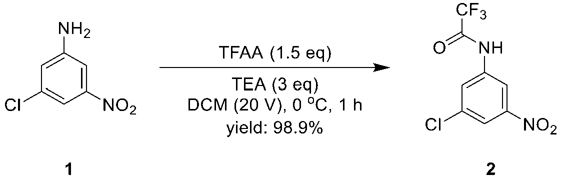

Compound 1 (500 mg, 2.90 mmol, 1 eq) and TEA (879 mg, 8.69 mmol, 1.21 mL, 3 eq) were dissolved in DCM (10 mL). TFAA (912 mg, 4.35 mmol, 604 µL, 1.5 eq) was added dropwise at 0°C. The reaction mixture was allowed to react at 0°C for 1 hour, and the reaction ended as monitored by LCMS. The reaction solution was diluted with water (30 mL) and extracted with EtOAc (30 mL ×3). The organic phases were combined and washed with 50 mL of saturated sodium chloride solution. The combined organic phases were dried over anhydrous sodium sulfate, filtered, and evaporated to dryness under reduced pressure to afford the target compound 2 (770 mg, 2.87 mmol, 98.9% yield) as a yellow oil. ESI-MS:[M+H]⁺, 317.3.

### (2) Synthesis steps of compound 3

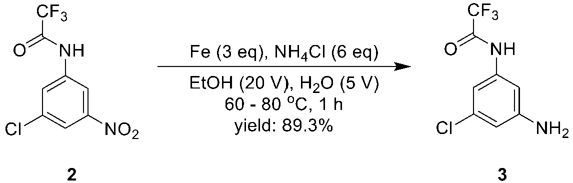

Compound 2 (770 mg, 2.87 mmol, 1 eq) was dissolved in EtOH (15.4 mL) and H₂O (4 mL). NH₄Cl (920 mg, 17.2 mmol, 6 eq) was added at 25°C. After warming to 60°C, iron powder (480 mg, 8.60 mmol, 3 eq) was added portionwise to the above reaction solution. The reaction mixture was allowed to react at 80°C for 1 hour, and the reaction ended as monitored by LCMS. The reaction solution was filtered through celite while hot. The filtrate was concentrated under reduced pressure, diluted with water (30 mL), and then extracted with EtOAc (30 mL ×3). The organic phases were combined and washed with 50 mL of saturated sodium chloride solution. The combined organic phases were dried over anhydrous sodium sulfate, filtered, and evaporated to dryness under reduced pressure to afford the target compound 3 (710 mg, 2.56 mmol, 89.3% yield, 86% purity) as a yellow oil. ESI-MS:[M+H]⁺, 239.0.

### (3) Synthesis steps of compound 5

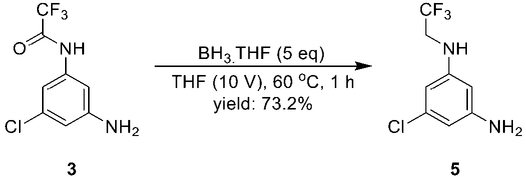

Compound 3 (710 mg, 2.98 mmol, 1 eq) was dissolved in THF (7.1 mL). 1M BH₃.THF (14.8 mL, 5 eq) was added dropwise at 25°C. The reaction mixture was allowed to react at 60°C for 1 hour, and the reaction ended as monitored by LCMS. The reaction solution was cooled to room temperature and saturated NH₄Cl solution (50 mL) was added dropwise to the reaction solution. The reaction solution was extracted with EtOAc (30 mL ×3). The organic phases were combined and washed with 50 mL of saturated sodium chloride. The combined organic phases were dried over anhydrous sodium sulfate, filtered, and evaporated to dryness under reduced pressure to afford the target compound 5 (670 mg, 2.18 mmol, 73.2% yield, 73% purity) as a yellow oil. ESI-MS:[M+H]⁺, 225.1.

### (4) Synthesis steps of compound 6

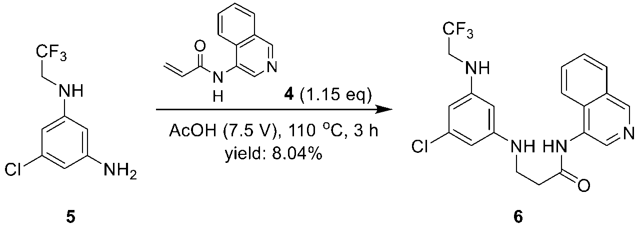

Compound 5 (670 mg, 2.18 mmol, 1 eq) and compound 4 (496 mg, 2.50 mmol, 1.15 eq) were dissolved in AcOH (5 mL). The mixture was allowed to react at 110°C for 3 hours, and the reaction ended as monitored by LCMS. The reaction solution was concentrated under reduced pressure, and then water (5 mL) was added. The reaction solution was extracted with EtOAc (3 mL ×3). The organic phases were combined and washed with 5 mL of saturated sodium chloride solution. The combined organic phases were dried over anhydrous sodium sulfate, filtered, and evaporated to dryness under reduced pressure. The residue was purified by thin-layer chromatography (Petroleum ether/EtOAc = 0:1) to afford the target compound 6 (74 mg, 175 µmol, 8.04% yield) as a yellow solid. ESI-MS:[M+H]⁺, 423.1.

### (5) Synthesis steps of compound GDI15-7617

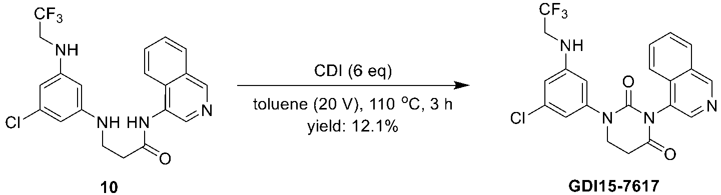

Compound 10 (74 mg, 175 µmol, 1 eq) was dissolved in toluene (1.5 mL). CDI (170 mg, 1.05 mmol, 6 eq) was added at 25°C. The reaction mixture was allowed to react at 110°C for 3 hours, and the reaction ended as monitored by LCMS. The reaction solution was concentrated under reduced pressure, and then water (3 mL) was added. The reaction solution was extracted with EtOAc (2 mL ×3). The organic phases were combined and washed with 5 mL of saturated sodium chloride solution. The combined organic phases were dried over anhydrous sodium sulfate, filtered, and evaporated to dryness under reduced pressure. The residue was purified by high performance liquid chromatography (column: Waters Xbridge BEH C18 100*30 mm*10 µm; mobile phase: [H₂O (10 mM NH₄HCO₃)-ACN]; gradient: 35%-65%, 8.0 min) to afford the target compound GDI15-7617 (9.5 mg, 21.2 µmol, 12.1% yield) as a yellow oil. ESI-MS:[M+H]⁺, 449.1.

¹H NMR (400 MHz, DMSO) δ 9.36 (s, 1 H), 8.44 (s, 1 H), 8.23 (d, *J* = 8.1 Hz, 1 H), 7.92 (d, *J =* 8.4 Hz, 1 H), 7.86 - 7.80 (m, 1 H), 7.78 - 7.71 (m, 1 H), 6.80 - 6.73 (m, 2 H), 6.73 - 6.63 (m, 2 H), 4.10 - 3.94 (m, 4 H), 3.25 - 3.18 (m, 1 H), 3.08 (s, 1 H).

### Example 18: Preparation of Compound GDI15-7550

### (1) Synthesis steps of compound 2

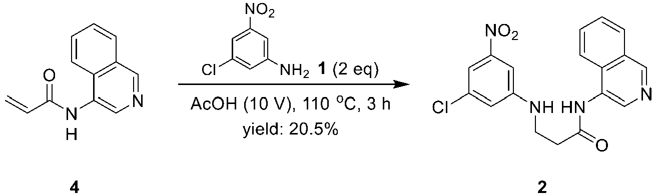

Compound 4 (2.15g, 10.8 mmol, 1 eq) and compound **1** (3.74g, 21.6 mmol, 2 eq) were dissolved in AcOH (21.5 mL). The reaction mixture was allowed to react at 110°C for 3 hours, and the reaction ended as monitored by LCMS. The reaction solution was concentrated under reduced pressure, and then water (50 mL) was added. The reaction solution was extracted with EtOAc (30 mL ×3). The organic phases were combined and washed with 50 mL of saturated sodium chloride solution. The combined organic phases were dried over anhydrous sodium sulfate, filtered, and evaporated to dryness under reduced pressure. The residue was purified by column chromatography (SiO₂, Petroleum ether/EtOAc = 20:1 to 1:1) to afford the target compound 2 (826 mg, 2.23 mmol, 20.5% yield) as a yellow solid. ESI-MS:[M+H]⁺, 371.0.

### (2) Synthesis steps of compound 3

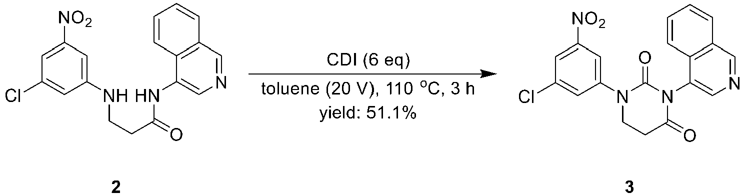

Compound 2 (820 mg, 2.21 mmol, 1 eq) was dissolved in toluene (16.4 mL). CDI (2.15g, 13.2 mmol, 6 eq) was added at 25°C. The reaction mixture was allowed to react at 110°C for 3 hours, and the reaction ended as monitored by LCMS. The reaction solution was concentrated under reduced pressure, and then water (30 mL) was added. The reaction solution was extracted with EtOAc (8 mL ×3). The organic phases were combined and washed with 30 mL of saturated sodium chloride solution. The combined organic phases were dried over anhydrous sodium sulfate, filtered, and evaporated to dryness under reduced pressure. The residue was purified by thin-layer chromatography (SiO₂, Petroleum ether/EtOAc = 0:1) to afford the target compound 3 (448 mg, 1.13 mmol, 51.1% yield) as a yellow oil. ESI-MS: [M+H]⁺, 397.1.

### (3) Synthesis steps of compound 5

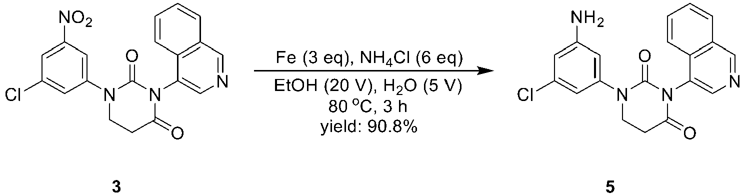

Compound 3 (448 mg, 1.03 mmol, 1 eq) was dissolved in EtOH (9 mL) and H₂O (2.3 mL). NH₄Cl (330 mg, 6.19 mmol, 6 eq) was added at 25°C. After warming to 80°C, iron (172 mg, 3.09 mmol, 3 eq) was added portionwise to the above mixture solution. The reaction mixture was allowed to react at 80°C for 3 hours, and the reaction ended as monitored by LCMS. Celite was added and the resulting mixture was filtered while hot, and then the filter cake was washed with hot ethanol (20 mL). The filtrate was concentrated under reduced pressure to afford the target compound 5 (380 mg, 936 µmol, 90.8% yield, 90.4% purity) as a yellow oil. ESI-MS: [M+H] ⁺, 367.1.

### (4) Synthesis steps of compound GDI15-7550

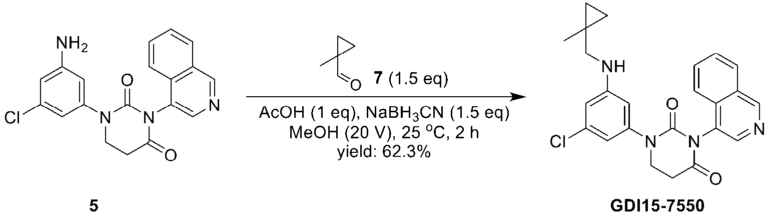

Compound 5 (70 mg, 190 µmol, 1 eq) and compound 7 (24.1 mg, 286 µmol, 1.5 eq) were dissolved in MeOH (1.4 mL). AcOH (11.4 mg, 190 µmol, 10.9 µL, 1 eq) was added at 25°C. After the reaction mixture was allowed to react at 25°C for 1 hour, NaBH₃CN (17.9 mg, 286 µmol, 1.5 eq) was added to the above mixture solution, and the reaction mixture was further allowed to react at 25°C for 1 hour, and the reaction ended as monitored by LCMS. The reaction solution was purified by high performance liquid chromatography (column: Waters Xbridge BEH C18 100*30 mm*10 µm; mobile phase: [H₂O (10 mM NH₄HCO₃)-ACN]; gradient: 35%-65%, 8.0 min) to afford the target compound GDI15-7550 (51.7 mg, 118 µmol, 62.3% yield) as a white solid. ESI-MS:[M+H]⁺, 435.2.

¹H NMR (400 MHz, DMSO) δ 9.36 (s, 1H), 8.45 (s, 1H), 8.24 (d, *J* = 8.0 Hz, 1H), 7.92 (d, *J* = 8.6 Hz, 1H), 7.83 (t, *J* = 7.1 Hz, 1H), 7.78 - 7.72 (m, 1H), 6.62 (d, *J* = 1.6 Hz, 2H), 6.52 (t, *J* = 1.8 Hz, 1H), 6.09 (t, *J* = 5.8 Hz, 1H), 4.05 (dt, *J* = 2.4, 6.5 Hz, 2H), 3.23 - 3.19 (m, 1H), 3.06 (d, *J* = 16.7 Hz, 1H), 2.89 (d, *J* = 5.3 Hz, 2H), 1.10 (s, 3H), 0.43 - 0.38 (m, 2H), 0.30 - 0.26 (m, 2H).

### Example 19: Preparation of Compound GDI15-7787

### (1) Synthesis steps of compound GDI15-7787

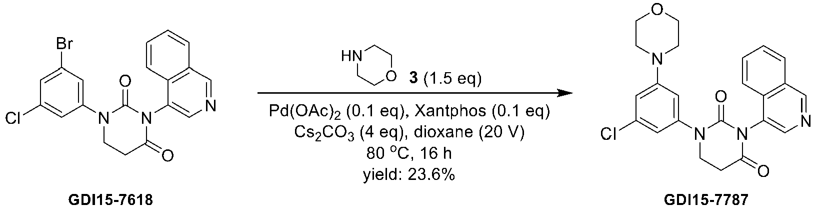

GDI15-7618 (50 mg, 116 µmol, 1 eq) and morpholine (15.2 mg, 174 µmol, 15.3 µL, 1.5 eq) were dissolved in 1,4-dioxane (1 mL). At 25°C, Cs₃CO₃ (228 mg, 870 µmol, 4 eq), Xantphos (6.72 mg, 11.6 µmol, 0.1 eq), and Pd(OAc)₂ (2.61 mg, 11.6 µmol, 0.1 eq) were added sequentially. The reaction mixture was allowed to react at 80°C for 16 hours under nitrogen atmosphere, and the reaction ended as monitored by LCMS. The reaction solution was added to water (5 mL) and extracted with EtOAc (2 mL × 3). The organic phases were combined and evaporated to dryness under reduced pressure. The residue was purified by thin-layer chromatography (SiO₂, EtOAc/MeOH = 10/1) and high performance liquid chromatography (column: Waters Xbridge BEH C18 100*30 mm*10 µm; mobile phase: [H₂O (10 mM NH₄HCO₃)-ACN]; gradient: 50%-80%, 8.0 min) to afford the target compound GDI15-7787 (24.6 mg, 54.8 µmol, 23.6% yield, 97.4% purity) as a white solid. ESI-MS:[M+H]⁺, 437.2.

¹H NMR (400 MHz, DMSO) δ 9.36 (s, 1 H), 8.44 (s, 1 H), 8.23 (d, *J* = 8.1 Hz, 1 H), 7.93 (d, *J* = 8.3 Hz, 1 H), 7.83 (s, 1 H), 7.75 (d, *J* = 7.9 Hz, 1 H), 7.00 - 6.82 (m, 3 H), 4.13 - 4.04 (m, 2 H), 3.74 - 3.70 (m, 4 H), 3.26 - 3.20 (m, 1 H), 3.18 - 3.12 (m, 4 H), 3.10 - 3.02 (m, 1 H).

### Example 20: Preparation of Compound GDI15-8096

### (1) Synthesis steps of compound 3

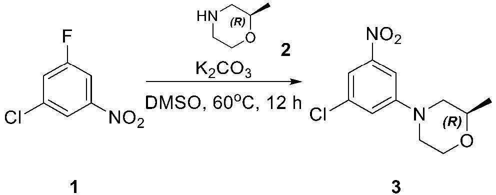

DMSO (10 mL) was added to compound 1 (1g, 5.7 mmol), and then compound 2 (0.63g, 6.2 mmol) and K₂CO₃ (2.36g, 17.1 mmol) were further added. The mixture was allowed to react at 60°C for 16 hours under N₂ atmosphere. After the reaction ended, water (50 mL) was added to the mixture, and the mixture was extracted with EA (50 mL ×3). The organic phases were combined and washed with saturated brine, dried over anhydrous sodium sulfate, filtered, and evaporated to dryness. The residue was further purified by column chromatography (EA/PE, 5% to 10%) to afford compound 3 (0.8g, 54.39% yield) as a yellow solid.

1H **NMR** (400 MHz, DMSO) δ 7.63 (d, *J* = 1.9 Hz, 1 H), 7.55 (s, 1 H), 7.43 (s, 1 H), 3.92 (m, 1 H), 3.81 (d, *J* = 12.1 Hz, 1 H), 3.69 (d, *J* = 12.2 Hz, 1 H), 3.60 (m, 2 H), 2.80 (m, 1 H), 2.45 (d, *J* = 10.8 Hz, 1 H), 1.16 (d, *J* = 6.2 Hz, 3 H).

### (2) Synthesis steps of compound 4

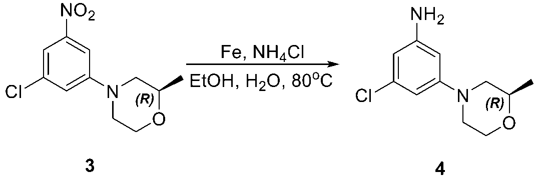

A mixed solution of EtOH/H₂O (1:1, 20 mL) was added to compound 3 (1.1g, 4.3 mmol), and then NH₄Cl (1.15g, 21.5 mmol) and iron (720 mg, 12.9 mmol) were further slowly added. The mixture was allowed to react at 80°C for 10 hours under nitrogen atmosphere. After the reaction ended, the mixture was cooled to room temperature, and filtered, and the solvent was removed in vacuo. Water was added to the residue, and the mixture was extracted with dichloromethane. The organic phases were combined, washed with saturated brine, dried over anhydrous sodium sulfate, filtered, and evaporated to dryness to afford compound 4 (880 mg, 90.70% yield) as a white solid. ESI-MS:[M+H]⁺, 227.15.

### (3) Synthesis steps of compound 6

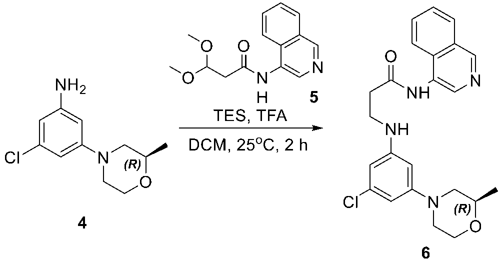

A solution of DCM (5 mL) was added to compound 4 (321 mg, 1.24 mmol), and then compound 5 (280 mg, 1.24 mmol), TFA (2.5 mL) and Et₃SiH (406 mg, 2.47 mmol) were further slowly added. The mixture was stirred and allowed to react at room temperature under nitrogen atmosphere for 16 hours. After the reaction ended, saturated Na₂CO₃ solution was added to the mixture to adjust the pH to 8-9. The mixture was then extracted with EtOAc (20 mL ×3). The organic phases were combined and washed with saturated brine, dried over anhydrous sodium sulfate, filtered, and evaporated to dryness. The residue was further purified by column chromatography (EtOAc/PE, 40% to 42%) to afford compound 6 (200 mg, 38.11 % yield) as a white solid. ESI-MS: [M+H]⁺, 425.10.

### (4) Synthesis steps of compound GDI15-8096

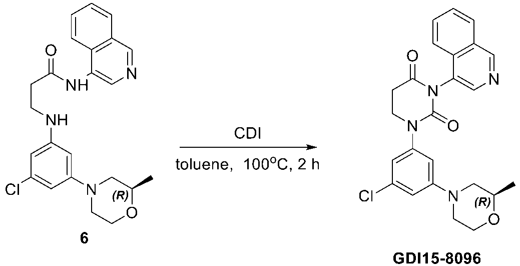

A solution of toluene (5 mL) was added to compound 6 (200 mg, 0.47 mmol), and then CDI (382 mg, 2.35 mmol) was further added. The mixture was stirred and allowed to react at 110°C for 2 hours, and the reaction ended as monitored by LCMS. The solvent was then removed in vacuo, and the residue was further purified by prep-HPLC (Gemini 5 µm C18 column, 150×21.2 mm, eluting with 75% to 95% MeCN/H₂O containing 0.1% FA) to afford compound GDI15-8096 (89.51 mg, 42.17% yield) as a yellow solid. ESI-MS:[M+H]⁺, 451.15.

**1H NMR** (400 MHz, DMSO) δ 9.36 (s, 1 H), 8.45 (s, 1 H), 8.23 (d, *J* = 8.1 Hz, 1 H), 7.93 (d, *J* = 8.4 Hz, 1 H), 7.83 (t, *J* = 7.6 Hz, 1 H), 7.74 (t, *J* = 7.5 Hz, 1 H), 6.93 (m, 3 H), 4.08 (m, 2 H), 3.90 (m, 1 H), 3.59 (m, 4 H), 3.24 (m, 1 H), 3.07 (m, 1 H), 2.69 (m, 1 H), 2.37 (t, *J* = 11.1 Hz, 1 H), 1.15 (d, *J* = 6.1 Hz, 3 H).

### Example 21: Preparation of Compound GDI15-8051

### (1) Synthesis steps of compound 3

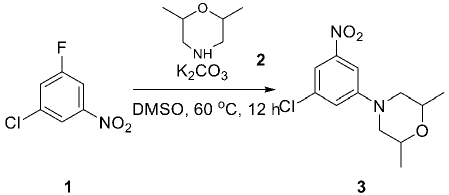

DMSO (5 mL) was added to a mixture of compound 1 (500 mg, 2.80 mmol) and compound 2 (1/1) (480 mg, 4.21 mmol). K₂CO₃ (1.16g, 8.40 mmol) was added under stirring, and the mixture was stirred and allowed to react at 60°C for 12 hours. After the reaction ended, water was added to the mixture, and the mixture was extracted with EtOAc (10 mL × 3). The organic phases were combined and washed with saturated brine, dried over anhydrous sodium sulfate, filtered, and evaporated to dryness. The residue was purified by column chromatography (PE/EtOAc, 0% to 10%) to afford compound 3 (380mg, 1.40 mmol) as a yellow solid.

**¹H NMR** *J* = 1.9 Hz, 1 H), 3.79 (m, 2 H), 3.60 - 3.45 (m, 2 H), 2.53 (t, *J* =11.2 Hz, 2 H), 1.29 (d, *J* = 6.3 Hz, 6 H).

### (2) Synthesis steps of compound 4

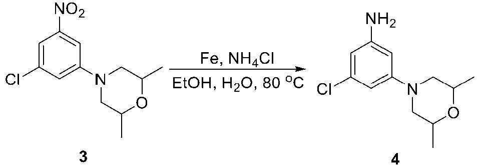

A mixed solution of EtOH/H₂O (1:1, 6 mL) was added to a mixture of compound 3 (380 mg, 1.40 mmol) and iron (392 mg, 7.02 mmol). NH₄Cl (375 mg, 7.02 mmol) was then added, and the mixture was allowed to react at 80°C for 2 hours. After the reaction ended, water was added, and the mixture was extracted with EtOAc (10 mL × 3). The organic phases were combined and washed with saturated brine, and dried over anhydrous sodium sulfate, to afford compound 4 (300 mg, 1.23 mmol) as a yellow oil. ESI-MS: [M+H] ⁺, 241.10.

### (3) Synthesis steps of compound 6

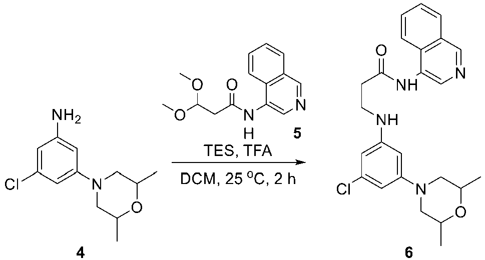

DCM (3 mL) was added to a mixture of compound 4 (250 mg, 0.96 mmol) and compound 5 (254 mg, 1.06 mmol). Under nitrogen atmosphere at room temperature, TES (394 mg, 2.40 mmol) and TFA (1 mL) were added to the mixture, and the mixture was further allowed to react at room temperature for 2 hours. After the reaction ended, the mixture was adjusted to pH 7-8 with aqueous NaHCO₃ solution and extracted with EtOAc (10 mL × 3). The organic phases were combined and washed with saturated brine, dried over anhydrous sodium sulfate, filtered, and evaporated to dryness. The residue was purified by column chromatography (DCM/MeOH, 0% to 5%) to afford compound 6 (200mg, 0.46 mmol) as a yellow solid. ESI-MS: [M+H] ⁺, 439.10.

### (3) Synthesis steps of compound GDI15-8051

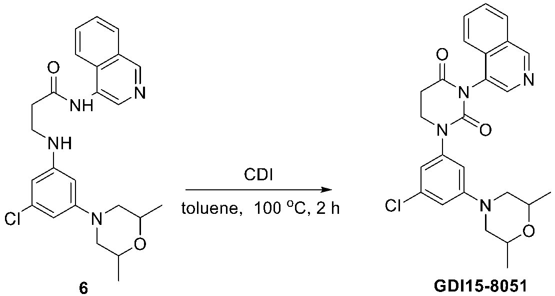

Toluene (3 mL) was added to a mixture of compound 6 (200 mg, 0.46 mmol) and CDI (443 mg, 2.73 mol). The mixture was heated to 100°C, stirred and allowed to react for 2 hours. After the reaction ended, water was added to the mixture, and the mixture was extracted with EtOAc (10 mL x 3). The organic phases were combined and washed with saturated brine, dried over anhydrous sodium sulfate, filtered, and concentrated. The residue was further purified by prep-HPLC (Gemini 5 µm C18 column, 150*21.2 mm, eluting with 30% to 90% MeCN/H₂O containing 0.1% FA) to afford compound GDI15-8051 (58 mg, 0.12 mmol) as a white solid. ESI-MS: [M+H] ⁺, 465.10.

**¹H NMR** *J* = 8.2 Hz, 1 H), 7.93 (d, *J* = 8.4 Hz, 1 H), 7.83 (t, *J* = 7.1 Hz, 1 H), 7.74 (t, *J =* 7.1 Hz, 1 H), 6.96 (s, 1 H), 6.93 - 6.88 (m, 2 H), 4.16 - 4.03 (m, 2 H), 3.65 (t, *J* = 11.2 Hz, 4 H), 3.27 - 3.20 (m, 1 H), 3.11 - 3.03 (m, 1 H), 2.29 (t, *J* = 11.4 Hz, 2 H), 1.15 (d, *J* = 6.2 Hz, 6 H).

### Example 22: Preparation of Compound GDI15-8054

### (1) Synthesis steps of compound 3

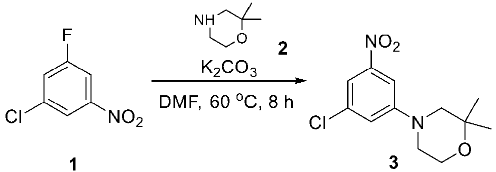

DMF (5 mL) was added to compound 1 (500 mg, 5.10 mmol) and compound 2 (660 mg, 5.71 mmol). K₂CO₃ (1.58g, 11.40 mmol) was then added, and the mixture was stirred and allowed to react at 60°C for 8 hours. After the reaction ended, water was added, and the mixture was extracted with EtOAc (10 mL × 3). The organic phases were combined and washed with saturated brine, dried over anhydrous sodium sulfate, filtered, and evaporated to dryness. The residue was purified by column chromatography (PE/EtOAc, 0% to 10%) to afford compound 3 (350 mg, 1.28 mol) as a yellow oil. ESI-MS: [M+H] ⁺, 270.08.

### (2) Synthesis steps of compound 4

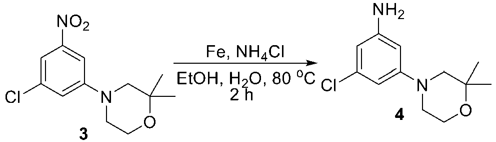

A solution of EtOH/H₂O (1:1, 6 mL) was added to a mixture of compound 3 (300 mg, 1.10 mmol) and iron (309 mg, 5.54 mmol). NH₄Cl (296 mg, 5.54 mmol) was then added, and the mixture was heated to 80°C and allowed to react for 2 hours. After the reaction ended, water was added, and the mixture was extracted with EtOAc (10 mL × 3). The organic phases were combined, washed with saturated brine, dried over anhydrous sodium sulfate, filtered, and evaporated to dryness to afford compound 4 (240 mg, 0.90 mmol) as a yellow oil. ESI-MS: [M+H] ⁺, 241.00.

### (3) Synthesis steps of compound 7

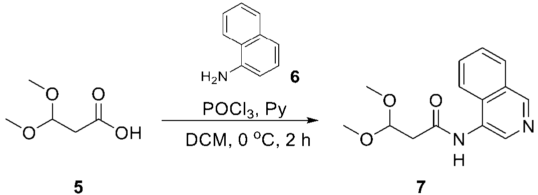

DCM (100 mL) was added to compound 5 (10.00g, 0.074 mol), compound 6 (7.6g, 0.050 mol) and pyridine (48.50g, 0.38 mol). POCl₃ (34.50g, 0.23 mol) was added slowly to the mixture at 0°C, and then the resulting solution was further allowed to react at 0°C for 2 hours. After the reaction ended, the mixture was washed with saturated brine, dried over anhydrous sodium sulfate, filtered, and evaporated to dryness. The residue was purified by column chromatography (PE/EtOAc, 0% to 50%) to afford compound 7 (4g, 0.021 mol) as a yellow solid. ESI-MS: [M+H] ⁺, 261.20.

### (3) Synthesis steps of compound 8

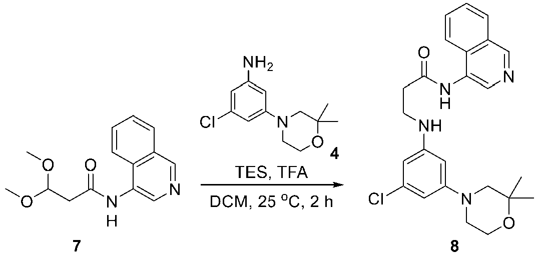

DCM (3 mL) was added to compound 7 (200 mg, 0.77 mmol) and compound 4 (203 mg, 0.84 mmol). Under nitrogen atmosphere, TES (316 mg, 1.92 mmol) and TFA (0.8 mL) were further added to the mixture, and the mixture was further allowed to react at room temperature for 2 hours. After the reaction ended, the mixture was adjusted to pH 7-8 with aqueous NaHCO₃ solution and extracted (10 mL × 3). The organic phases were combined and washed with saturated brine, dried over anhydrous sodium sulfate, filtered, and evaporated to dryness. The residue was purified by column chromatography (DCM/MeOH, 0% to 5%) to afford compound 8 (260 mg, 0.59 mmol) as a yellow solid. ESI-MS: [M+H] ⁺, 439.15.

### (4) Synthesis steps of compound GDI15-8054

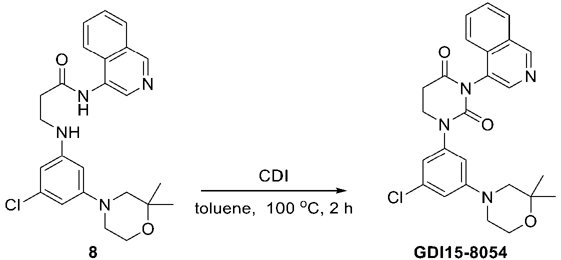

Toluene (3 mL) was added to compound 8 (260 mg, 0.59 mmol) and CDI (576 mg, 3.55 mol). The mixture was stirred and allowed to react at 100°C for 2 hours. After the reaction ended, water was added to the mixture, and the mixture was extracted with EtOAc (10 mL × 3). The organic phases were combined and washed with saturated brine, dried over anhydrous sodium sulfate, filtered, and evaporated to dryness. The residue was further purified by prep-HPLC (Gemini 5 µm C18 column, 150*21.2 mm, eluting with 30% to 90% MeCN/H₂O containing 0.1% NH₃.H₂O) to afford GD115-8054 (82 mg, 0.17 mmol) as a white solid. ESI-MS: [M+H] ⁺, 465.15.

**¹H NMR** *J* = 8.1 Hz, 1 H), 7.93 (d, *J* = 8.4 Hz, 1 H), 7.83 (t, *J* = 7.6 Hz, 1 H), 7.74 (t, *J =* 7.5 Hz, 1 H), 6.96 - 6.86 (m, 3 H), 4.17 - 4.03 (m, 2 H), 3.79 - 3.70 (m, 2 H), 3.28 - 3.20 (m, 1 H), 3.13 - 3.03 (m, 3 H), 3.00 (s, 2 H), 1.21 (s, 6 H).

### Example 23: Preparation of Compound GDI15-7886

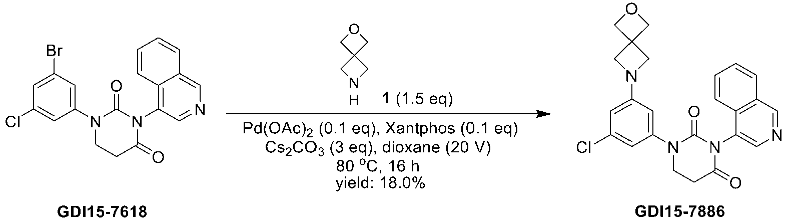

Compound GDI15-7618 (200 mg, 464 µmol, 1 eq) and compound 1 (69.1 mg, 697 µmol, 1.5 eq) were dissolved in 1,4-dioxane (4 mL). At 25°C, Cs₂CO₃ (454 mg, 1.39 mmol, 3 eq), Xantphos (26.9 mg, 46.4 µmol, 0.1 eq), and Pd(OAc)₂ (10.4 mg, 46.4 µmol, 0.1 eq) were added sequentially. The reaction mixture was allowed to react at 80°C for 16 hours under nitrogen atmosphere, and the reaction ended as monitored by LCMS. Water (10 mL) was added to the reaction mixture. The reaction mixture was extracted with EtOAc (5 mL ×3). The organic phases were combined and washed with saturated sodium chloride solution (10 mL). The combined organic phases were dried over anhydrous sodium sulfate, filtered, and evaporated to dryness under reduced pressure. The residue was purified by thin-layer chromatography (SiO₂, EtOAc/Methanol, 10/1) and high performance liquid chromatography (column: Phenomenex Luna C18 80*30 mm*3 µm; mobile phase: [H₂O (0.2% FA)-ACN]; gradient: 25%-60%, 8.0 min) to afford the target compound GDI15-7886 (37.8 mg, 83.4 µmol, 18.0% yield, 99.0% purity) as a white solid. ESI-MS:[M+H]⁺, 449.1.

¹H NMR (400 MHz, DMSO) δ 9.36 (s, 1 H), 8.45 (s, 1 H), 8.23 (d, *J =* 8.1 Hz, 1 H), 7.98 - 7.89 (m, 1 H), 7.83 (dt, *J* = 1.1, 7.6 Hz, 1 H), 7.78 - 7.68 (m, 1 H), 6.81 (t, *J =* 1.8 Hz, 1 H), 6.43 (t, *J* = 1.9 Hz, 1 H), 6.36 (t, *J =* 1.9 Hz, 1 H), 4.70 (s, 4 H), 4.09 - 4.03 (m, 2 H), 4.01 (s, 4 H), 3.26 - 3.18 (m, 1 H), 3.10 - 2.97 (m, 1 H).

### Example 24: Preparation of Compound GDI15-7888

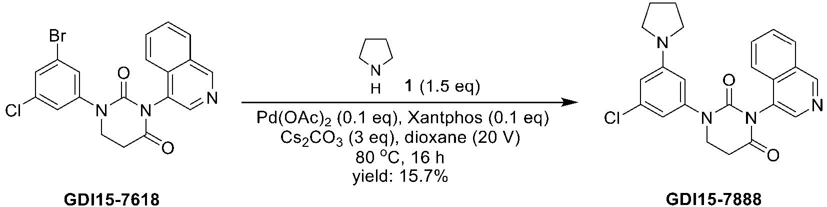

Compound GDI15-7618 (200 mg, 464 µmol, 1 eq) and compound 1 (49.5 mg, 697 µmol, 58.2 µL, 1.5 eq) were dissolved in 1,4-dioxane (4 mL). At 25°C, Cs₂CO₃ (454 mg, 1.39 mmol, 3 eq), Xantphos (26.9 mg, 46.4 µmol, 0.1 eq), and Pd(OAc)₂ (10.4 mg, 46.4 µmol, 0.1 eq) were added sequentially. The reaction mixture was allowed to react at 80°C for 16 hours under nitrogen atmosphere, and the reaction ended as monitored by LCMS. Water (10 mL) was added to the reaction solution. The resulting mixture was extracted with EtOAc (5 mL ×3). The organic phases were combined and washed with saturated sodium chloride solution (10 mL). The combined organic phases were dried over anhydrous sodium sulfate, filtered, and evaporated to dryness under reduced pressure. The residue was purified by thin-layer chromatography (SiO₂, EtOAc/Methanol, 10/1) and high performance liquid chromatography (column: 3_Phenomenex Luna C18 75*30 mm*3 µm; mobile phase: [H₂O (0.2% FA)-ACN]; gradient: 30%-60%, 8.0 min) to afford the target compound GDI15-7888 (32.2 mg, 72.8 µmol, 15.7% yield, 95.1% purity) as a white solid. ESI-MS:[M+H]⁺, 421.1.

¹H NMR (400 MHz, DMSO) δ 9.36 (s, 1 H), 8.45 (s, 1 H), 8.23 (d, *J* = 8.1 Hz, 1 H), 7.93 (d, *J* = 8.4 Hz, 1 H), 7.83 (dt, *J* = 1.1, 7.6 Hz, 1 H), 7.77 - 7.71 (m, 1 H), 6.70 (t, *J =* 1.6 Hz, 1 H), 6.53 (t, *J =* 1.9 Hz, 1 H), 6.43 (t, *J =* 1.9 Hz, 1 H), 4.13 - 3.99 (m, 2 H), 3.25 - 3.19 (m, 5 H), 3.12 - 3.01 (m, 1 H), 1.97 - 1.92 (m, 4 H).

### Example 25: Preparation of Compound GDI15-7970

### (1) Synthesis steps of compound 2

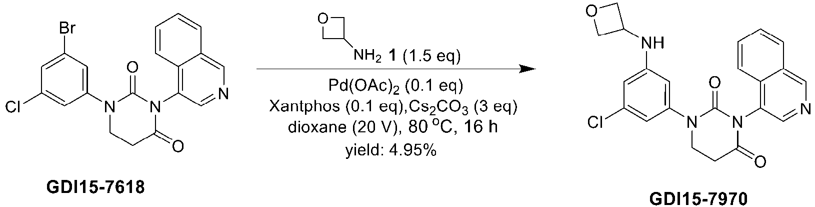

Compound GDI15-7618 (200 mg, 464 µmol, 1 eq) and compound 1 (50.9 mg, 697 µmol, 1.5 eq) were dissolved in 1,4-dioxane (4 mL). At 25°C, Cs₂CO₃ (454 mg, 1.39 mmol, 3 eq), Xantphos (26.9 mg, 46.4 µmol, 0.1 eq), and Pd(OAc)₂ (10.4 mg, 46.4 µmol, 0.1 eq) were added sequentially. The reaction mixture was allowed to react at 80°C for 16 hours under nitrogen atmosphere, and the reaction ended as monitored by LCMS. Water (8 mL) was added to the reaction solution, and the resulting mixture was extracted with EtOAc (3 mL × 3). The organic phases were combined, and the combined organic phases were dried over anhydrous sodium sulfate, filtered, and evaporated to dryness under reduced pressure. The residue was purified by thin-layer chromatography (EtOAc/Methanol = 10:1) and high performance liquid chromatography (column: Waters Xbridge BEH C18 100*30 mm*10 µm; mobile phase: [H₂O (10 mM NH₄HCO₃)-ACN]; gradient: 25%-55%, 8.0 min) to afford the target compound GDI15-7970 (10 mg, 23.1 µmol, 4.97% yield, 97.6% purity) as a white solid. ESI-MS: [M+H]⁺, 423.1.

¹H NMR (400 MHz, DMSO) δ 9.36 (s, 1 H), 8.44 (s, 1 H), 8.23 (d, *J* = 8.0 Hz, 1 H), 7.93 (d, *J* = 8.7 Hz, 1 H), 7.86 - 7.81 (m, 1 H), 7.77 - 7.71 (m, 1 H), 6.87 (d, *J =* 6.6 Hz, 1 H), 6.72 (s, 1 H), 6.57 - 6.50 (m, 1 H), 6.37 (s, 1 H), 4.83 (t, *J =* 6.5 Hz, 2 H), 4.58 - 4.50 (m, 1 H), 4.40 - 4.33 (m, 2 H), 4.09 - 3.98 (m, 2 H), 3.27 - 3.16 (m, 1 H), 3.11 - 3.00 (m, 1 H).

### Example 26: Preparation of Compound GDI15-7646

### (1) Synthesis steps of compound 2

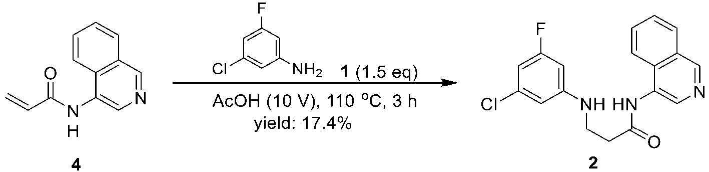

Compound 4 (730 mg, 3.68 mmol, 1 eq) and compound **1** (804 mg, 5.52 mmol, 1.5 eq) were dissolved in AcOH (7.3 mL). The mixture was allowed to react at 110°C for 3 hours, and the reaction ended as monitored by LCMS. The reaction solution was concentrated under reduced pressure, and then water (20 mL) was added. The reaction solution was extracted with EtOAc (10 mL ×3). The organic phases were combined and washed with 20 mL of saturated sodium chloride solution. The combined organic phases were dried over anhydrous sodium sulfate, filtered, and evaporated to dryness under reduced pressure. The residue was purified by thin-layer chromatography (Petroleum ether/EtOAc = 1:1) to afford the target compound 2 (220 mg, 639 µmol, 17.4% yield) as a white solid. ESI-MS:[M+H]+, 344.2.

### (2) Synthesis steps of compound GDI15-7646

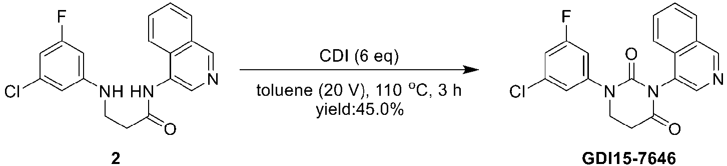

Compound 2 (230 mg, 669 µmol, 1 eq) was dissolved in toluene (4.6 mL). CDI (650 mg, 4.01 mmol, 6 eq) was added at 25°C. The reaction mixture was allowed to react at 110°C for 3 hours, and the reaction ended as monitored by LCMS. The reaction solution was concentrated under reduced pressure, and then water (10 mL) was added. The reaction solution was extracted with EtOAc (7 mL ×3). The organic phases were combined and washed with 10 mL of saturated sodium chloride solution. The combined organic phases were dried over anhydrous sodium sulfate, filtered, and evaporated to dryness under reduced pressure. The residue was purified by high performance liquid chromatography (column: CAPCELL PAK ADME 20 × 150 mm, 5 µm; mobile phase: [H₂O (0.1% TFA)-ACN]; gradient: 20%-50%, 15.0 min) to afford the target compound GDI15-7646 (111 mg, 301 µmol, 45.0% yield) as a white solid. ESI-MS:[M+H]+, 370.2.

1H NMR (400 MHz, DMSO) δ 9.37 (s, 1 H), 8.46 (s, 1 H), 8.23 (d, *J* = 8.1 Hz, 1 H), 7.96 (d, *J* = 8.3 Hz, 1 H), 7.86 - 7.80 (m, 1 H), 7.77 - 7.72 (m, 1 H), 7.45 (s, 1 H), 7.40 - 7.33 (m, 2 H), 4.19 - 4.13 (m, 2 H), 3.27 - 3.21 (m, 1 H), 3.11 - 3.04 (m, 1 H).

### Example 27: Preparation of Compound GDI15-7703

### (1) Synthesis steps of compound 2

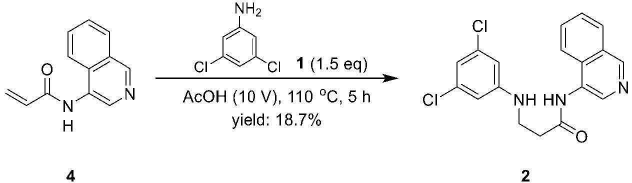

Compound 4 (500 mg, 2.52 mmol, 1 eq) and compound **1** (613 mg, 3.78 mmol, 1.5 eq) were dissolved in AcOH (5 mL). The reaction mixture was allowed to react at 110°C for 3 hours, and the reaction ended as monitored by LCMS. The reaction solution was concentrated under reduced pressure and evaporated to dryness, and then water (20 mL) was added. The reaction solution was extracted with EtOAc (10 mL ×3). The organic phases were combined and washed with 20 mL of saturated sodium chloride solution. The washed organic phases were dried over anhydrous sodium sulfate, filtered, and evaporated to dryness under reduced pressure. The residue was purified by thin-layer chromatography (Petroleum ether/EtOAc, 1:1) to afford the target compound 2 (170 mg, 471 µmol, 18.7% yield) as a white solid. ESI-MS:[M+H]⁺, 361.2.

### (2) Synthesis steps of compound GDI15-7703

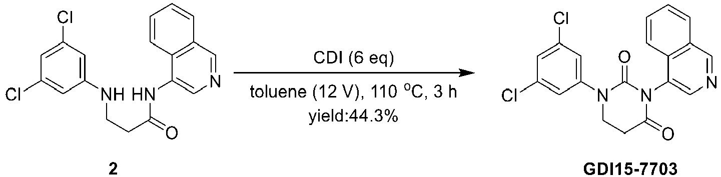

Compound 2 (170 mg, 471 µmol, 1 eq) was dissolved in toluene (2 mL). CDI (382 mg, 2.36 mmol, 6 eq) was added at 25°C. The reaction mixture was allowed to react at 110°C for 3 hours, and the reaction ended as monitored by LCMS. The reaction solution was concentrated under reduced pressure and evaporated to dryness, and then water (10 mL) was added. The reaction solution was extracted with EtOAc (7 mL ×3). The organic phases were combined and washed with 10 mL of saturated sodium chloride solution. The washed organic phases were dried over anhydrous sodium sulfate, filtered, and evaporated to dryness under reduced pressure. The residue was purified by high performance liquid chromatography (column: Waters Xbridge BEH C18 100*30 mm*10 µm; mobile phase: [H₂O (10 mM NH₄HCO₃)-ACN]; gradient: 37%-65%, 8.0 min) to afford the target compound GDI15-7703 (80.8 mg, 209 µmol, 44.3% yield) as a white solid. ESI-MS:[M+H]⁺, 387.2.

¹H NMR (400 MHz, DMSO) δ 9.37 (s, 1 H), 8.45 (s, 1 H), 8.23 (d, *J* = 8.2 Hz, 1 H), 7.96 (d, *J* = 8.3 Hz, 1 H), 7.83 (dt, *J =* 1.1, 7.7 Hz, 1 H), 7.78 - 7.72 (m, 1 H), 7.57 (d, *J* = 1.9 Hz, 2 H), 7.54 - 7.51 (m, 1 H), 4.20 - 4.11 (m, 2 H), 3.30 - 3.20 (m, 1 H), 3.08 (td, *J* = 6.1, 16.7 Hz, 1 H).

### Example 28: Preparation of Compound GDI15-7650

### (1) Synthesis steps of compound 2

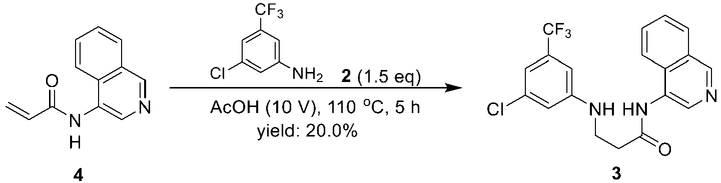

Compound 4 (730 mg, 3.68 mmol, 1 eq) and compound 2 (1.08g, 5.52 mmol, 1.5 eq) were dissolved in AcOH (7.3 mL). The mixture was allowed to react at 110°C for 3 hours, and the reaction ended as monitored by LCMS. The reaction solution was concentrated under reduced pressure, and then water (20 mL) was added. The reaction solution was extracted with EtOAc (10 mL ×3). The organic phases were combined and washed with 20 mL of saturated sodium chloride solution. The combined organic phases were dried over anhydrous sodium sulfate, filtered, and evaporated to dryness under reduced pressure. The residue was purified by thin-layer chromatography (Petroleum ether/EtOAc = 1:1) to afford the target compound 3 (270 mg, 639 µmol, 20% purity) as a white solid. ESI-MS:[M+H]+, 394.2.

### (2) Synthesis steps of compound GDI15-7650

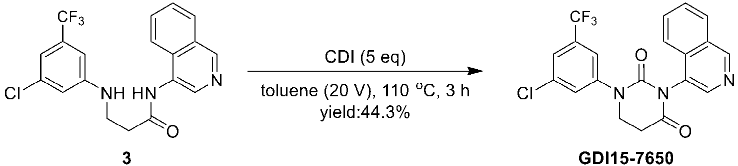

Compound 3 (270 mg, 685 µmol, 1 eq) was dissolved in toluene (6 mL). CDI (555 mg, 3.43 mmol, 5 eq) was added at 25°C. The reaction mixture was allowed to react at 110°C for 3 hours, and the reaction ended as monitored by LCMS. The reaction solution was concentrated under reduced pressure, and then water (10 mL) was added. The reaction solution was extracted with EtOAc (7 mL ×3). The organic phases were combined and washed with 10 mL of saturated sodium chloride solution. The combined organic phases were dried over anhydrous sodium sulfate, filtered, and evaporated to dryness under reduced pressure. The residue was purified by high performance liquid chromatography (column: Waters Xbridge BEH C18 100*30 mm*10 µm; mobile phase: [H₂O (10 mM NH₄HCO₃)-ACN]; gradient: 40%-70%, 8.0 min) to afford the target compound GDI15-7650 (128 mg, 304 µmol, 44.3% yield) as a white solid. ESI-MS:[M+H]+, 420.2.

1H NMR (400 MHz, DMSO) δ 9.37 (s, 1 H), 8.47 (s, 1 H), 8.24 (d, *J* = 8.2 Hz, 1 H), 7.98 (d, *J* = 8.4 Hz, 1 H), 7.89 (s, 1 H), 7.86 - 7.80 (m, 2 H), 7.78 - 7.72 (m, 2 H), 4.25 - 4.15 (m, 2 H), 3.30 - 3.22 (m, 1 H), 3.15 - 3.05 (m, 1 H).

### Example 29: Preparation of Compound GDI15-7702

### (1) Synthesis steps of compound 2

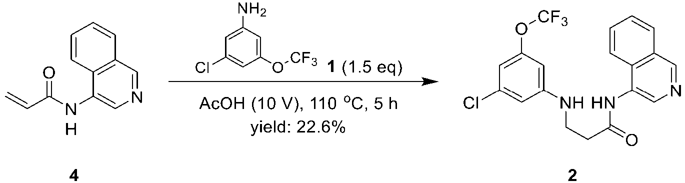

Compound 4 (300 mg, 1.51 mmol, 1 eq) and compound **1** (480 mg, 2.27 mmol, 1.5 eq) were dissolved in AcOH (3 mL). The reaction mixture was allowed to react at 110°C for 3 hours, and the reaction ended as monitored by LCMS. The reaction solution was concentrated under reduced pressure and evaporated to dryness, and then water (10 mL) was added. The reaction solution was extracted with EtOAc (5 mL ×3). The organic phases were combined and washed with 20 mL of saturated sodium chloride solution. The washed organic phases were dried over anhydrous sodium sulfate, filtered, and evaporated to dryness under reduced pressure. The residue was purified by thin-layer chromatography (Petroleum ether/EtOAc, 1:1) to afford the target compound 2 (140 mg, 341 µmol, 22.6% yield) as a white solid. ESI-MS:[M+H]⁺, 410.2.

### (2) Synthesis steps of compound GDI15-7702

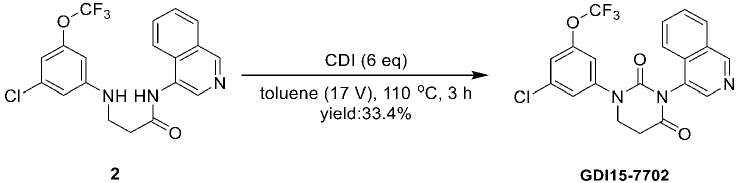

Compound 2 (120 mg, 292 µmol, 1 eq) was dissolved in toluene (2 mL). CDI (237 mg, 1.46 mmol, 6 eq) was added at 25°C. The reaction mixture was allowed to react at 110°C for 3 hours, and the reaction ended as monitored by LCMS. The reaction solution was concentrated under reduced pressure and evaporated to dryness, and then water (10 mL) was added. The reaction solution was extracted with EtOAc (7 mL ×3). The organic phases were combined and washed with saturated sodium chloride solution (10 mL). The washed organic phases were dried over anhydrous sodium sulfate, filtered, and evaporated to dryness under reduced pressure. The residue was purified by high performance liquid chromatography (column: 3_Phenomenex Luna C18 75*30 mm*3 µm; mobile phase: [H₂O (0.2% FA)-ACN]; gradient: 25%-55%, 8.0 min) to afford the target compound GDI15-7702 (42.6 mg, 97.7 µmol, 33.4% yield) as a white solid. ESI-MS:[M+H]⁺, 436.2.

¹H NMR (400 MHz, DMSO) δ 9.37 (s, 1 H), 8.47 (s, 1 H), 8.29 - 8.19 (m, 1 H), 7.97 (d, *J* = 8.2 Hz, 1 H), 7.88 - 7.80 (m, 1 H), 7.79 - 7.70 (m, 1 H), 7.64 (t, *J =* 1.7 Hz, 1 H), 7.49 (d, *J* = 14.7 Hz, 2 H), 4.26 - 4.13 (m, 2 H), 3.28 - 3.23 (m, 1 H), 3.15 - 3.05 (m, 1 H).

### Example 30: Preparation of Compound GDI15-7737

### (1) Synthesis steps of compound 2

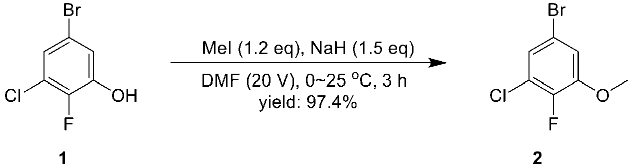

Compound 1 (3g, 13.3 mmol, 1 eq) was dissolved in DMF (60 mL). NaH (798 mg, 19.9 mmol, 60% purity, 1.5 eq) was added portionwise at 0°C. After the reaction mixture reacted at 0°C for 1 hour, MeI (2.27g, 15.9 mmol, 994 µL, 1.2 eq) was added to the above mixture. The reaction mixture was further allowed to react at 25°C for 2 hours, and the reaction ended as monitored by LCMS. The reaction solution was added dropwise to saturated ammonium chloride solution (100 mL) at 0°C, and extracted with EtOAc (50 mL × 3). The organic phases were combined and washed with 200 mL of saturated brine. The combined organic phases were dried over anhydrous sodium sulfate, filtered, and evaporated to dryness under reduced pressure to afford compound 2 (3.2 g, 12.9 mmol, 97.4% yield, 97% purity) as a yellow oil.

### (2) Synthesis steps of compound 3

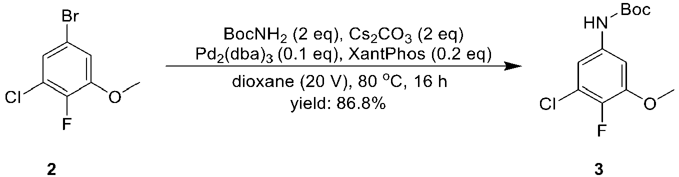

Compound 2 (3.2g, 13.3 mmol, 1 eq) and BocNH₂ (3.13g, 26.7 mmol, 2 eq) were dissolved in 1,4-dioxane (64 mL). Under nitrogen atmosphere, Cs₂CO₃ (8.71g, 26.7 mmol, 2 eq), Pd₂(dba)₃ (1.22g, 1.34 mmol, 0.1 eq) and Xantphos (1.55g, 2.67 mmol, 0.2 eq) were added sequentially. The reaction mixture was allowed to react at 80°C for 16 hours, and the reaction ended as monitored by LCMS. The reaction mixture was filtered, and the filtrate was purified by column chromatography (SiO₂, Petroleum ether/EtOAc = 20:1 to 5:1) to afford compound 3 (3.2g, 11.6 mmol, 86.8% yield) as a yellow solid. ESI-MS:[M+H]⁺, 235.2.

### (3) Synthesis steps of compound 4

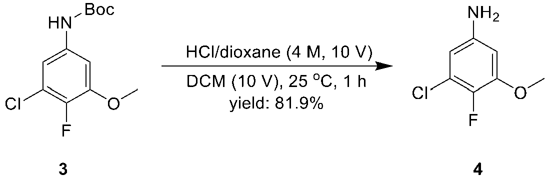

Compound 3 (3.2g, 11.6 mmol, 1 eq) was dissolved in DCM (32 mL). HCl/dioxane (32 mL, 4 M) was added at 25°C. The reaction mixture was allowed to react at 25°C for 1 hour, and the reaction ended as monitored by LCMS. The reaction solution was concentrated under reduced pressure, and EtOAc (20 mL) was then added. The mixture solution was filtered, and the filter cake was collected. Water (30 mL) was added to the filter cake, and the pH was adjusted to 8 with the saturated sodium carbonate solution. Then the resulting mixture was extracted with EtOAc (20 mL ×3). The organic phases were combined and washed with saturated sodium chloride solution (80 mL). The combined organic phases were dried over anhydrous sodium sulfate, filtered, and evaporated to dryness under reduced pressure to afford compound 4 (1.67g, 9.51 mmol, 81.9% yield) as a yellow solid. ESI-MS: [M+H] ⁺, 176.1.

### (4) Synthesis steps of compound 6

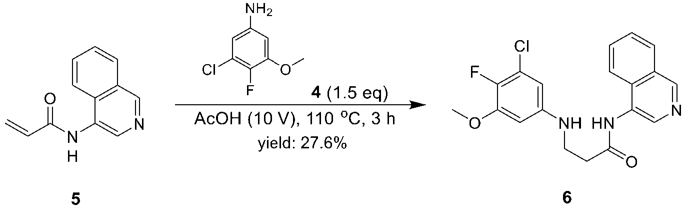

Compound 5 (250 mg, 1.26 mmol, 1 eq) and compound **4** (332mg, 1.89 mmol, 1.5 eq) were dissolved in AcOH (2.5 mL). The mixture was allowed to react at 110°C for 3 hours, and the reaction ended as monitored by LCMS. The reaction solution was concentrated under reduced pressure, and then water (10 mL) was added. The reaction solution was extracted with EtOAc (2 mL ×3). The organic phases were combined and washed with 10 mL of saturated sodium chloride solution. The combined organic phases were dried over anhydrous sodium sulfate, filtered, and evaporated to dryness under reduced pressure. The residue was purified by thin-layer chromatography (SiO₂, PE/EtOAc = 0/1) to afford the target compound 6 (130 mg, 347 µmol, 27.6% yield) as a yellow solid. ESI-MS: [M+H] ⁺, 374.2.

### (5) Synthesis steps of compound GDI15-7737

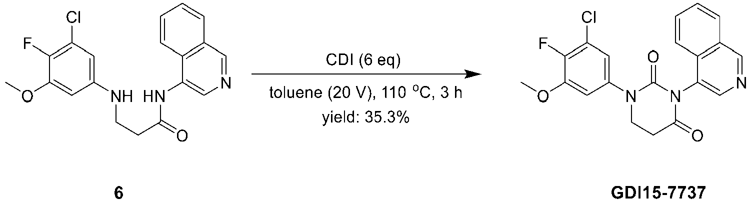

Compound 6 (50 mg, 133 µmol, 1 eq) was dissolved in toluene (1 mL). CDI (130 mg, 802 µmol, 6 eq) was added at 25°C. The reaction mixture was allowed to react at 110°C for 3 hours, and the reaction ended as monitored by LCMS. The reaction solution was concentrated under reduced pressure, and then water (10 mL) was added. The reaction solution was extracted with EtOAc (2 mL ×3). The organic phases were combined and washed with 10 mL of saturated sodium chloride solution. The combined organic phases were dried over anhydrous sodium sulfate, filtered, and evaporated to dryness under reduced pressure. The residue was purified by high performance liquid chromatography (aters Xbridge BEH C18 100*30 mm*10 µm; mobile phase: [H₂O (10 mM NH₄HCO₃)-ACN]; gradient: 40%-70%, 8.0 min) to afford the target compound GDI15-7737 (19.4 mg, 47.3 µmol, 35.3% yield, 97.4% purity) as a yellow solid. ESI-MS:[M+H]⁺, 400.1.

¹H NMR (400 MHz, DMSO) δ 9.37 (s, 1 H), 8.46 (s, 1 H), 8.24 (d, *J* = 8.2 Hz, 1 H), 7.95 (d, *J* = 8.4 Hz, 1 H), 7.84 (t, *J* = 7.2 Hz, 1 H), 7.75 (t, *J =* 7.6 Hz, 1 H), 7.28 (dt, *J =* 2.4, 6.8 Hz, 2 H), 4.18 - 4.06 (m, 2 H), 3.89 (s, 3 H), 3.29 - 3.22 (m, 1 H), 3.15 - 3.05 (m, 1 H).

### Example 31: Preparation of Compound GDI15-7727

### (1) Synthesis steps of compound 3

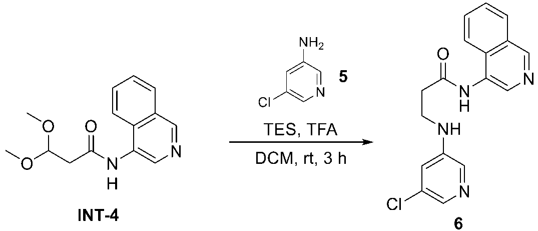

TFA (2.5 mL), compound 5 (246.95 mg, 1.92 mmol) and Et₃SiH (558.36 mg, 4.80 mmol) were added to a solution of compound INT-4 (500 mg, 1.92 mmol) in DCM (5 mL). The mixture was allowed to react at room temperature for 3 hours. The reaction ended as monitored by LCMS. After the reaction ended, the mixture was concentrated, and the pH of the mixture was adjusted to 7-8 with saturated aqueous NaOH solution. The filter cake was collected by filtration and washed with water to afford compound 6 (300 mg, 47.79% yield) as a brown solid. ESI-MS: [M+H] ⁺, 327.0.

### (2) Synthesis steps of compound GDI15-7727

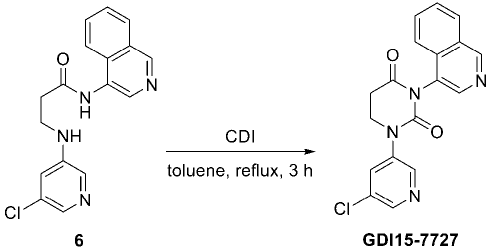

CDI (893.07 mg, 5.508 mmol) was slowly added to a solution of compound 6 (300 mg, 0.918 mmol) in toluene (3 mL). The mixture was heated to 100°C and allowed to react for 3 hours. Then the residue was further purified by Biotage Isolera One (C18 column, eluting with 10% to 90% MeCN/H₂O containing 0.1% formic acid) to afford compound GDI-7727 (18.15 mg, 5.60% yield) as a yellow solid. ESI-MS: [M+H] ⁺, 353.10.

**¹H NMR (400 MHz, DMSO)** *J* = 2.1 Hz, 1 H), 8.52 (s, 1 H), 8.52 (s, 1 H), 8.28 (d, *J* = 8.1 Hz, 1 H), 8.08 (t, *J* = 2.2 Hz, 1 H), 8.02 (d, *J* = 8.1 Hz, 1 H), 7.88 (t, *J* = 7.6 Hz, 1 H), 7.79 (t, *J* = 7.5 Hz, 1 H), 4.23 - 4.20 (m, 2 H), 3.33 - 3.25 (m, 1 H), 3.12 (dd, *J* = 14.6, 7.6 Hz, 1 H).

### Example 32: Preparation of Compound GDI15-7543

### (1) Synthesis steps of compound 3

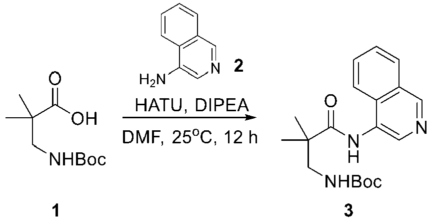

DMF (45 mL) and DIPEA (8.03g, 0.062 mol) were added to a mixture of compound 1 (4.5g, 0.021 mol), compound 2 (3g, 0.021 mol) and HATU (15.74g, 0.042 mol). The mixture was stirred and allowed to react at room temperature for 12 **hours.** After the reaction ended, EtOAc (50 mL *3) was added to the mixture for extraction. The organic phases were combined, washed with saturated brine and dried over anhydrous sodium sulfate. The residue was purified by pre-TCL (DCM/MeOH, 5%) to afford compound 3 (500 mg, 1.30 mmol) as a yellow solid. ESI-MS: [M+H] ⁺, 344.05.

### (2) Synthesis steps of compound 4

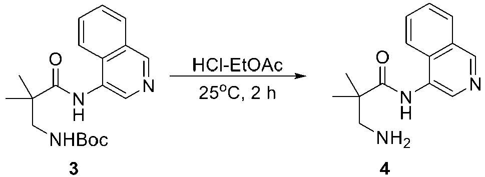

HCl-EtOAc (4 mL) was added to a solution of compound 3 (500 mg, 1.300 mmol) in EtOAc (2 mL), and the mixture was then stirred at room temperature for 2 hours. After the reaction ended, pH of the mixture was adjusted to 7-8 with aqueous NaHCO₃ solution. The organic phase was then isolated and concentrated to afford compound 4 (320 mg, 1.052 mmol) as a yellow solid. ESI-MS: [M+H] ⁺, 244.00.

### (3) Synthesis steps of compound 5

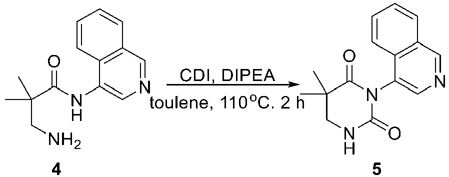

A solution of toluene (4 mL) was added to a mixture of compound 4 (320 mg, 1.052 mmol), CDI (1.3g, 7.891 mmol) and DIPEA (510 mg, 3.946 mmol). The mixture was then heated to 110°C and allowed to react for 2 hours. After the reaction ended, water and EtOAc (10 mL × 3) were added for extraction. The organic phases were combined, washed with saturated brine, dried over anhydrous sodium sulfate, filtered, and evaporated to dryness. The residue was purified by column chromatography (DCM/MeOH, 0% to 5%) to afford compound 5 (100 mg, 0.371 mmol) as a yellow solid. ESI-MS: [M+H] ⁺, 270.10.

### (4) Synthesis steps of compound GDI15-7543

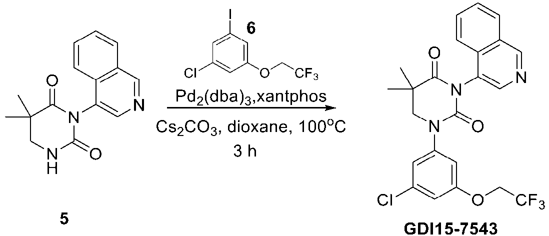

1,4-dioxane (2 mL) was added to a mixture of compound 5 (100 mg, 0.371 mmol), compound 6 (150 mg, 0.446 mmol), Pd₂(dba)₃ (68 mg, 0.074 mmol), Xantphos (107 mg, 0.186 mmol) and Cs₂CO₃ (363 mg, 1.114 mmol). The mixture was heated to 100°C, stirred and allowed to react for 3 hours under nitrogen atmosphere. After the reaction ended, the mixture was filtered and concentrated, and the residue was further purified by prep-HPLC (column: Gemini 5 µm C18 column, 150*21.2 mm, eluting with 30% to 90% MeCN/H₂O containing 0.1% FA) to afford compound GDI15-7543 (30 mg, 0.062 mmol) as a yellow solid. ESI-MS: [M+H]⁺, 478.00.

### Example 33: Preparation of Compound GDI15-7567

### (1) Synthesis steps of compound 3

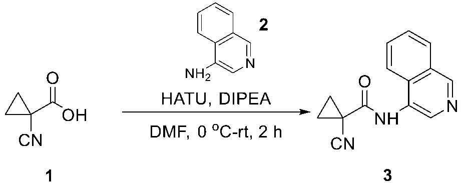

DMF (20 mL) was added to a mixture of compound 1 (1g, 9 mmol), compound 2 (1.3g, 9 mmol), HATU (4.11g, 10.7 mmol) and DIPEA (3.48g, 26.9 mmol). The mixture was then stirred and allowed to react at room temperature for 1 hour under nitrogen atmosphere. After the reaction ended, water (50 mL) was added to the mixture, and the mixture was extracted with EtOAc (50 mL × 3). The organic phases were combined and washed with saturated brine, dried over anhydrous sodium sulfate, filtered, and evaporated to dryness. The residue was further purified by column chromatography (EtOAc/PE, 10% to 50%) to afford compound 3 (1.1g, 51.11% yield) as a brown solid.

### (2) Synthesis steps of compound 4

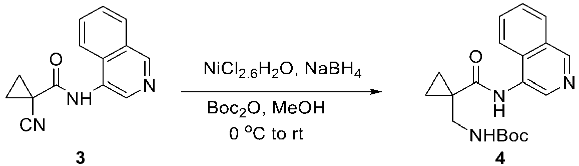

NiCl₂.6H₂O (3.08g, 12.96 mmol) was added to a solution of compound 3 (2.05g, 8.64 mmol) in MeOH (20 mL) at 0°C under nitrogen atmosphere, and the mixture was further allowed to react at this temperature for 10 minutes. NaBH₄ (1.63g, 43.20 mmol) was then added to the mixture in three portions, and the resulting mixture was further stirred and allowed to react for 20 minutes. The reaction ended as monitored by LCMS. (Boc₂)O (7.54g, 34.56 mmol) was added dropwise to the mixture. The mixture was slowly warmed to room temperature and allowed to react for 16 hours at room temperature, and the reaction ended as monitored by LCMS. The solvent was then removed under reduced pressure. Water (200 mL) was added to the mixture, and the mixture was extracted with EtOAc (200 mL × 3). The organic phases were combined and washed with saturated brine, dried over anhydrous sodium sulfate, filtered, and evaporated to dryness. The residue was further purified by column chromatography (MeOH/DCM, 5% to 10%) to afford compound 4 (910mg, 30.85% yield) as a gray-brown solid. ESI-MS: [M+H] ⁺, 342.1.

### (3) Synthesis steps of compound 5

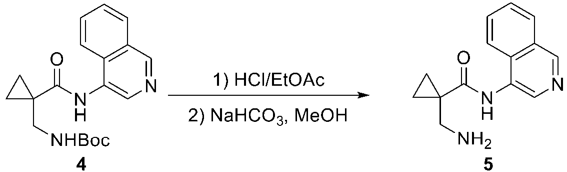

A solution of HCl in EtOAc (2 M, 10 mL) was added to compound 4 (900 mg, 2.64 mmol) and the mixture was stirred at room temperature for 2 hours. The reaction ended as monitored by LCMS. After the reaction ended, the mixture was concentrated. After concentration, the residue was treated with EtOAc and 1 M NaOH, respectively. The organic phases were isolated, washed with water, combined, dried over anhydrous sodium sulfate, filtered and evaporated to dryness to afford compound 5 (360 mg, 48.11 % yield) as a gray-brown solid. ESI-MS: [M+H] ⁺, 242.1.

### (4) Synthesis steps of compound 6

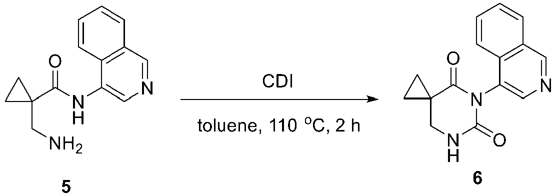

CDI (470 mg, 2.90 mmol) was added to a solution of compound 5 (350 mg, 1.45 mmol) in toluene (4 mL). The mixture was heated to 110°C for reaction for 2 hours under nitrogen atmosphere. The reaction ended as monitored by LCMS. The mixture was concentrated under reduced pressure. Water (50 mL) was added to the mixture, and the mixture was extracted with EtOAc (50 mL × 3). The organic phases were combined and washed with saturated brine, dried over anhydrous sodium sulfate, filtered, and evaporated to dryness. The residue was purified by column chromatography (MeOH/DCM, 5% to 10%) to afford compound 6 (160 mg, 41.27% yield) as a reddish-brown oil. ESI-MS: [M+H] ⁺, 268.1.

### (5) Synthesis steps of compound GDI15-7567

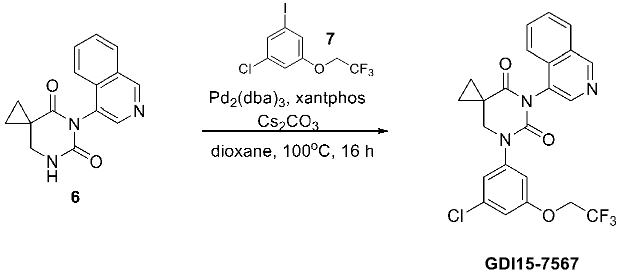

Compound 7 (303.03 mg, 0.8979 mmol), Xantphos (34.64 mg, 0.05986 mmol), Cs₂CO₃ (585.11 mg, 1.7958 mmol) and Pd₂(dba)₃ (54.82 mg, 0.0598 mmol) were added to a solution of compound 6 (160 mg, 0.5986 mmol) in 1,4-dioxane (2 mL). The mixture was heated to 100°C for reaction for 16 hours under nitrogen atmosphere. The reaction ended as monitored by LCMS. The mixture was concentrated. The residue was further purified by prep-HPLC (Gemini 5 µm C18 column, 150*21.2 mm, eluting with 90% to 95% ACN/H₂O containing 0.05% NH₃.H₂O) to afford compound GDI15-7567 (15.8 mg, 5.54 % yield) as a white solid. ESI-MS: [M+H] ⁺, 476.0.

¹H NMR (400 MHz, DMSO) δ 9.37 (s, 1 H), 8.47 (s, 1 H), 8.24 (d, *J* = 7.9 Hz, 1 H), 7.86 - 7.82 (m, 2 H), 7.77 - 7.69 (m, 1 H), 7.24 (s, 1 H), 7.13 (s, 2H), 4.88 - 4.81 (m, 2H), 4.12 - 4.04 (m, 2H), 1.37 - 1.31 (m, 2H), 1.28 - 1.21 (m, 2H).

### Example 34: Preparation of Compound GDI15-7842

### (1) Synthesis steps of compound 3

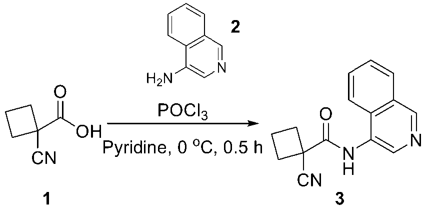

Compound 1 (10.00g, 0.0799 mol) and compound 2 (1.94g, 0.0759 mol) were dissolved in pyridine (100 mL). Then POCl₃ (73.51g, 0.4794 mol) was added slowly at 0°C under nitrogen atmosphere, and the mixture was further allowed to react at 0°C for 0.5 hours. After the reaction ended, water (200 mL) was added to the mixture, and EtOAc (500 mL × 3) was added for extraction. The organic phases were combined, washed with saturated brine, dried over anhydrous sodium sulfate, filtered, and evaporated to dryness. The residue was further purified by column chromatography (EtOAc/PE, 50% to 60%) to afford compound 3 (8.5g, 42.30% yield) as a brown solid. ESI-MS: [M+H] ⁺, 252.3.

### (2) Synthesis steps of compound 4

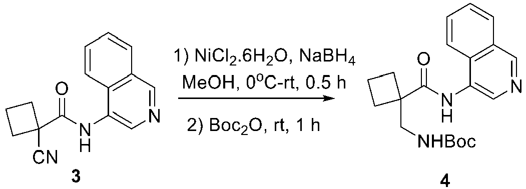

Compound 3 (5g, 19.9 mmol) was dissolved in MeOH (50 mL). NiCl₂.6H₂O (7.09g, 29.8 mmol) was then added dropwise. The mixture was allowed to react under nitrogen atmosphere at 0°C. Then NaBH₄ (2.3g, 59.7 mmol) was added slowly, and the mixture was then further allowed to react at 0°C for 30 minutes. Finally, (Boc)₂O (13g, 59.7 mmol) was added dropwise. Then the mixture was slowly warmed to room temperature, stirred and allowed to react at room temperature for 1 hour. After the reaction ended, water was added to the mixture, and the mixture was extracted with EtOAc. The organic phases were combined, washed with saturated brine, dried over anhydrous sodium sulfate, filtered, and evaporated to dryness. The residue was purified by column chromatography (EtOAc/PE, 8% to 10%) to afford compound 4 (800mg, 11.28% yield) as a brown solid. ESI-MS: [M+H] ⁺, 356.1.

### (3) Synthesis steps of compound 5

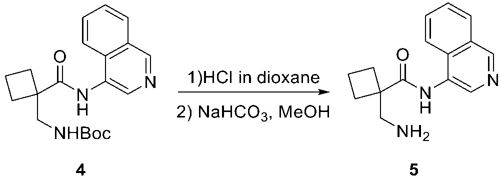

1,4-dioxane (2 mL) was added to compound 4 (1.0g, 2.8055 mmol), and then a solution of HCl in 1,4-dioxane (2.1 mL, 4 mol/L) was further added dropwise at room temperature. The mixture was further allowed to react at room temperature for 2 hours. The solvent was then evaporated, and MeOH and NaHCO₃ were added to the residue. The remaining solvent was finally evaporated, and the residue was lyophilized to afford the final product, compound 5. ESI-MS: [M+H] ⁺, 256.1.

### (4) Synthesis steps of compound 7

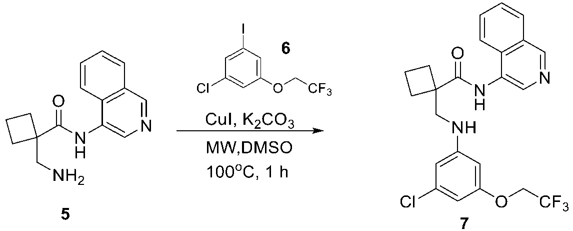

DMSO (5 mL) was added to compound 5 (190 mg, 0.7442 mmol), and then compound 6 (200.93 mg, 0.5954 mmol), K₂CO₃ (411.41 mg, 2.9768 mmol), and copper(I) iodide (141.73 mg, 0.7442 mmol) were added to the mixture. The mixture was subjected to microwave irradiation for reaction under nitrogen atmosphere at 100°C for 1 hour. After the reaction ended, the mixture was cooled to room temperature, filtered, and concentrated in vacuo. The residue was then diluted with water and extracted with EtOAc. The organic phases were combined and washed with saturated brine, dried over anhydrous sodium sulfate, filtered, and evaporated to dryness. The residue was further purified by prep-TLC (EtOAc/PE = 1:1) to afford compound 7 (30 mg, 8.67% yield) as a white solid. ESI-MS: [M+H] ⁺, 492.15.

### (5) Synthesis steps of compound GDI15-7842

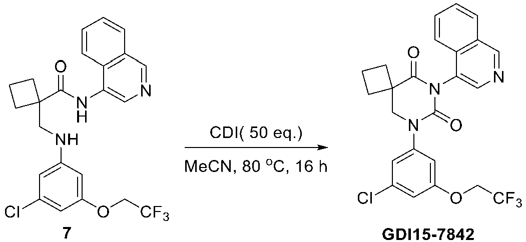

A solution of MeCN (3 mL) was added to compound 7 (15 mg, 0.03 mmol), and then CDI (261.86 mg, 1.6 mmol) was slowly added at 0°C under nitrogen atmosphere. The mixture was further allowed to react at 80°C for 16 hours. After the reaction ended, water (20 mL) was added, and the mixture was extracted with EtOAc (20 mL × 3). The organic phases were combined, washed with saturated brine, dried over anhydrous sodium sulfate, filtered, and evaporated to dryness. The residue was further purified by prep-TLC (EtOAc/PE = 1:1) to afford the final compound GDI15-7842 (2.39 mg, 15.17% yield) as a white solid.

ESI-MS: [M+H] ⁺, 490.2. **¹H NMR (400 MHz, DMSO)** δ 9.37 (s, 1 H), 8.45 (s, 1 H), 8.25 (d, *J* = 8.0 Hz, 1 H), 7.88 - 7.82 (m, 1H), 7.76 (dd, *J* = 14.8, 7.2 Hz, 2 H), 7.27 (s, 1 H), 7.16 (s, 2 H), 4.86 (q, *J* = 8.8 Hz, 2 H), 4.25 (q, *J* = 12.4 Hz, 2 H), 2.62 - 2.53 (m, 2 H), 2.25 - 2.09 (m, 3 H), 2.00 - 1.92 (m, 1 H).

### Example 35: Preparation of Compound GDI15-7945

### (1) Synthesis steps of compound 2

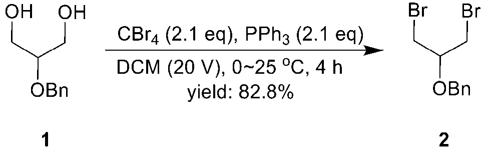

Compound 1 (10g, 54.8 mmol, 1 eq) and CBr₄ (38.2g, 115 mmol, 2.1 eq) were dissolved in DCM (200 mL). PPh₃ (30.2g, 115 mmol, 2.1 eq) was added at 0°C. The reaction mixture was allowed to react at 25°C for 4 hours, and the reaction ended as monitored by LCMS. The reaction solution was concentrated under reduced pressure and purified by column chromatography (PE/EtOAc, 20:1 to 8:1) to afford the target compound 2 (14g, 45.4 mmol, 82.8% yield) as a colorless oil.

### (2) Synthesis steps of compound 4

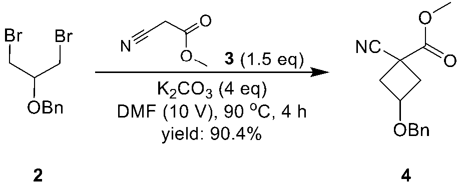

Compound 2 (7.5g, 24.3 mmol, 1 eq) and **compound 3** (3.62g, 36.5 mmol, 3.22 mL, 1.5 eq) were dissolved in DMF (75 mL). K₂CO₃ (13.4g, 97.4 mmol, 4 eq) was added at 25°C. The reaction mixture was allowed to react at 90°C for 4 hours, and the reaction ended as monitored by LCMS. After cooling, the reaction solution was slowly added into water (300 mL). The resulting mixture was extracted with EtOAc (100 mL ×3). The organic phases were combined and washed with saturated sodium chloride solution (300 mL). The combined organic phases were dried over anhydrous sodium sulfate, filtered, and evaporated to dryness under reduced pressure. The residue was purified by column chromatography (PE/EtOAc, 20/1 to 2/1) to afford the target compound 4 (5.4g, 22.0 mmol, 90.4% yield) as a yellow oil. ESI-MS: [M+H]⁺, 246.2.

### (3) Synthesis steps of compound 5

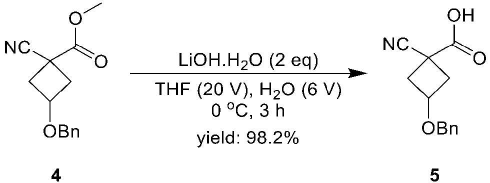

Compound 4 (5.4g, 22.0 mmol, 1 eq) was dissolved in THF (108 mL) and H₂O (32 mL). LiOH.H₂O (1.85g, 44.0 mmol, 2 eq) was added at 0°C. The reaction mixture was allowed to react at 0°C for 3 hours, and the reaction ended as monitored by LCMS. The reaction solution was slowly added into water (100 mL). The mixture was extracted with EtOAc (40 mL) to remove impurities. The pH of the aqueous phase was then adjusted to 4 with 1 M HCl, and then the aqueous phase was extracted with EtOAc (40 mL × 3). The organic phases were combined and washed with saturated brine (200 mL). The combined organic phases were dried over anhydrous sodium sulfate, filtered, and evaporated to dryness under reduced pressure to afford the target compound 5 (5g, 20.4 mmol, 98.2% yield) as a yellow oil. ESI-MS:[M+H]⁺, 232.2.

### (4) Synthesis steps of compound 7

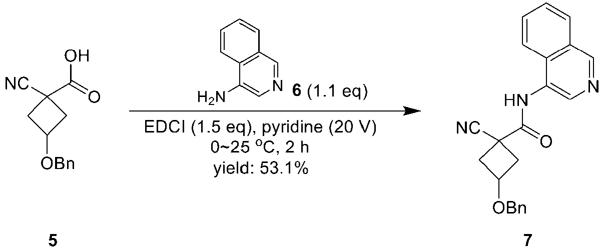

Compound 6 (3.43g, 23.7 mmol, 1.1 eq) and **compound 5** (5g, 21.6 mmol, 1 eq) were dissolved in pyridine (100 mL). EDCI (6.22g, 32.4 mmol, 1.5 eq) was added portionwise at 0°C. The reaction mixture was stirred and allowed to react at 25°C for 2 hours, and the reaction ended as monitored by LCMS. The reaction solution was concentrated under reduced pressure, and then water (100 mL) was added. The reaction solution was extracted with EtOAc (30 mL ×3). The organic phases were combined and washed with saturated sodium chloride solution (100 mL). The combined organic phases were dried over anhydrous sodium sulfate, filtered, and evaporated to dryness under reduced pressure. The residue was purified by column chromatography (SiO₂, PE/EtOAc, 20/1 to 1/1) to afford compound 7 (4.1g, 11.4 mmol, 53.1% yield) as a yellow oil. ESI-MS:[M+H]⁺, 358.2.

### (5) Synthesis steps of compound 8

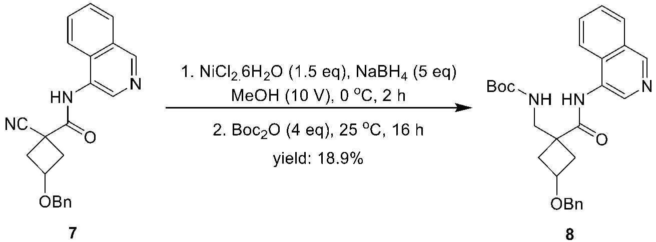

Compound 7 (3.4g, 9.51 mmol, 1 eq) was dissolved in MeOH (34 mL). NiCl₂.6H₂O (3.39g, 14.2 mmol, 1.5 eq) was added at 25°C. After the mixture was allowed to react at 0°C for 10 minutes, NaBH₄ (1.80g, 47.5 mmol, 5 eq) was added portionwise to the above reaction solution. The reaction mixture was stirred and allowed to react at 0°C for 2 hours. The reaction ended as monitored by LCMS. (Boc₂)O (8.30g, 38.1 mmol, 8.74 mL, 4 eq) was added to the above reaction solution. The reaction mixture was allowed to react at 25°C for 16 hours, and the reaction ended as monitored by LCMS. The reaction solution was added into water (100 mL) and filtered under reduced pressure. The filtrate was extracted with EtOAc (50 mL × 3). The organic phases were combined and washed with saturated brine (150 mL). After washing, the organic phases were concentrated under reduced pressure, and the residue was purified by column chromatography (SiO₂, petroleum ether/EtOAc = 20:1 to 0:1) to afford compound 8 (650 mg, 1.80 mmol, 18.9% yield) as a yellow oil. ESI-MS:[M+H]⁺, 462.3.

### (6) Synthesis steps of compound 9

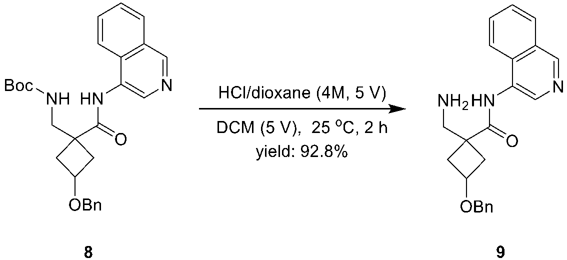

Compound 8 (550 mg, 1.19 mmol, 1 eq) was dissolved in DCM (3 mL). HCl/1,4-dioxane (4 M, 3 mL) was added at 25°C. The reaction mixture was stirred and allowed to react at 25°C for 2 hours, and the reaction ended as monitored by LCMS. The reaction solution was concentrated under reduced pressure, and then water (10 mL) was added. The pH was adjusted to 8 with saturated aqueous sodium carbonate solution. The reaction solution was extracted with EtOAc (2 mL ×3). The organic phases were combined and washed with saturated sodium chloride solution (10 mL). The combined organic phases were dried over anhydrous sodium sulfate, filtered, and evaporated to dryness under reduced pressure to afford compound 9 (400 mg, 1.11 mmol, 92.8% yield) as a yellow solid. ESI-MS:[M+H]⁺, 362.2.

### (7) Synthesis steps of compound 10

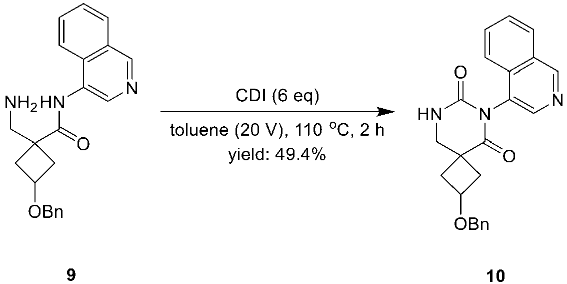

Compound 9 (400 mg, 1.11 mmol, 1 eq) was dissolved in toluene (8 mL). CDI (1.08g, 6.64 mmol, 6 eq) was added at 25°C. The reaction mixture was allowed to react at 110°C for 2 hours, and the reaction ended as monitored by LCMS. The reaction solution was concentrated under reduced pressure, and then water (10 mL) was added. The reaction solution was extracted with EtOAc (2 mL ×3). The organic phases were combined and washed with saturated sodium chloride solution (10 mL). The washed organic phases were dried over anhydrous sodium sulfate, filtered, and evaporated to dryness under reduced pressure. The residue was purified by thin-layer chromatography (SiO₂, EtOAc/Methanol = 10/1) to afford the target compound 10 (212 mg, 547 µmol, 49.4% yield) as a yellow solid. ESI-MS:[M+H]⁺, 388.2.

### (8) Synthesis steps of compound 12

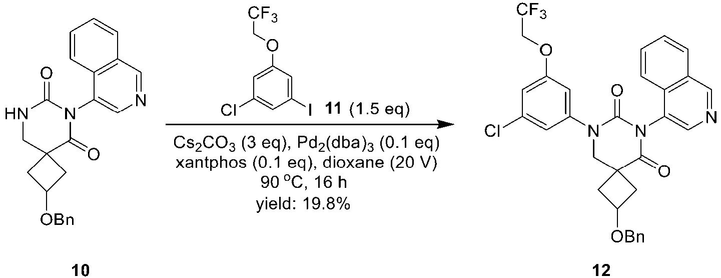

Compound 10 (210 mg, 542 µmol, 1 eq) and compound 11 (273 mg, 813 µmol, 1.5 eq) were dissolved in 1,4-dioxane (4.2 mL). At 25°C, Cs₂CO₃ (529 mg, 1.63 mmol, 3 eq), Xantphos (31.3 mg, 54.2 µmol, 0.1 eq), and Pd₂(dba)₃ (49.6 mg, 54 µmol, 0.1 eq) were added sequentially. The reaction mixture was stirred and allowed to react at 90°C for 16 hours under nitrogen atmosphere, and the reaction ended as monitored by LCMS. The reaction solution was concentrated under reduced pressure, and then water (10 mL) was added. The reaction solution was extracted with EtOAc (2 mL ×3). The organic phases were combined and washed with saturated sodium chloride solution (10 mL). The combined organic phases were dried over anhydrous sodium sulfate, filtered, and evaporated to dryness under reduced pressure. The residue was purified by thin-layer chromatography (SiO₂, PE/EtOAc = 0/1) to afford the target compound 12 (64 mg, 107 µmol, 19.8% yield) as a yellow oil. ESI-MS:[M+H]⁺, 596.2.

### (9) Synthesis steps of compound GDI15-7945

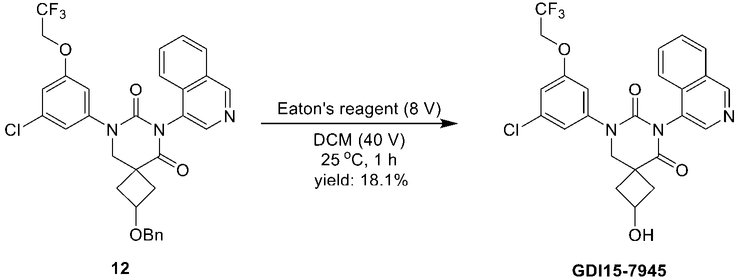

Compound 12 (64 mg, 107 µmol, 1 eq) was dissolved in DCM (2.5 mL). Eaton's reagent (757 mg, 3.18 mmol, 0.5 mL, 29.6 eq) was added at 25°C. The reaction mixture was allowed to react at 25°C for 1 hour, and the reaction ended as monitored by LCMS. The reaction solution was directly added into water (5 mL), and the pH was adjusted to 9 with saturated Na₂CO₃ solution. The mixture was then extracted with DCM (2 mL × 3). The organic phases were combined and concentrated under reduced pressure. The residue was purified by high performance liquid chromatography (column: Waters Xbridge BEH C18 100*30 mm*10 µm; mobile phase: [H₂O (10 mM NH₄HCO₃)-ACN]; gradient: 38%-68%, 8.0 min) to afford the target compound GDI15-7945 (9.8 mg, 19.3 µmol, 18.1% yield, 100% purity) as a white solid. ESI-MS:[M+H]⁺, 506.1.

¹H NMR (400 MHz, DMSO) δ 9.37 (s, 1 H), 8.48 - 8.41 (m, 1 H), 8.24 (d, *J* = 8.1 Hz, 1 H), 7.89 - 7.78 (m, 2 H), 7.75 (t, *J* = 7.4 Hz, 1 H), 7.25 (s, 1 H), 7.15 (dd, *J* = 1.9, 3.4 Hz, 2 H), 5.33 (s, 1 H), 4.87 (q, *J* = 8.8 Hz, 2 H), 4.28 - 4.15 (m, 3 H), 2.91 - 2.79 (m, 2 H), 2.18 - 2.00 (m, 2 H).

### Example 36: Preparation of Compound GDI15-8075

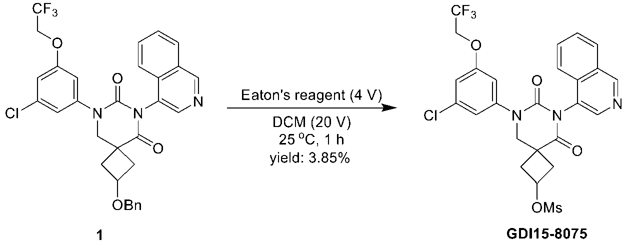

Compound 10 (53.0 mg, 88.9 µmol, 1 eq) was dissolved in DCM (1 mL). Eaton's reagent (0.2 mL) was added at 25°C. The reaction mixture was allowed to react at 25°C for 1 hour, and the reaction ended as monitored by LCMS. The reaction solution was concentrated under reduced pressure, and then water (10 mL) was added. The pH of the reaction solution was adjusted to 8 with saturated aqueous NaHCO₃ solution. The reaction solution was extracted with DCM (2 mL ×3). The organic phases were combined and washed with saturated sodium chloride solution (3 mL). The combined organic phases were dried over anhydrous sodium sulfate, filtered, and evaporated to dryness under reduced pressure. The residue was purified by high performance liquid chromatography (column: Waters xbridge 150*25 mm*10 µm; mobile phase: [H₂O (10 mM NH₄HCO₃)-ACN]; gradient: 30%-60%, 8.0 min) to afford the target compound GDI15-8075 (2 mg, 3.42 µmol, 3.85% yield) as a yellow solid. ESI-MS: [M+H]⁺, 584.1.

¹H NMR (400 MHz, Chloroform-*d*₃) δ 9.25 (s, 1 H), 8.34 (s, 1 H), 8.02 (d, *J* = 8.3 Hz, 1 H), 7.73 - 7.66 (m, 1 H), 7.63 - 7.57 (m, 1 H), 7.47 (d, *J* = 8.1 Hz, 1 H), 7.02 (t, *J* = 1.7 Hz, 1 H), 6.87 (t, *J* = 2.1 Hz, 1 H), 6.81 (t, *J* = 1.9 Hz, 1 H), 5.19 (t, *J* = 6.4 Hz, 1 H), 4.28 (q, *J* = 7.9 Hz, 2 H), 4.18 - 4.00 (m, 2 H), 3.22 - 3.13 (m, 2 H), 3.01 - 2.95 (m, 3 H), 2.60 (d, *J=* 6.0 Hz, 1 H), 2.49 - 2.37 (m, 1 H).

### Example 37: Preparation of Compound GDI15-8392

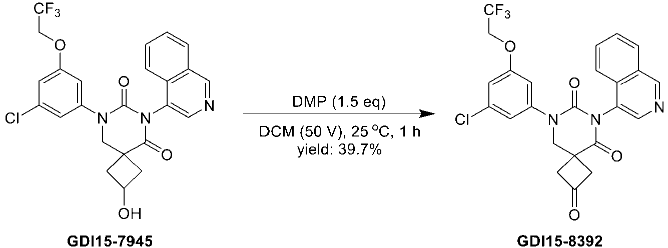

Compound **GDI15-7945** (10 mg, 19.7 µmol, 1 eq) was dissolved in DCM (0.5 mL). DMP (12.5 mg, 29.6 µmol, 9.19 µL, 1.5 eq) was added at 25°C. The reaction mixture was allowed to react at 25°C for 1 hour, and the reaction ended as monitored by LCMS. Water (2 mL) was added to the reaction mixture, and the mixture was extracted with DCM (2 mL × 3). The organic phases were combined and concentrated under reduced pressure. The residue was purified by high performance liquid chromatography (column: Waters Xbridge BEH C18 100*30 mm*10 µm; mobile phase: [H₂O (10 mM NH₄HCO₃)-ACN]; gradient: 40%-70%, 8.0 min) to afford the target compound GDI15-8392 (4.1 mg, 7.85 µmol, 39.7% yield, 96.9% purity) as a white solid. ESI-MS: [M+H] ⁺, 506.2.

¹H NMR (400 MHz, DMSO) δ 9.39 (s, 1 H), 8.55 (s, 1 H), 8.25 (d, *J* = 8.3 Hz, 1 H), 7.92 - 7.81 (m, 2 H), 7.80 - 7.71 (m, 1 H), 7.30 (s, 1 H), 7.18 (d, *J* = 6.3 Hz, 2 H), 4.86 (q, *J* = 8.6 Hz, 2 H), 4.53 - 4.44 (m, 1 H), 4.43 - 4.37 (m, 1 H), 3.73 - 3.60 (m, 2 H), 3.47 (dd, *J* = 6.7, 12.4 Hz, 2 H).

### Example 38: Preparation of Compound GDI15-8505

### (1) Synthesis steps of compound 1

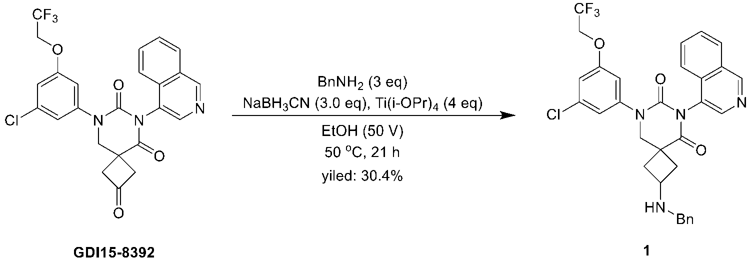

Compound GDI15-8392 (40 mg, 79.3 µmol, 1 eq), BnNH₂ (25.5 mg, 238 µmol, 25.9 µL, 3 eq), and Ti(i-PrO)₄ (90.2 mg, 317 µmol, 93.7 µL, 4 eq) were successively dissolved in EtOH (2 mL). The reaction mixture was allowed to react at 50°C for 5 hours, and then cooled to 25°C. NaBH₃CN (14.9 mg, 238 µmol, 3 eq) was added, and the reaction mixture was further allowed to react at 50°C for 16 hours. The reaction ended as monitored by LCMS. The reaction mixture was evaporated to dryness under reduced pressure and purified by high performance liquid chromatography (column: Waters Xbridge BEH C18 100*30 mm*10 µm; mobile phase: [H₂O (10 mM NH₄HCO₃)-ACN]; gradient: 35%-70%, 8.0 min) to afford the target compound 1 (16 mg, 2.58 µmol, 3.25% yield, 96% purity) as a white solid. ESI-MS: [M+H]⁺, 596.2.

¹H NMR (400 MHz, DMSO) δ 9.37 (s, 1 H), 8.44 (d, *J* = 13.6 Hz, 1 H), 8.24 (d, *J* = 8.0 Hz, 1 H), 7.88 - 7.82 (m, 1 H), 7.80 - 7.72 (m, 2 H), 7.32 - 7.23 (m, 3 H), 7.27 - 7.23 (m, 1 H), 7.23 - 7.18 (m, 1 H), 7.17 - 7.13 (m, 2 H), 5.76 (s, 1 H), 4.89 - 4.82 (m, 2 H), 4.23 (q, *J* = 12.4 Hz, 1 H), 4.17 (s, 1 H), 3.63 (d, *J* = 4.6 Hz, 2 H), 3.48 (t, *J* = 7.7 Hz, 1 H), 2.80 - 2.73 (m, 1 H), 2.34 - 2.22 (m, 2 H), 2.12 - 2.03 (m, 1 H), 1.98 (dd, *J* = 7.4, 12.6 Hz, 1 H).

### (2) Synthesis steps of compound GDI15-8505

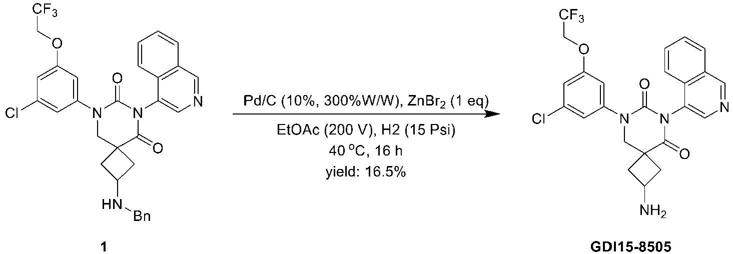

Under an argon atmosphere, Pd/C (90.1 mg, 84.7 µmol, 10% purity) was added to the reaction flask, and then EtOAc (6 mL) was added along a wall of the flask. Compound **1** (30 mg, 50.4 µmol, 1 eq) and ZnBr₂ (11.3 mg, 50.4 µmol, 2.52 µL, 1 eq) were successively added under the argon atmosphere. The reaction mixture was allowed to react at 40°C for 16 hours under a hydrogen atmosphere, and the reaction ended as monitored by LCMS. The reaction mixture was filtered through celite, and the filtrate was evaporated to dryness under reduced pressure. The residue was purified by high performance liquid chromatography (column: Waters xbridge 150*25 mm*10 µm; mobile phase: [H₂O (10 mM NH₄HCO₃)-ACN]; gradient: 27%-52%, 8.0 min) to afford the target compound GDI15-8505 (5 mg, 8.32 µmol, 16.5% yield, 84% purity) as a white solid. ESI-MS: [M+H]+, 505.2.

1H NMR (400 MHz, DMSO) δ 9.37 (s, 1 H), 8.48 - 8.40 (m, 1 H), 8.24 (d, *J* = 8.4 Hz, 1 H), 7.88 - 7.76 (m, 2 H), 7.76 - 7.72 (m, 1 H), 7.29 - 7.23 (m, 1 H), 7.18 - 7.12 (m, 2 H), 4.90 - 4.82 (m, 2 H), 4.25 - 4.13 (m, 2 H), 3.60 - 3.39 (m, 1 H), 2.84 - 2.75 (m, 1 H), 2.69 - 2.63 (m, 1 H), 2.29 - 2.12 (m, 1 H), 2.05 - 1.82 (m, 2 H).

### Example 39: Preparation of Compound GDI15-8660

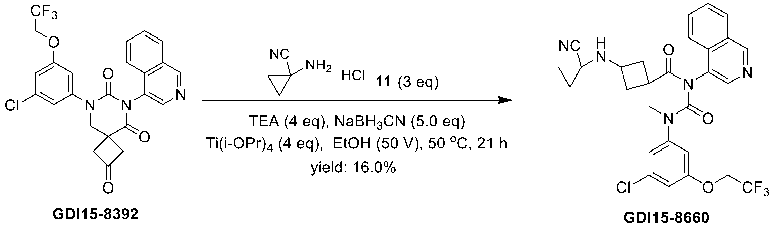

Compound 11 (49.4 mg, 417 µmol, 3 eq, HCl) was dissolved in EtOH (3.5 mL). TEA (56.2 mg, 556 µmol, 77.4 µL, 4 eq) was added at 25°C, and the pH of the reaction solution was determined to be 7-8. Then compound GDI15-8392 (70 mg, 139 µmol, 1 eq) and Ti(i-PrO)₄ (158 mg, 556 µmol, 164 µL, 4 eq) were added successively to the above reaction solution. After the reaction mixture was stirred at 50°C for 5 hours, NaBH₃CN (43.7 mg, 695 µmol, 5 eq) was added to the mixture. The reaction mixture was further allowed to react at 50°C for 16 hours, and the reaction ended as monitored by LCMS. The reaction solution was concentrated under reduced pressure, and H₂O (5 mL) was added. The reaction solution was extracted with EtOAc (5 mL ×3). The organic phases were combined and washed with saturated sodium chloride solution (5 mL). The combined organic phases were concentrated under reduced pressure and purified by high performance liquid chromatography (column: Phenomenex Luna C18 100*40 mm*5 µm; mobile phase: [H₂O (0.2% FA)-ACN] to afford the target compound GDI15-8660 (12.7 mg, 22.3 µmol, 16.0% yield, 100% purity) as a white solid. ESI-MS: [M+H]+, 570.1.

1H NMR (400 MHz, Chloroform-*d*₃) δ 9.32 (s, 1 H), 8.50 - 8.36 (m, 1 H), 8.10 (d, *J* = 8.0 Hz, 1 H), 7.80 (t, *J* = 7.4 Hz, 1 H), 7.74 - 7.66 (m, 1 H), 7.64 - 7.52 (m, 1 H), 7.21 - 7.06 (m, 1 H), 7.02 - 6.94 (m, 1 H), 6.92 - 6.77 (m, 1 H), 4.42 - 4.30 (m, 2 H), 4.23 - 4.11 (m, 1 H), 4.10 - 4.00 (m, 1 H), 3.94 - 3.67 (m, 1 H), 3.20 - 3.11 (m, 0.5 H), 2.80 (ddd, *J* = 3.8, 7.6, 11.8 Hz, 0.5 H), 2.73 - 2.64 (m, 1 H), 2.62 - 2.55 (m, 1 H), 2.23 - 1.96 (m, 1 H), 1.32 - 1.16 (m, 2 H), 1.13 - 0.99 (m, 2 H).

### Example 40: Preparation of Compound GDI15-8661

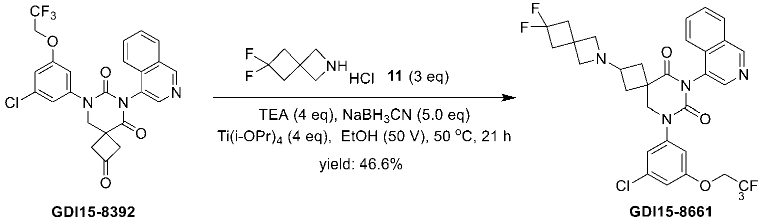

Compound 11 (50.5 mg, 298 µmol, 3 eq, HCl) was dissolved in EtOH (2.5 mL). TEA (40.2 mg, 397 µmol, 55.3 µL, 4 eq) was added at 20°C, and the pH of the reaction solution was determined to be 7-8. Then compound GDI15-8392 (50 mg, 99.2 µmol, 1 eq) and Ti(i-PrO)₄ (113 mg, 397 µmol, 117 µL, 4 eq) were added successively to the above reaction solution. After the reaction mixture was stirred at 50°C for 5 hours, NaBH₃CN (31.2 mg, 496 µmol, 5 eq) was added to the mixture. The reaction mixture was further allowed to react at 50°C for 16 hours, and the reaction ended as monitored by LCMS. The reaction solution was concentrated under reduced pressure, and H₂O (5 mL) was added. The reaction solution was extracted with EtOAc (5 mL ×3). The organic phases were combined and washed with saturated sodium chloride solution (5 mL). The combined organic phases were concentrated under reduced pressure and purified by high performance liquid chromatography (column: Phenomenex Luna C18 100*40 mm*5 µm; mobile phase: [H₂O (0.2% FA)-ACN] to afford the target compound GDI15-8661 (28.7 mg, 46.2 µmol, 46.6% yield, 100% purity) as a white solid. ESI-MS: [M+H]+, 621.2.

¹H NMR (400 MHz, Chloroform-*d*₃) δ 9.32 (s, 1 H), 8.41 (s, 1 H), 8.09 (d, *J* = 8.0 Hz, 1 H), 7.81 - 7.72 (m, 1 H), 7.71 - 7.63 (m, 1 H), 7.55 (dd, *J* = 3.7, 8.4 Hz, 1 H), 7.17 - 7.09 (m, 1 H), 7.03 - 6.95 (m, 1 H), 6.88 (s, 1 H), 4.41 - 4.32 (m, 2 H), 4.31 - 4.22 (m, 1 H), 4.16 - 4.03 (m, 1 H), 3.48 - 3.38 (m, 4 H), 2.84 - 2.57 (m, 7 H), 2.41 - 2.33 (m, 0.5 H), 2.25 - 2.15 (m, 1 H), 2.10 - 2.04 (m, 0.5 H).

### Example 41: Preparation of Compound GDI15-7945

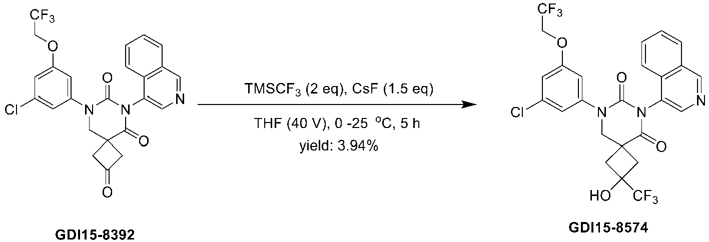

Compound GDI15-8392 (50 mg, 99.2 µmol, 1 eq) was dissolved in THF (2 mL). CsF (22.6 mg, 149 µmol, 1.5 eq) and TMSCF₃ (28.2 mg, 198 µmol, 2 eq) were added successively at 0°C. The reaction mixture was allowed to react at 25°C for 5 hours under nitrogen atmosphere. The reaction solution was concentrated under reduced pressure and purified by high performance liquid chromatography (column: WePure Biotech XP tC18 150×40×7 µm; mobile phase: [H₂O (10 mM NH₄HCO₃)-CN]; gradient: 45%-75%, 8.0 min) to afford a TMS-protected product. After lyophilization twice, the TMS groups were completely removed to obtain the final product GDI15-8574 (2.5 mg, 4.3 µmol, 89.8% purity, 3.94% yield) as a white solid. ESI-MS:[M+H]⁺, 574.1.

¹H NMR (400 MHz, DMSO) δ 9.39 (s, 1 H), 8.50 (s, 1 H), 8.26 (d, *J* = 8.1 Hz, 1 H), 7.86 (d, *J* = 3.8 Hz, 2 H), 7.76 (td, J = 4.0, 8.1 Hz, 1 H), 7.28 (s, 1 H), 7.17 (s, 2 H), 6.84 (s, 1 H), 4.86 (q, *J* = 8.8 Hz, 2 H), 4.11 (s, 2 H), 2.85 - 2.59 (m, 4 H).

### Example 42: Preparation of Compound GDI15-7597

### (1) Synthesis steps of compound 2

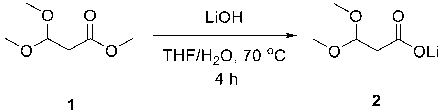

THF (20 mL) was added to a mixture of compound 1 (2.00g, 13.5 mmol), LiOH (0.62g, 14.9 mmol) and H₂O (0.49g, 27.0 mmol). The reaction mixture was stirred and allowed to react at 70°C for 4 hours, and then lyophilized to afford the target compound 2.

**¹H NMR (400 MHz, DMSO)** *δ* 4.72 (t, *J* = 5.6 Hz, 1 H), 3.20 (s, 6 H), 2.24 (d, *J* = 5.6 Hz, 2 H).

### (2) Synthesis steps of compound 4

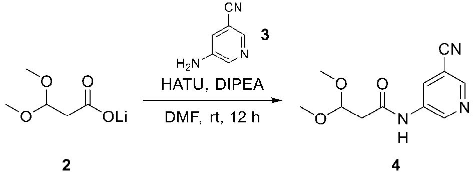

HATU (5.40g, 14.2 mmol), DIPEA (2.75g, 21.3 mmol) and compound 3 (0.85g, 7.1 mmol) were slowly added to a solution of compound 3 (1g, 0.0071 mol) in DMF (5 mL). The mixture was stirred and allowed to react at room temperature for 12 hours under nitrogen atmosphere. After the reaction ended, water (50 mL) was added to the mixture, and the mixture was extracted with EtOAc (100 mL × 3). The organic phases were combined and washed with saturated brine, dried over anhydrous sodium sulfate, filtered, and evaporated to dryness under reduced pressure. The residue was further purified by column chromatography (EtOAc/PE, 40% to 50%) to afford compound 4 (1.0g, 60.56% yield) as a colorless oil. ESI-MS: [M+H]⁺, 236.0.

### (3) Synthesis steps of compound 6

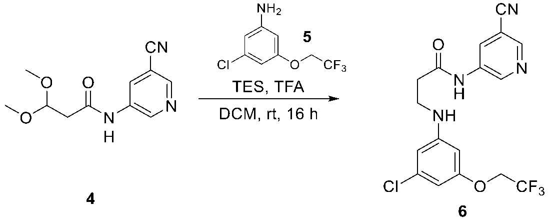

Compound 5 (577.97 mg, 2.55 mmol), TFA (3 mL) and Et₃SiH (741.40 mg, 6.38 mmol) were slowly added to a solution of compound 4 (600 mg, 2.55 mmol) in DCM (6 mL). The mixture was then stirred and allowed to react at room temperature for 16 hours. After the reaction ended, the aqueous Na₂CO₃ solution was added to the mixture to adjust the pH to 8-9, and the mixture was extracted with EtOAc (100 mL × 3). The organic phases were combined and washed with saturated brine, dried over anhydrous sodium sulfate, filtered, and evaporated to dryness. The residue was purified by column chromatography (EtOAc/PE, 40% to 42%) to afford compound 6 (400mg, 39.23% yield) as a white solid. ESI-MS: [M+H]⁺, 399.0.

### (3) Synthesis steps of compound GDI15-7597

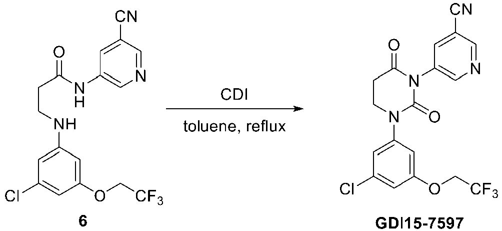

CDI (730.02 mg, 4.50 mmol) was added to a solution of compound 6 (300.00 mg, 0.75 mmol) in toluene (3 mL), and the mixture was stirred and allowed to react at 110°C for 2.5 hours. After the reaction ended, water (100 mL) was added to the mixture, and the mixture was extracted with EtOAc (100 mL × 3). The organic phases were combined, washed with saturated brine, dried over anhydrous sodium sulfate, filtered, and evaporated to dryness. The residue was purified by column chromatography (EtOAc/PE, 30% to 35%) to afford compound GDI15-7597 (105.62 mg, 33.06% yield) as a white solid. ESI-MS: [M+H] ⁺, 425.1.

**¹H NMR (400 MHz, DMSO)** *δ* 9.04 (d, *J* = 1.6 Hz, 1H), 8.82 (d, *J* = 2.0 Hz, 1H), 8.37 (m, 1H), 7.24 (t, 1H), 7.17 - 7.11 (m, 2H), 4.85 (q, *J* = 8.8 Hz, 2H), 3.98 (t, *J* = 6.4 Hz, 2H), 3.04 (t, *J* = 6.4 Hz, 2H).

### Example 43: Preparation of Compound GDI15-7639

### (1) Synthesis steps of compound 3

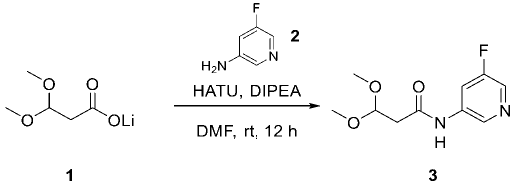

HATU (5.40g, 0.0142 mol), DIPEA (2.56g, 0.0213 mol) and compound 2 (0.80g, 0.0071 mol) were slowly added to a solution of compound 1 (1.00g, 0.0071 mol) in DMF (10 mL) at room temperature under nitrogen atmosphere. The mixture was further allowed to react at room temperature for 12 hours. The reaction ended as monitored by LCMS. After the reaction ended, water (50 mL) was added to the mixture, and the mixture was extracted with EtOAc (50 mL ×3). The organic phases were combined and washed with saturated brine, dried over anhydrous sodium sulfate, filtered, and evaporated to dryness. The residue was further purified by column chromatography (EtOAc/PE, 30% to 35%) to afford compound 3 (1g, 61.97% yield) as a yellow oil. ESI-MS: [M+H]⁺, 228.9.

### (2) Synthesis steps of compound 5

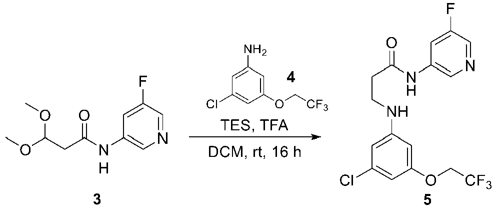

Compound 4 (595.74 mg, 2.63 mmol), TFA (3 mL) and Et₃SiH (1079.66 mg, 6.57 mmol) were slowly added to a solution of compound 3 (600 mg, 2.63 mmol) in DCM (6 mL) at room temperature under nitrogen atmosphere. The mixture was further allowed to react at room temperature for 16 hours. After the reaction ended, the saturated aqueous sodium bicarbonate solution was added to the mixture to adjust the pH to 8-9, and the mixture was extracted with EtOAc (100 mL × 3). The organic phases were combined and washed with saturated brine, dried over anhydrous sodium sulfate, filtered, and evaporated to dryness. The residue was purified by column chromatography (EtOAc/PE, 30% to 35%) to afford compound 5 (400mg, 38.74% yield) as a yellow solid. ESI-MS: [M+H]⁺, 392.1.

### (3) Synthesis steps of compound GDI15-7639

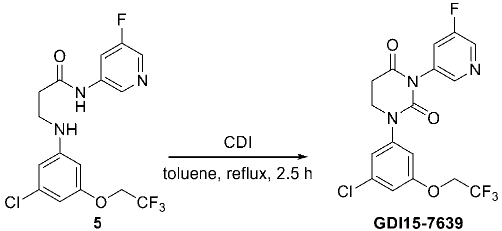

CDI (743.06 mg, 4.58 mmol) was added to a solution of compound 5 (300.00 mg, 0.7638 mmol) in toluene (3 mL), and the mixture was heated 110°C and allowed to react at for 2.5 hours. After the reaction ended, water (50 mL) was added to the mixture, and the mixture was extracted with EtOAc (50 mL × 3). The organic phases were combined, washed with saturated brine, dried over anhydrous sodium sulfate, filtered, and evaporated to dryness. The residue was purified by column chromatography (EtOAc/PE, 30% to 35%) to afford compound GDI-7639 (47.03 mg, 14.70% yield) as a white solid. ESI-MS: [M+H]⁺, 418.0.

**¹H NMR (400 MHz, DMSO)** *δ* 8.62 (d, *J* = 2.4 Hz, 1H), 8.40 (t, 1H), 7.82 (dt, *J* = 9.6, 2.0 Hz, 1H), 7.23 (t, 1 H), 7.14 (dd, *J* = 3.8, 1.8 Hz, 2H), 4.85 (q, *J* = 8.8 Hz, 2H), 3.97 (t, *J* = 6.6 Hz, 2H), 3.02 (t, *J* = 6.4 Hz, 2H).

### Example 44: Preparation of Compound GDI15-7820

### (1) Synthesis steps of compound 3

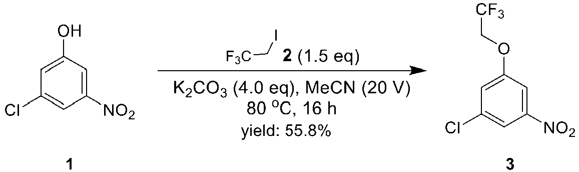

Compound 1 (2g, 11.5 mmol, 1 eq) and **compound 2** (3.63g, 17.3 mmol, 1.70 mL, 1.5 eq) were successively dissolved in MeCN (40 mL). K₂CO₃ (6.37g, 46.1 mmol, 4 eq) was added at 25°C. The reaction mixture was allowed to react at 80°C for 16 hours, and the reaction ended as monitored by LCMS. The reaction solution was concentrated under reduced pressure, and then water (20 mL) was added. The reaction solution was extracted with EtOAc (10 mL ×3). The organic phases were combined and washed with saturated sodium chloride solution (15 mL). The combined organic phases were dried over anhydrous sodium sulfate, filtered, and evaporated to dryness under reduced pressure. The residue was purified by column chromatography (SiO₂, Petroleum ether/EtOAc = 20:1 to 5:1) to afford the target compound 3 (1.8g, 6.43 mmol, 55.8% yield, 91.3% purity) as a yellow oil. ESI-MS:[M+H]⁺, 255.9.

### (2) Synthesis steps of compound 4

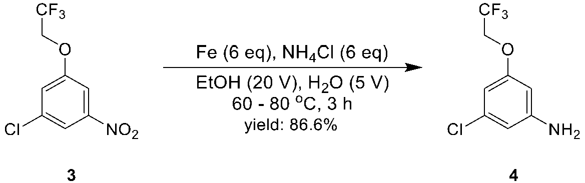

Compound 3 (1.8g, 7.04 mmol, 1 eq) was dissolved in EtOH (36 mL) and H₂O (9 mL). NH₄Cl (2.26g, 42.3 mmol, 6 eq) was added at 25°C. After warming to 60°C, iron powder (2.36g, 42.3 mmol, 6 eq) was added portionwise. The reaction mixture was further allowed to react at 80°C for 3 hours, and the reaction ended as monitored by LCMS. The reaction solution was filtered while hot, concentrated under reduced pressure, and evaporated to dryness. Water (10 mL) was added, and the mixture was extracted with EtOAc (5 mL × 3). The organic phases were combined, dried over anhydrous sodium sulfate, filtered under reduced pressure, and evaporated to dryness. The residue was purified by column chromatography (SiO₂, Petroleum ether/EtOAc = 20:1 to 3:1) to afford the target compound 4 (1.6g, 6.10 mmol, 86.6% yield, 86.0% purity) as a yellow oil. ESI-MS:[M+H]⁺, 226.1.

### (3) Synthesis steps of compound 5

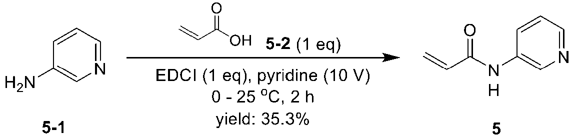

Compound **5**-1 (2.0g, 21.3 mmol, 1 eq) and compound **5**-2 (1.53g, 21.3 mmol, 1 eq) were dissolved in pyridine (20 mL). EDCI (4.07g, 21.3 mmol, 1 eq) was added portionwise at 0°C. The reaction mixture was allowed to react at 25°C for 2 hours, and the reaction ended as monitored by LCMS. The reaction solution was concentrated under reduced pressure, and then water (5 mL) was added. The reaction solution was extracted with EtOAc (3 mL ×3). The organic phases were combined and washed with saturated sodium chloride solution (5 mL). The combined organic phases were dried over anhydrous sodium sulfate, filtered, and evaporated to dryness under reduced pressure. The residue was purified by column chromatography (SiO₂, Petroleum ether/EtOAc = 20:1 to 5:1) to afford the target compound 5 (1.11g, 7.49 mmol, 35.3% yield) as a yellow oil. ESI-MS:[M+H]⁺, 149.1.

### (4) Synthesis steps of compound 6

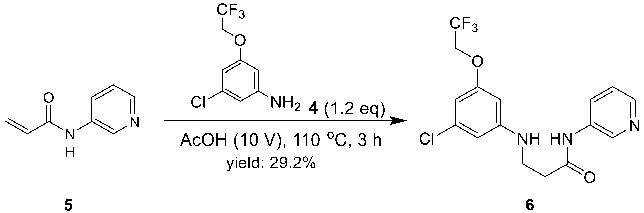

Compound 5 (0.3g, 2.02 mmol, 1 eq) and compound **4** (548 mg, 2.43 mmol, 1.2 eq) were successively dissolved in AcOH (3 mL). The reaction mixture was allowed to react at 110°C for 3 hours, and the reaction ended as monitored by LCMS. The reaction solution was concentrated under reduced pressure, and then water (5 mL) was added. The reaction solution was extracted with EtOAc (3 mL ×3). The organic phases were combined and washed with saturated sodium chloride solution (5 mL). The combined organic phases were dried over anhydrous sodium sulfate, filtered, and evaporated to dryness under reduced pressure. The residue was purified by thin-layer chromatography (SiO₂, Petroleum ether/EtOAc = 1:1) to afford the target compound 6 (0.24g, 591 µmol, 29.2% yield, 92% purity) as a yellow solid. ESI-MS:[M+H]⁺, 374.1.

### (5) Synthesis steps of compound GDI15-7820

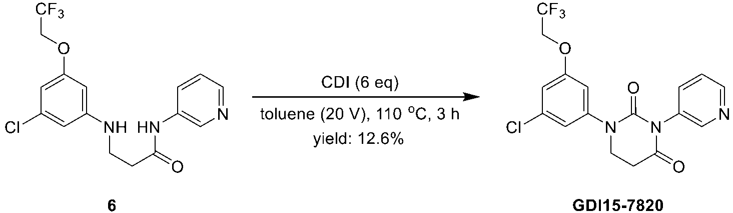

Compound 6 (120 mg, 2.02 mmol, 1 eq) and the compound CDI (315 mg, 1.94 mmol, 6 eq) were dissolved in toluene (2.4 mL). The reaction mixture was allowed to react at 110°C for 3 hours, and the reaction ended as monitored by LCMS. The reaction solution was concentrated under reduced pressure, water (5mL) was added to the residue, and the mixture was extracted with EtOAc (3 mL × 3). The organic phases were combined, filtered under reduced pressure, and evaporated to dryness. Then the residue was purified by high performance liquid chromatography (column: Waters Xbridge BEH C18 100*30 mm*10 µm; mobile phase: [H₂O (10 mM NH₄HCO₃)-ACN]; gradient: 21%-51%, 8.0 min, SiO₂, Petroleum ether/EtOAc, 1/1) to afford the target compound GDI15-7820 (16.4 mg, 40.4 µmol, 12.6% yield, 98.6% purity) as a yellow solid. ESI-MS:[M+H]+, 400.1.

¹H NMR (400 MHz, DMSO) δ 8.55 (dd, *J* = 1.5, 4.8 Hz, 1 H), 8.46 (d, *J* = 2.2 Hz, 1 H), 7.76 - 7.70 (m, 1 H), 7.50 (dd, *J* = 4.8, 8.1 Hz, 1 H), 7.22 (t, *J* = 1.7 Hz, 1 H), 7.13 (q, *J* = 2.1 Hz, 2 H), 4.84 (d, *J* = 8.8 Hz, 2 H), 3.97 (t, *J* = 6.5 Hz, 2 H), 3.02 (t, *J* = 6.5 Hz, 2 H).

### Example 45: Preparation of Compound GDI15-7958

### (1) Synthesis steps of compound 3

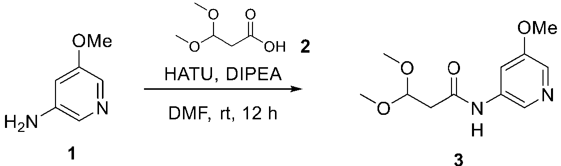

DMF (5 mL) was added to a mixture of compound 1 (500 mg, 4.03 mmol, 1 eq) and compound 2 (540 mg, 4.03 mmol, 1 eq). Under the nitrogen atmosphere, HATU (2.29g, 6.05 mmol) and DIPEA (1.56g, 12.09 mmol) were further added at 0°C, and the mixture was further allowed to react at room temperature for 12 hours. After the reaction ended, water (10 mL) was added to the mixture, and the mixture was extracted with EtOAc (10 mL × 3). The organic phases were combined and washed with saturated brine, dried over anhydrous sodium sulfate, filtered, and evaporated to dryness under reduced pressure. The residue was purified by column chromatography (MeOH/DCM, 0% to 2%) to afford compound 3 (600 mg, 62% yield) as a brown oil. ESI-MS: [M+H] +, 241.05.

### (2) Synthesis steps of compound 5

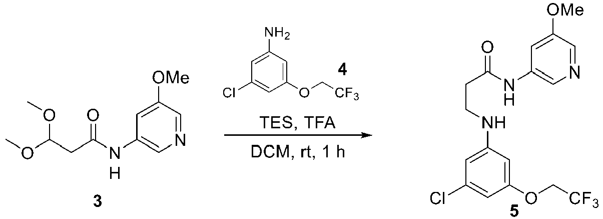

DCM (5 mL) was added to a mixture of compound 3 (550 mg, 2.28 mmol) and compound 4 (513 mg, 2.28 mmol). Under nitrogen atmosphere, Et₃SiH (663 mg, 5.7 mmol) and TFA (2.28 mL) were further added at room temperature, and the mixture was stirred and allowed to react at room temperature for 1 hour. After the reaction ended, aqueous NaHCO₃ solution was added to the mixture to adjust the pH to 8-9. The mixture was filtered, and the filter cake was collected and washed with water to afford compound 5 (600 mg, 71% yield) as a white solid. ESI-MS: [M+H] +, 404.00.

### (3) Synthesis steps of compound GDI15-7958

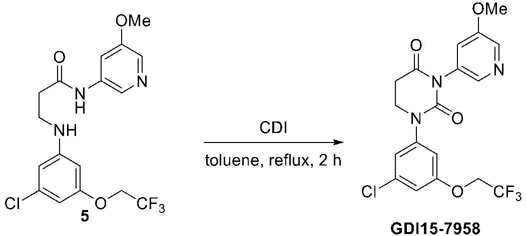

Toluene (5 mL) was added to compound 5 (300 mg, 0.74 mmol), then CDI (722 mg, 4.45 mmol) was added, and the mixture was stirred and allowed to react at 100°C for 2 hours. After the reaction ended, the mixture was concentrated. The residue was further purified by prep-HPLC (Gemini 5 µm C18 column, 150*21.2 mm, eluting with 75% to 95% MeCN/H₂O containing 0.1% TFA) to afford compound GDI15-7958 (17.97 mg, 6.25% yield) as a white solid.

ESI-MS: [M+H] +, 430.00. **¹H NMR** (400 MHz, DMSO) δ 8.31 (d, *J* = 2.8 Hz, 1 H), 8.09 (d, *J* = 1.6 Hz, 1 H), 7.43 - 7.38 (m, 1 H), 7.23 (t, *J* = 1.6 Hz, 1 H), 7.13 (dd, *J* = 3.2, 2.0 Hz, 2 H), 4.85 (q, *J* = 8.8 Hz, 2 H), 3.97 (t, *J* = 6.4 Hz, 2 H), 3.84 (s, 3 H), 3.01 (t, *J* = 6.4 Hz, 2 H).

### Example 46: Preparation of Compound GDI15-7987

### (1) Synthesis steps of compound 3

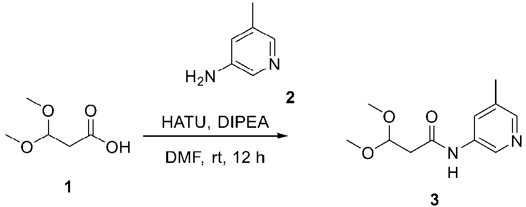

DMF (8 mL) was added to compound 1 (800 mg, 7.41 mmol, 1 eq) and compound 2 (1g, 7.41 mmol, 1 eq). Under the nitrogen atmosphere, HATU (4.25g, 11.1 mmol) and DIPEA (2.87g, 22.2 mmol) were further added at 0°C, and the mixture was stirred and allowed to react at room temperature for 12 hours. After the reaction ended, water (20 mL) was added to the mixture, and the mixture was extracted with EtOAc (30 mL × 3). The organic phases were combined and washed with saturated brine, dried over anhydrous sodium sulfate, filtered, and evaporated to dryness. The residue was purified by column chromatography (MeOH/DCM, 0% to 5%) to afford compound 3 (1.3g, 78% yield) as a red solid. ESI-MS: [M+H] +, 242.95.

### (2) Synthesis steps of compound 5

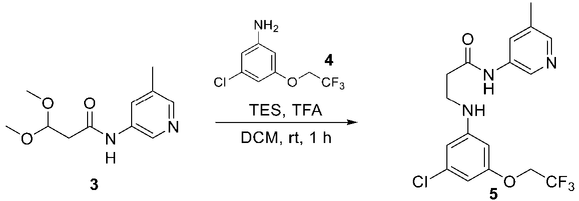

DCM (5 mL) was added to a mixture of compound 3 (300 mg, 1.34 mmol) and compound 4 (302 mg, 1.34 mmol). Under nitrogen atmosphere, Et₃SiH (390 mg, 3.35 mmol) and TFA (1.34 mL) were slowly added at room temperature, and the mixture was further allowed to react at room temperature for 1 hour. After the reaction ended, aqueous NaHCO₃ solution was added to the mixture to adjust the pH to 8-9. The mixture was filtered, and the filter cake was washed with water to afford compound 5 (350 mg, 67% yield) as a white solid. ESI-MS: [M+H] +, 388.05.

### (3) Synthesis steps of compound GDI15-7987

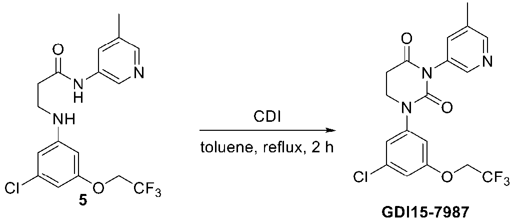

Toluene (5 mL) was added to compound 5 (350 mg, 0.90 mmol), then CDI (877 mg, 5.41 mmol) was added, and the mixture was stirred and allowed to react at 100°C for 2 hours. After the reaction ended, the mixture was concentrated. The residue was further purified by prep-HPLC (Gemini 5 µm C18 column, 150*21.2 mm, eluting with 75% to 95% MeCN/H₂O containing 0.1% TFA) to afford compound GDI15-7987 (52.11 mg, 13.94% yield) as a white solid. ESI-MS: [M+H] +, 414.00.

**¹H NMR** *J* = 7.6 Hz, 1 H), 7.69 (s, 1 H), 7.22 (t, *J* = 1.6 Hz, 1 H), 7.15 - 7.07 (m, 2 H), 4.83 (t, *J* = 8.8 Hz, 2 H), 3.97 (t, *J =* 6.4 Hz, 2 H), 3.01 (t, *J* = 6.4 Hz, 2 H), 2.36 (s, 3 H).

### Example 47: Preparation of Compound GDI15-8050

### (1) Synthesis steps of compound 3

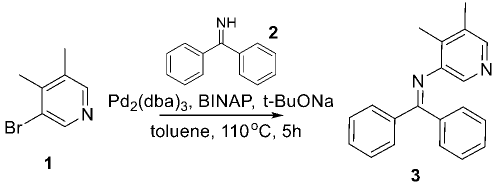

Compound 1 (1g, 5.4 mmol), compound 2 (1.17g, 6.4 mmol), t-BuOK (1.82g, 16.2 mmol), BINAP (1.34g, 2.1 mmol), and Pd₂(dba)₃ were dissolved in toluene (25 mL). The mixture was stirred and allowed to react at 90°C for 16 hours under nitrogen atmosphere. The reaction ended as monitored by LCMS. After the mixture was cooled to room temperature, water (50 mL) was added, and the mixture was extracted with EtOAc (50 mL × 3). The organic phases were combined, washed with brine, dried over anhydrous sodium sulfate, filtered, and evaporated to dryness. The residue was purified by column chromatography (DCM/TEA, 200v:1v) to afford compound 3 (1.4g, 4.9 mmol, 90.74% yield) as a yellow solid. ESI-MS: [M+H] +, 287.15.

### (2) Synthesis steps of compound 4

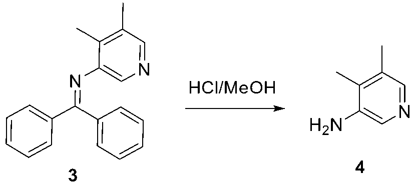

HCl/MeOH (20 mL) was added to compound 3 (1.4g, 4.9 mmol) at room temperature under nitrogen atmosphere, and the mixture was further stirred at room temperature for 2 hours. After the reaction ended, the mixture was concentrated and evaporated to dryness in vacuo to afford the crude product, compound 4 (520 mg, 87.76% yield) as a yellow solid. ESI-MS:[M+H]+, 123.0.

### (3) Synthesis steps of compound 6

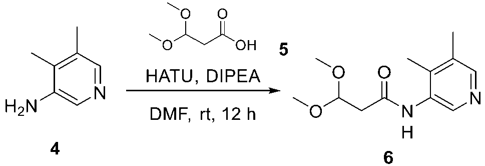

DMF (6 mL) was added to compound 4 (520 mg, 4.26 mmol), and then compound 5 (628 mg, 4.68 mmol), DIPEA (1.65g, 12.77 mmol), and HATU (2.4g, 6.38 mmol) were further added. The mixture was stirred at room temperature for reaction for 16 hours under nitrogen atmosphere. After the reaction ended, water (10 mL) was added to the mixture, and the mixture was extracted with EtOAc (10 mL × 3). The organic phases were combined, washed with brine, dried over anhydrous sodium sulfate, filtered, and evaporated to dryness. The residue was purified by column chromatography (MeOH/DCM, 2% to 3%) to afford compound 6 (210 mg, 20.71% yield) as a yellow solid. ESI-MS:[M+H]+, 239.3.

### (4) Synthesis steps of compound 8

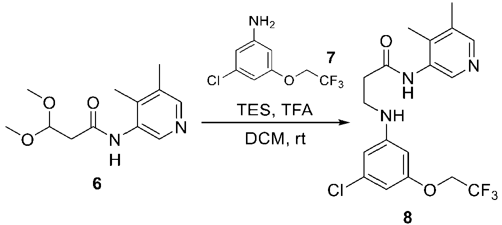

DCM (5 mL) was added to compound 6 (210 mg, 0.88 mmol), and then compound 7 (240 mg, 1.06 mmol), TFA (1 mL) and Et₃SiH (290 mg, 1.76 mmol) were further slowly added. The mixture was allowed to react at room temperature under nitrogen atmosphere for 16 hours. After the reaction ended, aqueous sodium carbonate solution was added to adjust the pH of the mixture to 8-9. The mixture was extracted with EtOAc (20 mL × 3). The organic phases were combined, washed with saturated brine, dried over anhydrous sodium sulfate, filtered, and evaporated to dryness. The residue was purified by column chromatography (EtOAc/PE, 40% to 42%) to afford compound 8 (130 mg, 36.62% yield) as a white solid. ESI-MS:[M+H]+, 402.15.

### (5) Synthesis steps of compound GDI15-8050

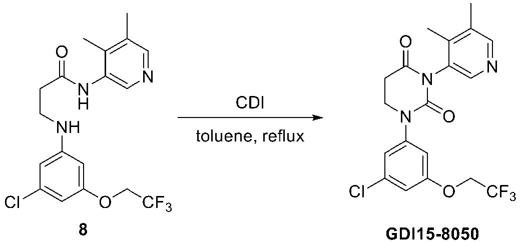

Toluene (5 mL) was added to compound 8 (130 mg, 0.32 mmol), then (262 mg, 1.61 mmol) was added, and the mixture was further allowed to react at 110°C for 2 hours. The reaction ended as monitored by LCMS. Then the mixture was concentrated. The residue was further purified by prep-HPLC (column: Gemini 5 µm C18 column, 150*21.2 mm, eluting with 75% to 95% MeCN/H₂O containing 0.1% FA) to afford compound GDI15-8050 (4.88 mg, 3.53% yield) as a white solid. ESI-MS:[M+H]+, 428.0.

**¹H NMR** *J=* 1.7 Hz, 1 H), 7.13 (m, 2 H), 4.83 (t, *J=* 8.9 Hz, 2 H), 4.02 (m, 2 H), 3.06 (m, 2 H), 2.27 (s, 3 H), 2.04 (s, 3 H).

### Example 48: Preparation of Compound GDI15-8208

### (1) Synthesis steps of compound 2

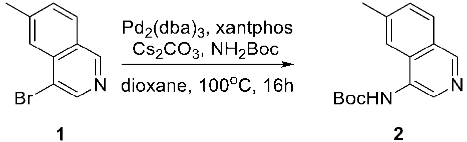

1,4-dioxane (15 mL) was added to compound 1 (1g, 4 mmol). Then, tert-butyl carbamate (790 mg, 6.7 mmol), Cs₂CO₃ (4.4g, 13.4 mmol), Xantphos (1.04g, 1.8 mmol), and Pd₂(dba)₃ (820 mg, 0.9 mmol) were further added under nitrogen atmosphere. The mixture was stirred and allowed to react at 100°C for 16 hours. After the reaction ended, water (5 mL) was added to the mixture, and the mixture was extracted with EtOAc (10 mL × 3). The organic phases were combined, washed with brine, dried over anhydrous sodium sulfate, filtered, and evaporated to dryness. The residue was purified by column chromatography (MeOH/DCM, 2% to 3%) to afford compound 2 (1.1 g, 95.56% yield) as a yellow solid. ESI-MS:[M+H]+, 259.20.

### (2) Synthesis steps of compound 3

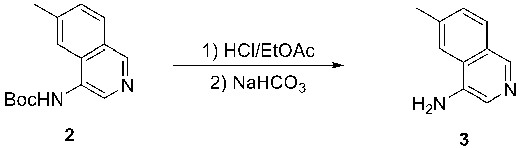

HCl/MeOH (10 mL) was added dropwise to compound 1 (1.1g, 4.3 mmol) at 0°C. The mixture was then warmed slowly to room temperature, stirred and allowed to react at room temperature for 2 hours, and then a small amount of mixed solution of MeOH/DCM and NaHCO₃ (1.08g, 12.9 mmol) was added. After the reaction ended, the mixture was filtered and concentrated in vacuo to afford the crude product, compound 3 (600 mg, 88.37% yield), to be used directly in the next step without further purification. ESI-MS: [M+H]+, 159.28.

### (3) Synthesis steps of compound 5

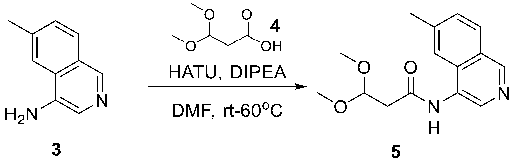

DCM (10 mL) was added to compound 3 (560 mg, 3.54 mmol), compound 4 (570 mg, 4.25 mmol), pyridine (1.4g, 17.70 mmol) and POCl₃ (1.09g, 7.08 mmol). The mixture was stirred and allowed to react at room temperature for 3 hours under nitrogen atmosphere. The reaction ended as monitored by LCMS. After the reaction ended, water (50 mL) was added to the mixture, and the mixture was extracted with EtOAc (50 mL × 3). The organic phases were combined and washed with saturated brine, dried over anhydrous sodium sulfate, filtered, and evaporated to dryness. The residue was purified by column chromatography (DCM/MeOH, 20v: 1v) to afford compound 5 (220 mg, 0.80 mmol, 22.66% yield) as a yellow solid. ESI-MS: [M+H] +, 275.0.

### (4) Synthesis steps of compound 7

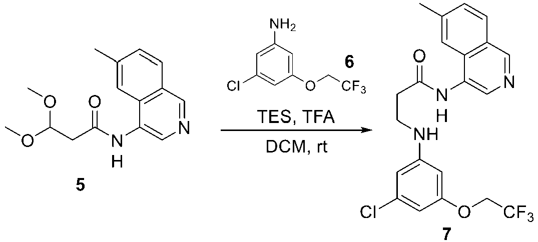

DCM (4 mL) was added to compound 5 (220 mg, 0.80 mmol), and then compound 6 (200 mg, 0.88 mmol), TFA (1 mL), and Et₃SiH (395 mg, 2.4 mmol) were further slowly added under nitrogen atmosphere. The mixture was stirred at room temperature for reaction for 16 hours. After the reaction ended, aqueous sodium carbonate solution was added to the mixture to adjust the pH to 8-9, and the mixture was extracted with EtOAc (20 mL × 3). The organic phases were combined, washed with brine, dried over anhydrous sodium sulfate, filtered, and evaporated to dryness. The residue was purified by column chromatography (EtOAc/PE, 40% to 42%) to afford compound 7 (150 mg, 36.62% yield) as a white solid. ESI-MS: [M+H] +, 438.0.

### (4) Synthesis steps of compound GDI15-8208

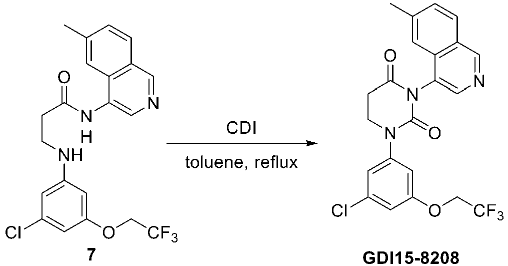

Toluene (5 mL) was added to compound 7 (150 mg, 0.34 mmol), and then CDI (554 mg, 3.42 mmol) was added to the mixture. The mixture was stirred and allowed to react at 110°C for 12 hours, and the reaction ended as monitored by LCMS. The mixture was concentrated, and the residue was further purified by prep-HPLC (Gemini 5 µm C18 column, 150×21.2 mm, eluting with 75% to 95% MeCN/H₂O containing 0.1% FA) to afford compound GDI15-8208 (35.84 mg, 22.56% yield) as a white solid. ESI-MS: [M+H] +, 464.05.

1H NMR (400 MHz, DMSO) δ 9.27 (s, 1 H), 8.39 (s, 1 H), 8.12 (d, *J* = 8.4 Hz, 1 H), 7.66 (s, 1 H), 7.57 (d, *J* = 8.5 Hz, 1 H), 7.26 (t, *J* = 1.8 Hz, 1 H), 7.15 (m, 2 H), 4.86 (m, 2 H), 4.14 (m, 2 H), 3.25 (m, 1 H), 3.07 (m, 1 H), 2.55 (s, 3 H).

### Example 49: Preparation of Compound GDI15-8242

### (1) Synthesis steps of compound 3

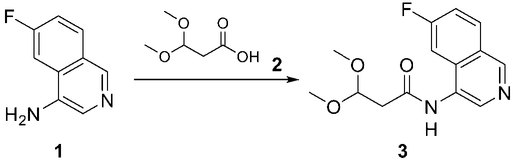

DCM (5 mL) was added to compound 1 (250 mg, 1.54 mmol). Then, compound 2 (227 mg, 1.70 mmol), DIPEA (598 mg, 4.63 mmol), and POCl₃ (473 mg, 3.08 mmol) were further added under nitrogen atmosphere. The mixture was stirred and allowed to react at room temperature for 2 hours. After the reaction ended, the mixture was concentrated. The residue was further purified by prep-HPLC (Gemini 5 µm C18 column, 150*21.2 mm, eluting with 30% to 90% MeCN/H₂O containing 0.1% FA) to afford compound 3 (109 mg, 25.41% yield) as a yellow solid. ESI-MS: [M+H] +, 279.10.

### (2) Synthesis steps of compound 5

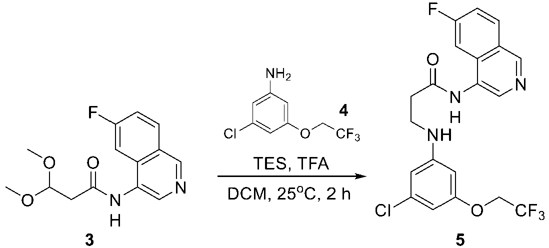

DCM (5 mL) was added to compound 3 (109 mg, 0.39 mmol), and then compound 4 (98 mg, 0.43 mmol), TFA (1 mL) and Et₃SiH (193 mg, 1.18 mmol) were further slowly added at room temperature under nitrogen atmosphere. The mixture was stirred and allowed to react at room temperature for 16 hours. After the reaction ended, aqueous Na₂CO₃ solution was added to the mixture to adjust the pH of the mixture to 8-9. The mixture was extracted with EtOAc (20 mL ×3). The organic phases were combined and washed with saturated brine, dried over anhydrous sodium sulfate, filtered, and evaporated to dryness. The residue was further purified by column chromatography (EtOAc/PE, 40% to 42%) to afford compound 5 (45 mg, 25.94% yield) as a yellow oil. ESI-MS: [M+H] +, 441.95.

### (3) Synthesis steps of compound GDI15-8242

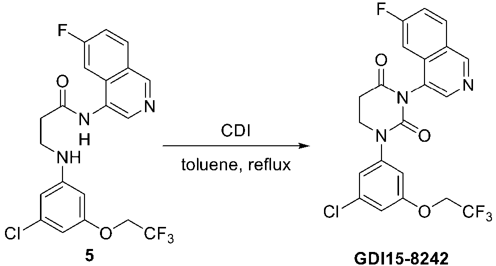

Toluene (5 mL) was added to compound **5** (20 mg, 0.045 mmol), then CDI (44 mg, 0.27 mmol) was further added, and the mixture was stirred and allowed to react at 110°C for 2 hours. The reaction ended as monitored by LCMS. After the reaction ended, the mixture was concentrated. The residue was further purified by prep-HPLC (Gemini 5 µm C18 column, 150*21.2 mm, eluting with 75% to 95% MeCN/H₂O containing 0.1% FA) to afford compound GDI15-8242 (3.00 mg, 14.16% yield) as a white solid. ESI-MS: [M+H] +, 468.00.

1H NMR (400 MHz, DMSO) δ 9.37 (s, 1 H), 8.48 (s, 1 H), 8.36 (m, 1 H), 7.82 (d, *J* = 10.0 Hz, 1 H), 7.66 (m, 1 H), 7.26 (s, 1 H), 7.14 (d, *J* = 6.2 Hz, 2 H), 4.84 (t, *J* = 8.8 Hz, 2 H), 4.13 (m, 2 H), 3.26 (d, *J* = 8.3 Hz, 1 H), 3.03 (d, *J* = 16.6 Hz, 1 H).

### Example 50: Preparation of Compound GDI15-8241

### (1) Synthesis steps of compound 2

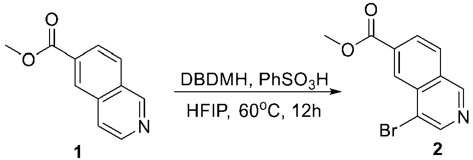

Compound 1 (6g, 32.1 mmol) and PhSO₃H (50 mg, 0.3 mmol) were dissolved in HFIP (60 mL). DBDMH (5.5g, 19.2 mmol) was then added, and the mixture was stirred and allowed to react at 60°C for 12 hours. Then water was added, and the mixture was extracted with EtOAc (50 mL × 3). The organic phases were combined and washed with saturated brine, dried over anhydrous sodium sulfate, filtered, and evaporated to dryness. The residue was purified by column chromatography (PE/EtOAc, 0% to 50%) to afford compound 2 (1.8g, 6.8 mmol) as a yellow solid. ESI-MS: [M+H] ⁺, 265.85.

### (2) Synthesis steps of compound 3

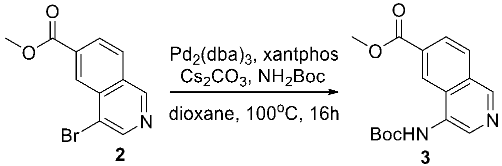

1,4-dioxane (20 mL) was added to compound 2 (1.8g, 6.8 mmol), NH₂Boc (1.1g, 8.8 mmol), Pd₂(dba)₃ (190 mg, 0.2 mmol), and Xantphos (200 mg, 0.3 mmol), and then Cs₂CO₃ (4.4g, 13.6 mmol) was further added. The mixture was stirred and allowed to react at 100°C for 16 hours. After the reaction ended, water was added to the mixture, and the mixture was extracted with EtOAc (20 mL × 3). The organic phases were combined and washed with saturated brine, dried over anhydrous sodium sulfate, filtered, and evaporated to dryness. The residue was purified by column chromatography (PE/EtOAc, 0% to 50%) to afford compound 3 (1.1g, 3.6 mmol) as a yellow solid. ESI-MS: [M+H] +, 303.15.

### (3) Synthesis steps of compound 4

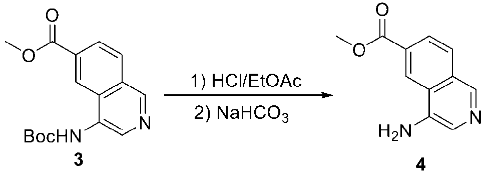

1 M HCl in EtOAc (15 mL) was added to compound 3 (1.1g, 3.6 mmol). The mixture was stirred and allowed to react at room temperature for 2 hours. After the reaction ended, the mixture was filtered, and the filter cake was dissolved in MeOH. Aqueous NaHCO₃ solution was then added to adjust the pH to 7-8, and the mixture was concentrated to afford compound 4 (700 mg, 3.4 mmol) as a yellow solid. ESI-MS: [M+H] +, 203.00.

### (4) Synthesis steps of compound 6

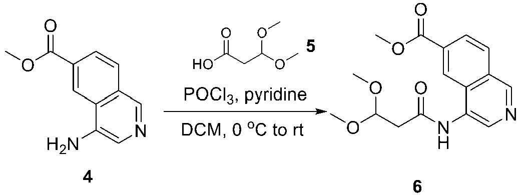

DCM (10 mL) was added to a mixture of compound 4 (700 mg, 3.4 mmol), compound 5 (652 mg, 4.1 mmol) and pyridine (1.4g, 17.0 mmol). Then, POCl₃ (1.5g, 9.7 mmol) was added slowly to the mixture at 0°C, and then the mixture was warmed slowly to room temperature, stirred and allowed to react at room temperature for 2 hours. After the reaction ended, the mixture was quenched by adding aqueous sodium carbonate solution. The organic phases were extracted, combined, washed with saturated brine, dried over anhydrous sodium sulfate, filtered, and evaporated to dryness. The residue was purified by column chromatography (DCM/MeOH, 0% to 10%) to afford compound 6 (370 mg, 1.1 mmol) as a yellow solid. ESI-MS: [M+H] ⁺, 318.95.

### (4) Synthesis steps of compound 8

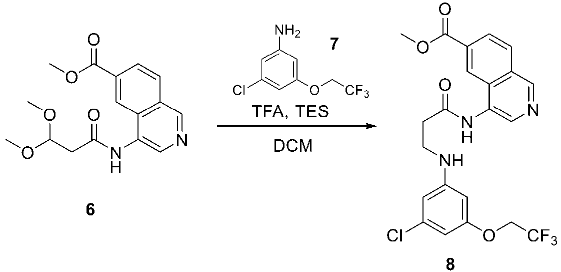

DCM (4 mL) was added to compound 6 (370 mg, 1.1 mmol), compound 7 (237 mg, 1.1 mmol) and TFA (1.1 mL). Then TES (479 mg, 2.9 mmol) was further slowly added to the mixture. The mixture was stirred and allowed to react at room temperature for 2 hours. After the reaction ended, NaHCO₃ was added to the mixture to adjust the pH to 7-8. The mixture was filtered, to afford compound 8 (200 mg, 0.4 mmol) as a white solid. ESI-MS: [M+H] ⁺, 482.00.

### (5) Synthesis steps of compound GDI15-8241

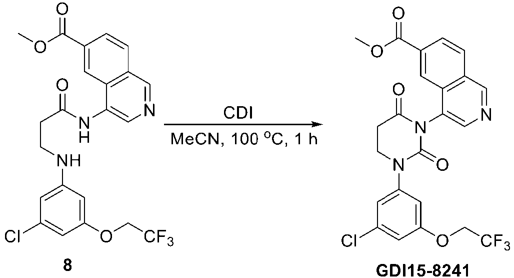

CDI (1.12g, 7.2 mmol) was added to a solution of compound 8 (70 mg, 0.1 mmol) in MeCN (2 mL). Then, the mixture was stirred and allowed to react at 100°C for 1 hour. After the reaction ended, water was added, and the mixture was extracted with EtOAc (10 mL × 3). The organic phases were combined and washed with saturated brine, dried over anhydrous sodium sulfate, filtered, and evaporated to dryness. The residue was purified by column chromatography (PE/EtOAc, 0% to 50%) to afford compound **GDI15-8241** (30 mg, 0.06 mol) as a white solid. ESI-MS: [M+H] +, 508.00.

**¹H NMR** *J* = 8.6 Hz, 2 H), 8.23 - 8.12 (m, 1 H), 7.27 (s, 1 H), 7.22 (d, *J* = 13.2 Hz, 2 H), 4.86 (q, *J* = 8.8 Hz, 2 H), 4.23 - 4.07 (m, 2 H), 3.95 (s, 3 H), 3.30 (d, *J* = 5.8 Hz, 1 H), 3.19 - 2.99 (m, 1 H).

### Example 51: Preparation of Compound GDI15-8110

### (1) Synthesis steps of compound 1-3

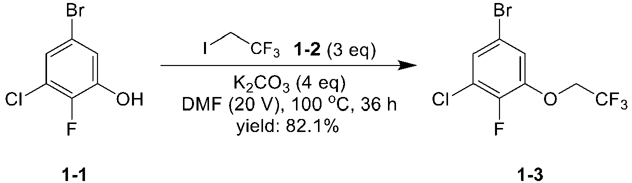

Compound 1-1 (1g, 4.44 mmol, 1 eq) and **compound 1-2** (2.79g, 13.3 mmol, 1.31 mL, 3 eq) were dissolved in DMF (20 mL). K₂CO₃ (2.45g, 17.7 mmol, 4 eq) was added at 25°C. The reaction mixture was allowed to react at 100°C for 36 hours, and the reaction ended as monitored by LCMS. The reaction solution was concentrated under reduced pressure, and then water (50 mL) was added. The reaction solution was extracted with EtOAc (20 mL ×3). The organic phases were combined and washed with 100 mL of saturated sodium chloride solution. The combined organic phases were dried over anhydrous sodium sulfate, filtered, and evaporated to dryness under reduced pressure. The residue was purified by column chromatography (SiO₂, Petroleum ether/EtOAc = 20:1 to 5:1) to afford the target compound 1-3 (1.12g, 3.64 mmol, 82.1% yield) as a colorless oil.

### (2) Synthesis steps of compound 1-4

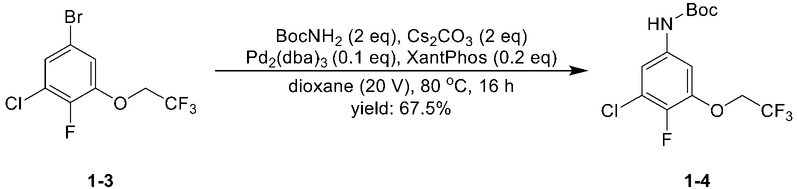

Compound **1-3** (1.1g, 3.58 mmol, 1 eq) was dissolved in 1,4-dioxane (22 mL). At 25°C, BocNH₂ (838 mg, 7.16 mmol, 2 eq), Cs₂CO₃ (2.33g, 7.16 mmol, 2 eq), Xantphos (414 mg, 715 µmol, 0.2 eq), and Pd₂(dba)₃ (327 mg, 357 µmol, 0.1 eq) were added successively. The reaction mixture was allowed to react at 80°C for 16 hours under nitrogen atmosphere, and the reaction ended as monitored by LCMS. The reaction solution was filtered, and the filtrate was concentrated under reduced pressure. The residue was purified by column chromatography (SiO₂, Petroleum ether/EtOAc = 20:1 to 5:1) to afford the target compound 1-4 (0.83g, 2.41 mmol, 67.5% yield) as a yellow solid. ESI-MS:[M+H]⁺, 288.2.

### (3) Synthesis steps of compound 1

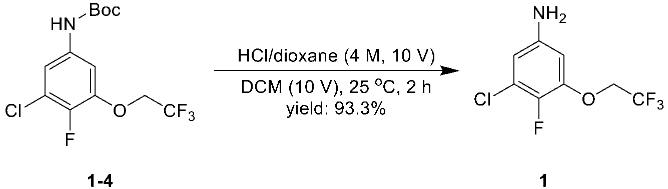

Compound **1-4** (830 mg, 2.41 mmol, 1 eq) was dissolved in DCM (8.3 mL). 4 M HCl/1,4-dioxane (8.3 mL) was added at 25°C. The reaction mixture was allowed to react at 25°C for 2 hours, and the reaction ended as monitored by LCMS. The reaction solution was concentrated under reduced pressure, and then water (10 mL) was added. The pH was adjusted to 8 with saturated aqueous sodium carbonate solution. The reaction solution was then extracted with EtOAc (5 mL ×3). The organic phases were combined and washed with saturated sodium chloride solution (10 mL). The combined organic phases were dried over anhydrous sodium sulfate, filtered, and evaporated to dryness under reduced pressure to afford compound 1 (610 mg, 2.25 mmol, 93.3% yield, 90% purity) as a yellow solid. ESI-MS:[M+H]⁺, 244.0.

### (4) Synthesis steps of compound 5

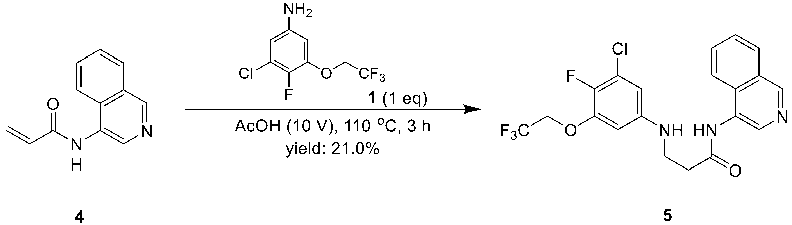

Compound 4 (250 mg, 1.26 mmol, 1 eq) and compound **1** (307 mg, 1.26 mmol, 1 eq) were dissolved in AcOH (2.5 mL). The reaction mixture was allowed to react at 110°C for 3 hours, and the reaction ended as monitored by LCMS. The reaction solution was concentrated under reduced pressure, and then water (10 mL) was added. The reaction solution was extracted with EtOAc (5 mL ×3). The organic phases were combined and washed with saturated sodium chloride solution (10 mL). The combined organic phases were dried over anhydrous sodium sulfate, filtered, and evaporated to dryness under reduced pressure. The residue was purified by thin-layer chromatography (SiO₂, Petroleum ether/EtOAc = 0:1) to afford the target compound 5 (117mg, 264 µmol, 21.0% yield) as a yellow solid. ESI-MS:[M+H]⁺, 442.2.

### (5) Synthesis steps of compound GDI15-8110

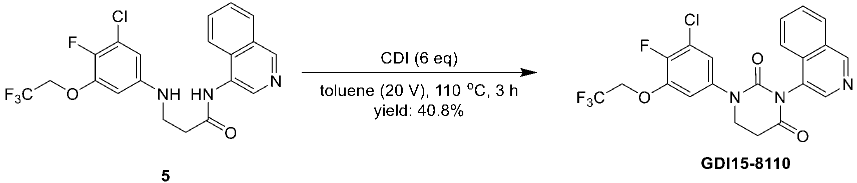

Compound 5 (117 mg, 264 µmol, 1 eq) was dissolved in toluene (2.3 mL). CDI (257 mg, 1.59 mmol, 6 eq) was added at 25°C. The reaction mixture was allowed to react at 110°C for 3 hours, and the reaction ended as monitored by LCMS. The reaction solution was concentrated under reduced pressure, and then water (10 mL) was added. The reaction solution was extracted with EtOAc (2 mL ×3). The organic phases were combined and washed with saturated sodium chloride solution (10 mL). The combined organic phases were dried over anhydrous sodium sulfate, filtered, and evaporated to dryness under reduced pressure. The residue was purified by high performance liquid chromatography (column: 2_Phenomenex Gemini C18 75*40 mm*3 µm; mobile phase: [H₂O (10 mM NH₄HCO₃)-ACN]; gradient: 30%-60%, 8.0 min) to afford the target compound GDI15-8110 (50.6 mg, 108 µmol, 40.8% yield) as a yellow solid. ESI-MS:[M+H]⁺, 468.1.

¹H NMR (400 MHz, DMSO) δ 9.37 (s, 1 H), 8.45 (s, 1 H), 8.24 (d, *J* = 8.0 Hz, 1 H), 7.96 - 7.91 (m, 1 H), 7.84 (t, *J* = 7.2 Hz, 1 H), 7.77 - 7.71 (m, 1 H), 7.46 - 7.40 (m, 2 H), 4.93 (q, *J* = 8.7 Hz, 2 H), 4.16 - 4.06 (m, 2 H), 3.30 - 3.22 (m, 1 H), 3.13 - 3.05 (m, 1 H).

### Example 52: Preparation of compound GDI15-8320

### (1) Synthesis steps of compound 1-3

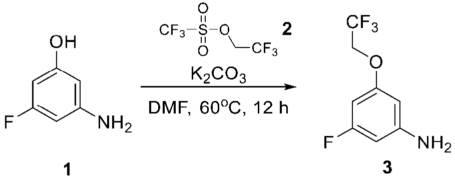

DMF (5 mL) was added to compound 1 (500 mg, 3.93 mmol). Compound 2 (1105 mg, 4.72 mmol) and K₂CO₃ (1.63g, 11.8 mmol) were further added to the mixture at room temperature under nitrogen atmosphere. The mixture was stirred and allowed to react at 60°C for 16 hours. After the reaction ended, water (50 mL) was added to the mixture, and the mixture was extracted with EtOAc (100 mL × 3). The organic phases were combined, washed with saturated brine, dried over anhydrous sodium sulfate, filtered, and evaporated to dryness. The residue was purified by column chromatography (EtOAc/PE, 20% to 30%) to afford compound 3 (310 mg, 37.50% yield) as a brown solid. ESI-MS:[M+H]⁺, 209.95.

### (2) Synthesis steps of compound 5

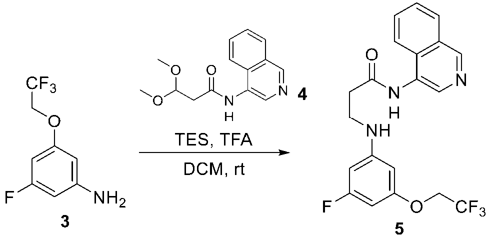

DCM (5 mL) was added to compound 3 (245 mg, 0.94 mmol), and then compound 4 (180 mg, 0.86 mmol), TFA (1 mL) and Et₃SiH (422 mg, 2.57 mmol) were further added at room temperature under nitrogen atmosphere. The mixture was further allowed to react at room temperature for 16 hours. After the reaction ended, aqueous Na₂CO₃ solution was added to the mixture to adjust the pH to 8-9. The mixture was extracted with EtOAc (20 mL ×3). The organic phases were combined and washed with saturated brine, dried over anhydrous sodium sulfate, filtered, and evaporated to dryness. The residue was further purified by column chromatography (EtOAc/PE, 40% to 42%) to afford White the surface of a solid 5 (125 mg, 35.74% yield) as a white solid. ESI-MS: [M+H]⁺, 408.10.

### (3) Synthesis steps of compound GDI15-8320

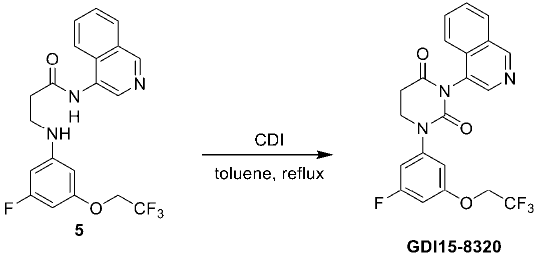

Toluene (5 mL) was added to compound 5 (125 mg, 0.31 mmol), and then (298 mg, 1.84 mmol) was further added to the mixture. The mixture was allowed to react at 110°C for 2 hours, and the reaction ended as monitored by LCMS. The mixture was concentrated, and the residue was further purified by prep-HPLC (Gemini 5 µm C18 column, 150×21.2 mm, eluting with 75% to 95% MeCN/H₂O containing 0.1% FA) to afford compound GDI15-8320 (80.92 mg, 60.86% yield) as a white solid. ESI-MS:[M+H]⁺, 434.05.

**¹H NMR** *J* = 8.1 Hz, 1 H), 7.94 (d, *J* = 8.4 Hz, 1 H), 7.84 (t, *J* = 7.6 Hz, 1 H), 7.75 (t, *J* = 7.5 Hz, 1 H), 7.03 (d, *J* = 6.0 Hz, 2 H), 6.96 (d, *J* = 10.6 Hz, 1 H), 4.83 (m, 2 H), 4.14 (m, 2 H), 3.25 (m, 1 H), 3.08 (m, 1 H).

### Example 53: Preparation of compound GDI15-8319

### (1) Synthesis steps of compound 3

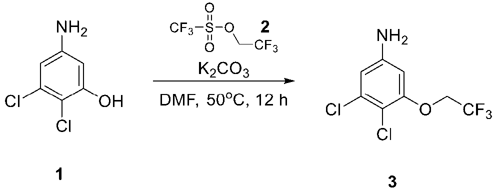

Compound 2 (631 mg, 2.70 mmol) and K₂CO₃ (932 mg, 6.74 mmol) were added to a solution of compound 1 (400 mg, 2.25 mmol) in DMF (5 mL). The mixture was allowed to react at 60°C for 16 hours under nitrogen atmosphere. After the reaction ended, water (50 mL) was added to the mixture, and the mixture was extracted with EtOAc (100 mL × 3). The organic phases were combined, washed with saturated brine, dried over anhydrous sodium sulfate, filtered, and evaporated to dryness. The residue was purified by column chromatography (EtOAc/PE, 20% to 30%) to afford compound 3 (190 mg, 32.39% yield) as a brown solid. ESI-MS: [M+H] ⁺, 260.0.

### (2) Synthesis steps of compound 5

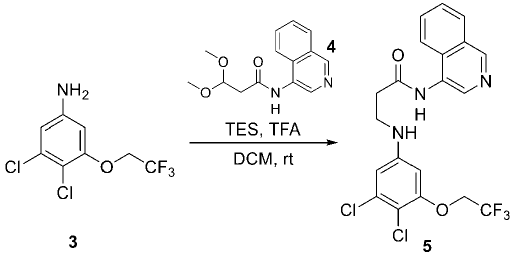

Compound 4 (190 mg, 0.73 mmol), TFA (1 mL) and Et₃SiH (359 mg, 2.18 mmol) were added into a solution of compound 3 (208 mg, 0.80 mmol) in DCM (5 mL). The mixture was allowed to react at room temperature under nitrogen atmosphere for 16 hours. After the reaction ended, the pH of the mixture was adjusted to 8-9 by adding the saturated aqueous Na₂CO₃ solution, and the mixture was extracted with EtOAc (20 mL × 3). The organic phases were combined and washed with brine, dried over anhydrous sodium sulfate, filtered, and evaporated to dryness. The residue was purified by column chromatography. The residue was further purified by column chromatography (EtOAc/PE, 40% to 42%) to afford compound 5 (120 mg, 35.90% yield) as a yellow solid. ESI-MS: [M+H] ⁺, 458.0.

### (3) Synthesis steps of compound GDI15-8319

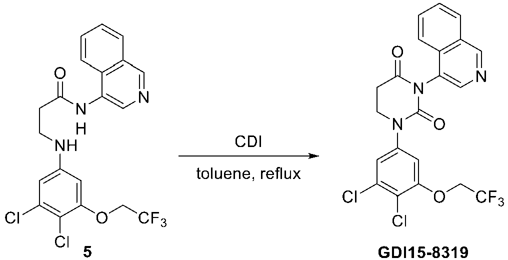

CDI (254 mg, 1.57 mmol) was added to a solution of compound 5 (120 mg, 0.26 mmol) in toluene (5 mL), and the mixture was stirred and allowed to react at 110°C for 2 hours. The reaction ended as monitored by LCMS. After the reaction ended, the mixture was concentrated. The residue was further purified by prep-HPLC (column: Gemini 5 µm C18 column, 150*21.2 mm, eluent: 75% to 95% MeCN/H₂O containing 0.1% FA) to afford compound GDI15-8319 (75.22 mg, 59.32% yield) as a yellow solid. ESI-MS: [M+H] ⁺, 484.0.

**¹H NMR** *J* = 8.3 Hz, 1 H), 7.96 (d, *J* = 8.3 Hz, 1 H), 7.86 (t, *J* = 7.7 Hz, 1 H), 7.76 (t, *J* = 7.4 Hz, 1 H), 7.50 (s, 1 H), 7.41 (s, 1 H), 4.93 (m, 2 H), 4.16 (d, *J* = 5.4 Hz, 2 H), 3.26 (s, 1 H), 3.12 (s, 1 H).

### Example 54: Preparation of compound GDI15-8688

### (1) Synthesis steps of compound 3

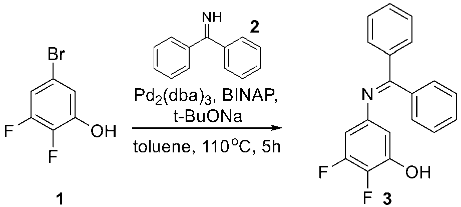

Toluene (15 mL) was added to a mixture of compound 1 (1.5g, 7.2 mmol), compound 2 (1.96g, 10.8 mmol), t-BuONa (2.08g, 21.6 mmol), BINAP (1.79g, 2.8 mmol) and Pd₂(dba)₃ (1.32g, 1.4 mmol). The mixture was stirred and allowed to react at 110°C for 16 hours under nitrogen atmosphere. The reaction ended as monitored by LCMS. After the reaction ended, H₂O (50 mL) was added to the mixture, and the mixture was extracted with EtOAc (50 mL × 3). The organic phases were combined and washed with saturated brine, dried over anhydrous sodium sulfate, filtered, and evaporated to dryness. The residue was purified by column chromatography (MeOH/DCM, 3% to 5%) to afford compound 3 (1.3g, 4.2 mmol, 58.33% yield) as a yellow solid. ESI-MS: [M+H] ⁺, 310.0.

### (2) Synthesis steps of compound 4

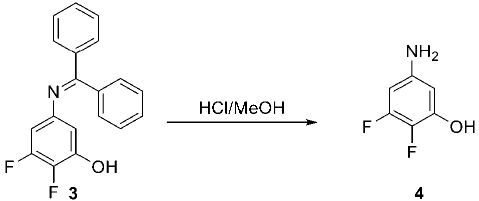

A mixed solution of HCl/MeOH (15 mL) was added to compound 3 (1.3g, 4.2 mmol), and the mixture was stirred and allowed to react at room temperature under nitrogen protection for 2 hours. After the reaction ended, the mixture was concentrated to afford the crude product, compound 4 (0.5g, 80.95% yield) as a yellow solid. ESI-MS: [M+H] ⁺, 146.2.

### (3) Synthesis steps of compound 6

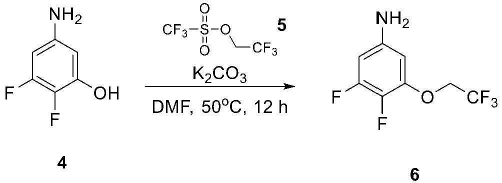

K₂CO₃ (1.46g, 10.54 mmol) was added into a solution of compound 4 (510 mg, 3.51 mmol) and compound 5 (1.23g, 5.27 mmol) in DMF (10 mL). The mixture was stirred and allowed to react at 60°C for 12 hours. After the reaction ended, water was added to the mixture, and the mixture was extracted with EtOAc (100 mL × 3). The organic phases were combined and washed with saturated brine, dried over anhydrous sodium sulfate, filtered, and evaporated to dryness. The residue was purified by column chromatography (PE/EtOAc, 0% to 10%) to afford compound 6 (260 mg, 1.14 mol, 32.43% yield) as a yellow oil. ESI-MS: [M+H] ⁺, 227.95.

### (3) Synthesis steps of compound 8

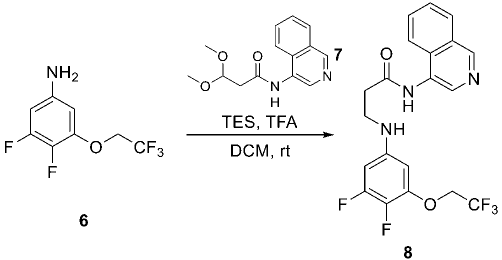

TFA (1 mL) was added to a solution of compound 6 (260 mg, 1.14 mmol) and compound 7 (445 mg, 1.71 mmol) in DCM (5 mL). The mixture was stirred for 30 minutes at room temperature. Then TES (562 mg, 3.42 mmol) was added to the mixture. The mixture was further stirred and allowed to react at room temperature for 12 hours. After the reaction ended, saturated sodium bicarbonate was added to adjust pH of the mixture to 7-8. The solid was filtered and washed with water to afford compound 8 (140 mg, 0.33 mmol, 28.81% yield) as a white solid. ESI-MS: [M+H] ⁺, 426.05.

### (4) Synthesis steps of compound GDI15-8688

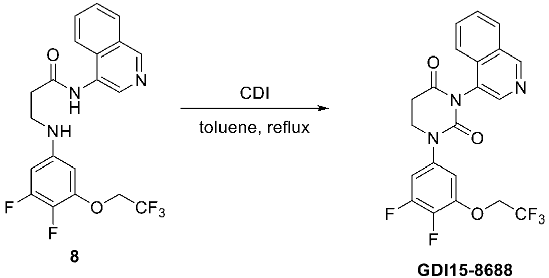

CDI (319 mg, 1.97 mmol) was added to a solution of compound **8** (140 mg, 0.33 mmol) in toluene (5 mL), and the mixture was stirred and allowed to react at 100°C for 6 hours. After the reaction ended, water was added to the mixture, and the mixture was extracted with EtOAc (80 mL × 3). The organic phases were combined and washed with saturated brine, dried over anhydrous sodium sulfate, filtered, and evaporated to dryness. The residue was purified by column chromatography (DCM/MeOH, 0% to 5%) to afford compound GDI15-8688 (19.32 mg, 0.04 mmol, 13% yield) as a white solid. ESI-MS: [M+H] ⁺, 452.0.

**¹H NMR** *J* = 8.2 Hz, 1 H), 7.93 (d, *J* = 8.3 Hz, 1 H), 7.84 (t, *J* = 7.3 Hz, 1 H), 7.75 (t, *J* = 7.4 Hz, 1 H), 7.31 (m, 2 H), 4.94 (m, 2 H), 4.11 (m, 2 H), 3.26 (m, 1 H), 3.10 (m, 1 H).

### Example 55: Preparation of compound GDI15-8532

### (1) Synthesis steps of compound 3

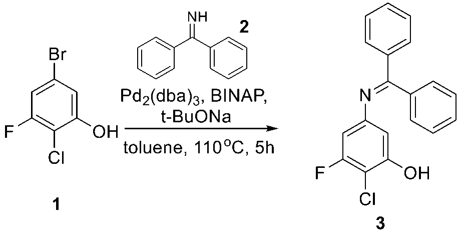

Toluene (30 mL) was added to a mixture of compound 1 (2g, 8.9 mmol), compound 2 (2.42g, 13.3 mmol), t-BuONa (2.57g, 26.7 mmol), BINAP (1.11g, 1.7 mmol) and Pd₂(dba)₃ (0.81g, 0.8 mmol). The mixture was stirred and allowed to react at 110°C for 16 hours under nitrogen atmosphere. The reaction ended as monitored by LCMS. After the reaction ended, water (50 mL) was added to the mixture, and the mixture was extracted with EtOAc (50 mL × 3). The organic phases were combined and washed with saturated brine, dried over anhydrous sodium sulfate, filtered, and evaporated to dryness. The residue was purified by column chromatography (MeOH/DCM, 3% to 5%) to afford compound 3 (2.8g, 8.6 mmol, 96.63% yield) as a yellow solid. ESI-MS: [M+H] ⁺, 326.0.

### (2) Synthesis steps of compound 4

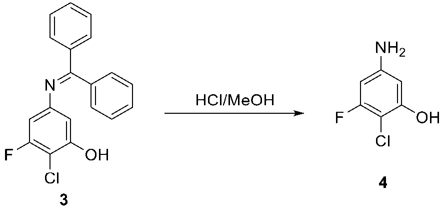

A solution of HCl/MeOH (30 mL) was added to compound 3 (2.8g, 8.6 mmol), and the mixture was allowed to react at room temperature under nitrogen protection for 2 hours. The mixture was concentrated to afford the crude product, compound 4 (1.2g, 86.05% yield) as a yellow solid. ESI-MS: [M+H] ⁺, 226.10.

### (3) Synthesis steps of compound 6

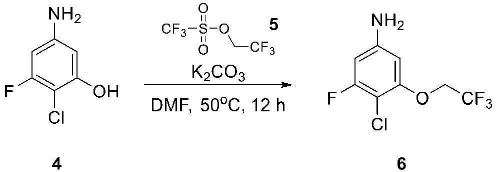

K₂CO₃ (3.07g, 22.2 mmol) was added to a solution of compound 4 (2.6g, 11.1 mmol) in DMF (12 mL). The mixture was stirred and allowed to react at 50°C for 12 hours. After the reaction ended, water was added, and the mixture was extracted with EtOAc (50 mL × 3). The organic phases were combined and washed with saturated brine, dried over anhydrous sodium sulfate, filtered, and evaporated to dryness. The residue was purified by column chromatography (PE/EtOAc, 0% to 10%) to afford compound 6 (680 mg, 2.8 mmol, 37.84% yield) as a yellow solid. ESI-MS: [M+H] ⁺, 243.95.

### (4) Synthesis steps of compound 10

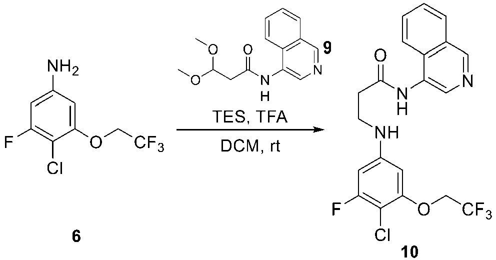

TFA (3 mL) was added to a mixture of compound 6 (350 mg, 1.43 mmol) and compound 9 (372 mg, 1.43 mmol) in DCM (5 mL). The mixture was stirred and allowed to react for 30 minutes at room temperature under nitrogen protection. Then TES (528 mg, 4.29 mmol) was further added to the mixture. The mixture was allowed to react at room temperature for 12 hours. After the reaction ended, saturated aqueous sodium bicarbonate solution was added to the mixture to adjust the pH to 7-8. The solid was filtered out and washed with water to afford compound 10 (180 mg, 0.41 mol, 28.41% yield) as a white solid. ESI-MS: [M+H] ⁺, 442.1.

### (5) Synthesis steps of compound GDI15-8532

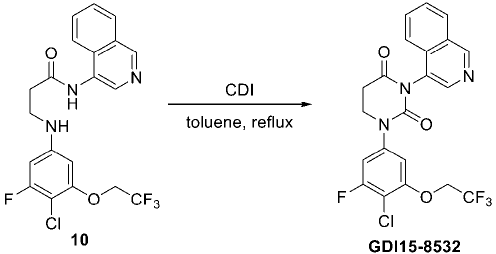

A solution (5 mL) was added to a mixture of compound 10 (180 mg, 0.41 mmol) and CDI (527 mg, 3.25 mmol). The mixture was stirred and allowed to react at 110°C for 12 hours under nitrogen atmosphere. The reaction ended as monitored by LCMS. Then, the mixture was concentrated. The residue was further purified by prep-HPLC (column: Gemini 5 µm C18 column, 150*21.2 mm, eluent: 10% to 20% ACN/H₂O) to afford compound GDI15-8532 (61.57 mg, 0.13 mmol, 32.30% yield) as a yellow solid. ESI-MS: [M+H] ⁺, 468.00.

**¹H NMR** *J* = 8.2 Hz, 1 H), 7.93 (d, *J* = 8.4 Hz, 1 H), 7.83 (t, *J* = 7.2 Hz, 1 H), 7.75 (t, *J* = 7.2 Hz, 1 H), 7.30 (m, 2 H), 4.95 (m, 2 H), 4.16 (m, 2 H), 3.26 (m, 1 H), 3.10 (m, 1 H).

### Example 56: Preparation of compound GDI15-8533

### (1) Synthesis steps of compound 3

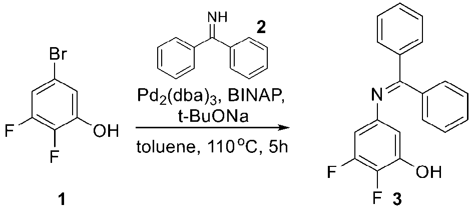

Toluene (15 mL) was added to compound 1 (1.5g, 7.2 mmol), compound 2 (1.96g, 10.8 mmol), t-BuONa (2.08g, 21.6 mmol), BINAP (1.79g, 2.8 mmol) and Pd₂(dba)₃ (1.32g, 1.4 mmol). The mixture was stirred and allowed to react at 110°C for 16 hours under nitrogen atmosphere. The reaction ended as monitored by LCMS. After the reaction ended, water (50 mL) was added to the mixture, and the mixture was extracted with EtOAc (50 mL × 3). The organic phases were combined and washed with saturated brine, dried over anhydrous sodium sulfate, filtered, and evaporated to dryness. The residue was purified by column chromatography (MeOH/DCM, 3% to 5%) to afford compound 3 (1.3g, 4.2 mmol, 58.33% yield) as a yellow solid. ESI-MS: [M+H] ⁺, 310.00.

### (2) Synthesis steps of compound 4

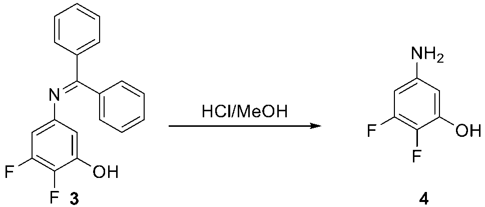

A mixed solution of HCl/MeOH (15 mL) was added to compound 3 (1.3g, 4.2 mmol), and the mixture was stirred and allowed to react at room temperature under nitrogen protection for 2 hours. The mixture was concentrated to afford the crude product, compound 4 (0.5g, 80.95% yield) as a yellow solid. ESI-MS: [M+H] ⁺, 146.20.

### (3) Synthesis steps of compound 6

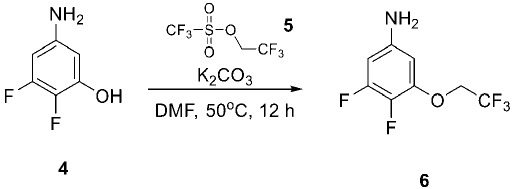

K₂CO₃ (1.46g, 10.54 mmol) was added into a solution of compound 4 (510 mg, 3.51 mmol) and compound 5 (1.23g, 5.27 mmol) in DMF (10 mL). The mixture was stirred and allowed to react at 60°C for 12 hours. After the reaction ended, water was added to the mixture, and the mixture was extracted with EtOAc (100 mL × 3). The organic phases were combined and washed with saturated brine, dried over anhydrous sodium sulfate, filtered, and evaporated to dryness. The residue was purified by column chromatography (PE/EtOAc, 0% to 10%) to afford compound 6 (260 mg, 1.14 mol, 32.43% yield) as a yellow oil. ESI-MS: [M+H] ⁺, 227.95.

### (4) Synthesis steps of compound 8

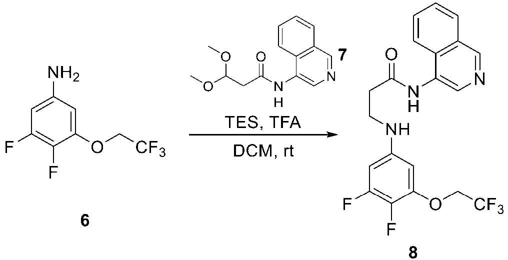

TFA (1 mL) was added to a solution of compound 6 (260 mg, 1.14 mmol) and compound 7 (445 mg, 1.71 mmol) in DCM (5 mL). The mixture was stirred for 30 minutes at room temperature. Then TES (562 mg, 3.42 mmol) was added to the mixture. The mixture was further stirred and allowed to react at room temperature for 12 hours. After the reaction ended, saturated aqueous sodium bicarbonate solution was added to the mixture to adjust pH of the mixture to 7-8. The mixture was filtered, and the filter cake was washed with water to afford compound 9 (140 mg, 0.33 mmol, 28.81% yield) as a white solid. ESI-MS: [M+H] ⁺, 426.05.

### (5) Synthesis steps of compound GDI15-8533

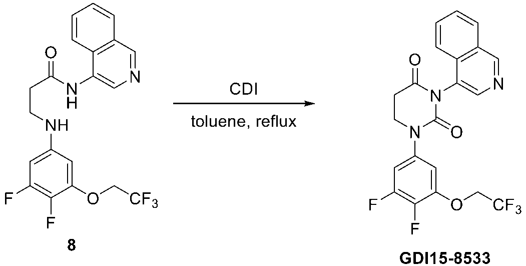

CDI (319 mg, 1.97 mmol) was added to a solution of compound **8** (140 mg, 0.33 mmol) in toluene (5 mL), and the mixture was stirred and allowed to react at 100°C for 6 hours. After the reaction ended, water was added to the mixture, and the mixture was extracted with EtOAc (80 mL × 3). The organic phases were combined and washed with saturated brine, dried over anhydrous sodium sulfate, filtered, and evaporated to dryness. The residue was purified by column chromatography (DCM/MeOH, 0% to 5%) to afford compound GDI15-8533 (19.32 mg, 0.04 mmol, 13% yield) as a white solid. ESI-MS: [M+H] ⁺, 452.00.

**¹H NMR** *J* = 8.2 Hz, 1 H), 7.93 (d, *J* = 8.3 Hz, 1 H), 7.84 (t, *J* = 7.3 Hz, 1 H), 7.75 (t, *J* = 7.4 Hz, 1 H), 7.31 (m, 2 H), 4.94 (m, 2 H), 4.11 (m, 2 H), 3.26 (m, 1 H), 3.10 (m, 1 H).

### Test Example 1: Activity measurement of a compound

### SARS-CoV-2/Hela-ACE Test

The compound was transferred to a 384-well plate (Greiner, Part. No. 781090-2B), and then Hela-ACE cells were added (cell density: 5000 cells/20 microliter of medium MEM with 2% FBS). The plate with cells was transferred to BSL3 laboratory. SARS-CoV-2 (USA-WA1/2020 Vero E6 cell proliferation) was diluted to MOI 0.75-1 until 30%-60% cells were infected. After the assay plate was incubated at 37°C in 5% CO₂ for 48 hours, formaldehyde was added until reaching a final concentration of 4% to fix the cells. A human polyclonal serum antibody was used as a primary antibody, and goat anti-human H+L conjugated Alexa 488 (Thermo Fisher Scientific A11013) was used as a secondary antibody. DAPI (Thermo Fisher Scientific D1306) was used for DNA staining.

### Toxicity Testing in Uninfected Cells

The compound was transferred to a 1536-well plate (Corning No. 9006BC), and then Hela-ACE cells were added (cell density: 600 cells/5 microliter of medium MEM with 2% FBS). After the assay plate was incubated at 37°C in 5% CO₂ for 48 hours, cell viability was tested. 2 µL of 50% Cell-Titer Glo (Promega No G7573) was diluted in water and added to a cell assay plate, and then a value on EnVision Plate Reader (Perkin Elmer) was read.

### Enzyme Activity Test

An inhibition test was conducted using 200 nM recombinant SARS-CoV-2 main protease and 15 µM fluorescent substrate (Dabcyl-TSAVL QSGFRK-Glu (EDANS); Genscript). An assay buffer consisted of 50 mM Tris-HCl and 1 mM EDTA at pH 7.3. SARS-CoV-2 3CLpro was dissolved in 25 µL of the assay buffer and mixed with compounds at different concentrations. The mixture was incubated at 37°C for 30 minutes. A substrate dissolved in 25 µL of the assay buffer was then added to initiate a reaction. Fluorescent signals at 350 nm (excitation)/490 nm (emission) were measured immediately each minute in 10 minutes at 37°C using a SpectraMax^{®} M5 multimode microplate reader. RFU at 6^{th} minute of the reaction with the compounds at different concentrations in contrast with a reaction with the lowest concentration was used to generate an IC₅₀ curve. IC₅₀ values corresponding to SARS-CoV-2 3CLpro were measured for each compound at 12 concentrations. Test data were analyzed using GraphPad Prism software.

### Activity results of the exemplary compound are shown in Table 1 and Table 2 below.

**Table 1: Activity Results of Exemplary Compound**

| **Examples** | **Structure** | **Hela ACE2-CoV-2 EC₅₀ (µM)** | **Hela ACE2-CC₅₀ uninfected (µM)** | **IC₅₀ (µM)** |
|---|---|---|---|---|
| 2 | 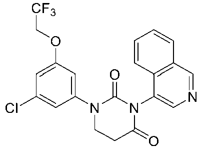 | 0.030 | > 99 | 0.042 |
| 3 | 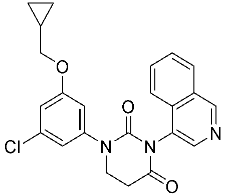 | 0.064 | 73.4 | 0.047 |
| 5 | 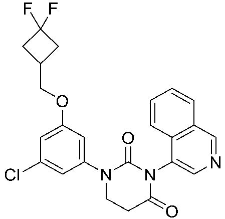 | 0.074 | > 99 | 0.043 |
| 23 | 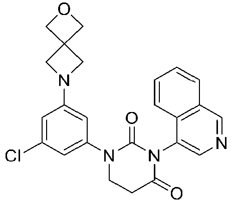 | 0.035 | > 99 | 0.032 |
| 33 | 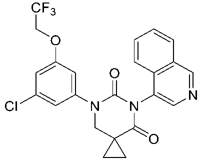 | 0.087 | > 99 | 0.05 |
| 48 | 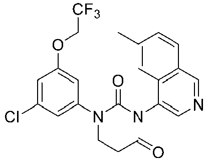 | 0.052 | > 99 | 0.050 |
| 55 | 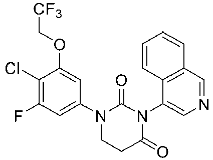 | 0.072 | 74 | 0.05 |

**Table 2: Activity Results of Exemplary Compound**

| **Examples** | **Structure** | **IC₅₀ (µM)** | **Examples** | **Structure** | **IC₅₀ (µM)** |
|---|---|---|---|---|---|
| 1 | 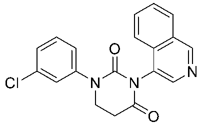 | 0.12 | 4 | 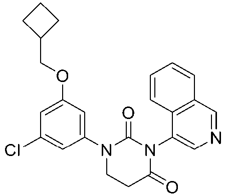 | 0.042 |
| 6 | 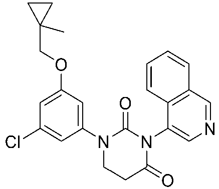 | 0.043 | 7 | 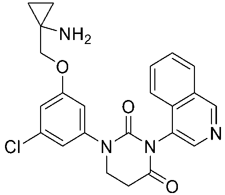 | 0.097 |
| 8 | 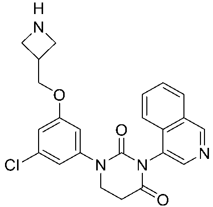 | 0.36 | 9 | 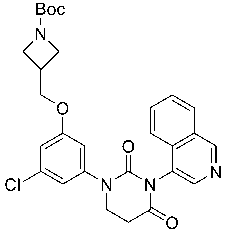 | 0.068 |
| 10 | 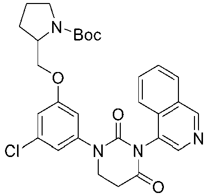 | 0.058 | 11 | 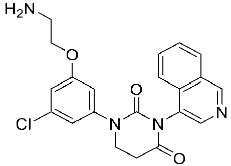 | 0.32 |
| 12 | 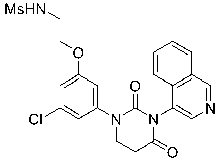 | 0.063 | 13 | 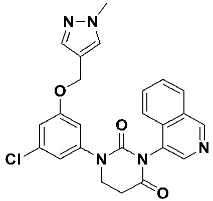 | 0.062 |
| 14 | 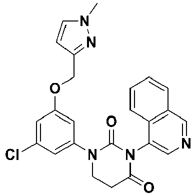 | 0.054 | 15 | 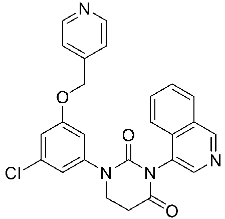 | 0.051 |
| 16 | 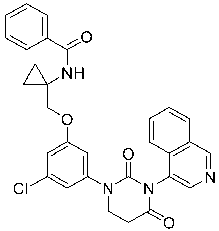 | 0.044 | 17 | 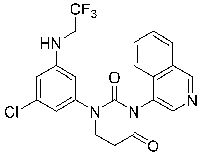 | 0.078 |
| 18 | 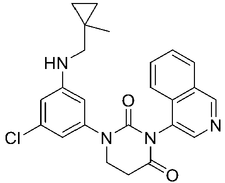 | 0.051 | 19 | 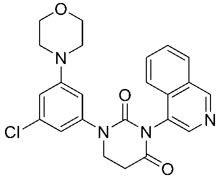 | 0.05 |
| 20 | 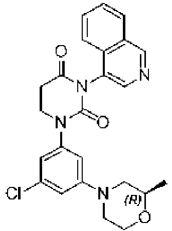 | 0.047 | 21 | 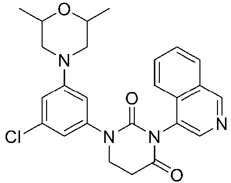 | 0.055 |
| 22 | 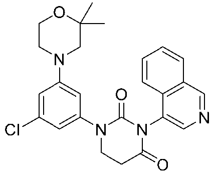 | 0.042 | 24 | 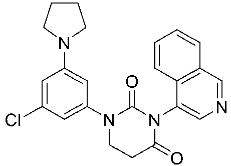 | 0.057 |
| 25 | 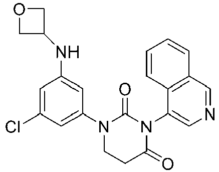 | 0.057 | 26 | 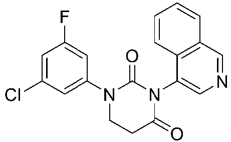 | 0.089 |
| 27 | 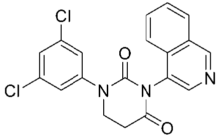 | 0.057 | 28 | 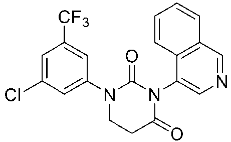 | 0.10 |
| 29 | 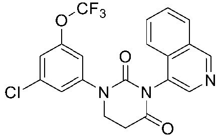 | 0.091 | 30 | 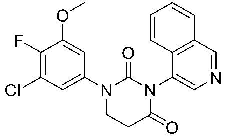 | 0.07 |
| 31 | 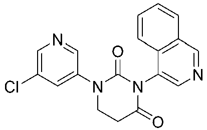 | 0.32 | 32 | 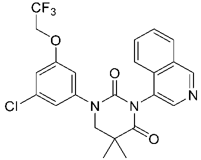 | 0.068 |
| 34 | 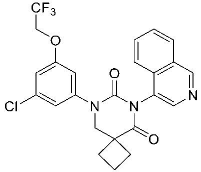 | 0.087 | 35 | 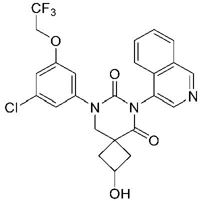 | 0.051 |
| 36 | 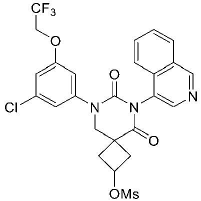 | 0.054 | 37 | 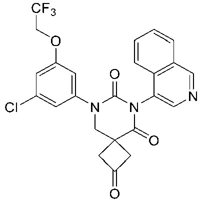 | 0.066 |
| 38 | 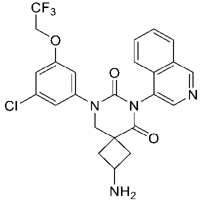 | 0.064 | 39 | 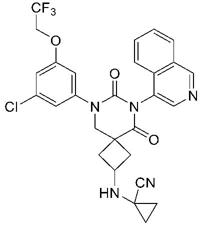 | 0.05 |
| 40 | 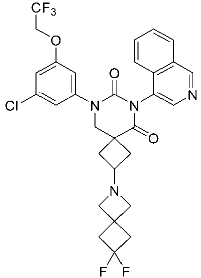 | 0.056 | 41 | 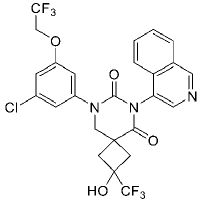 | 0.057 |
| 42 | 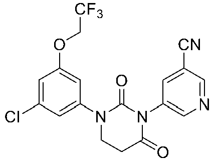 | 0.078 | 43 | 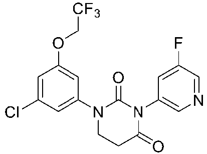 | 0.098 |
| 44 | 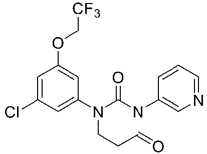 | 0.11 | 45 | 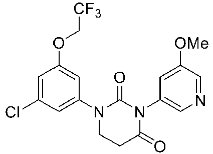 | 0.089 |
| 46 | 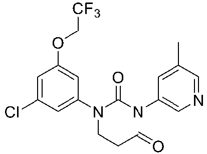 | 0.049 | 47 | 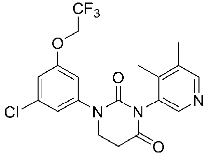 | 0.055 |
| 49 | 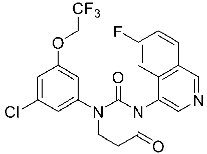 | 0.043 | 50 | 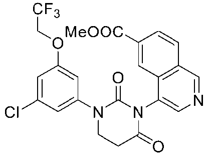 | 0.045 |
| 51 | 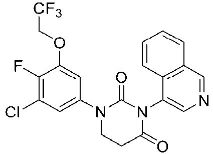 | 0.040 | 52 | 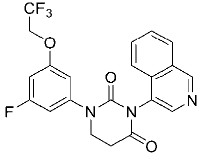 | 0.061 |
| 53 | 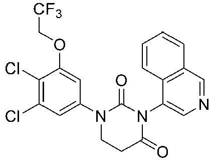 | 0.047 | 54 | 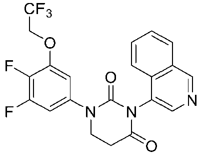 | 0.072 |
| 56 | 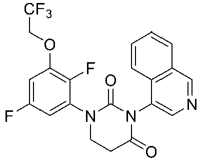 | 0.083 | | | |

## Claims

1. A compound of formula (I) or an isotope-labeled compound thereof, or an optical isomer, geometric isomer, tautomer, or isomer mixture thereof, or a pharmaceutically acceptable salt thereof, or a prodrug thereof, or a metabolite thereof; 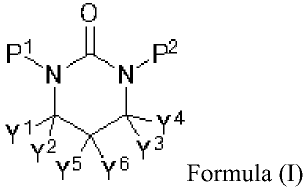 wherein:
Y¹ and Y² are each independently selected from hydrogen, halogen, a cyano group, an amino group, a C₁-C₆ alkylamino group, or a C₁-C₆ alkyl group optionally substituted with halogen or the cyano group; or Y¹ and Y² jointly form a carbonyl group; or Y¹ and Y², together with a carbon atom to which they are attached, form a C₃-C₆ cycloalkyl group or a 3- to 6-membered heterocycloalkyl group optionally substituted with halogen, a C₁-C₃ alkyl group, a C₁-C₃ haloalkyl group, a C₁-C₃ alkoxy group, a C₁-C₃ haloalkoxy group, the amino group, or a C₁-C₃ alkylamino group;
Y³ and Y⁴ are each independently selected from hydrogen, halogen, a cyano group, an amino group, a C₁-C₆ alkylamino group, or a C₁-C₆ alkyl group optionally substituted with halogen or the cyano group; or Y³ and Y⁴ jointly form a carbonyl group; or Y³ and Y⁴, together with a carbon atom to which they are attached, form a C₃-C₆ cycloalkyl group or a 3- to 6-membered heterocycloalkyl group optionally substituted with halogen, a C₁-C₃ alkyl group, a C₁-C₃ haloalkyl group, a C₁-C₃ alkoxy group, a C₁-C₃ haloalkoxy group, the amino group, or a C₁-C₃ alkylamino group;
Y⁵ and Y⁶ are each independently selected from hydrogen, halogen, a cyano group, an amino group, a C₁-C₆ alkylamino group, or a C₁-C₆ alkyl group optionally substituted with halogen or the cyano group; or Y⁵ and Y⁶, together with a carbon atom to which they are attached, form a C₃-C₆ cycloalkyl group or a 3- to 6-membered heterocycloalkyl group optionally substituted with halogen, a hydroxy group, a keto group, -OMs, a C₁-C₃ alkyl group, a C₁-C₃ haloalkyl group, a C₁-C₃ alkoxy group, a C₁-C₃ haloalkoxy group, the amino group, or a C₁-C₃ alkylamino group; and
optionally, one of Y⁵ and Y⁶, together with one of Y¹, Y², Y³, and Y⁴ and the carbon atom to which they are attached, form a C₃-C₆ cycloalkyl group or a phenyl group optionally substituted with halogen or a C₁-C₃ alkyl group;
further, wherein:
P¹ is -L¹-Z¹-;
P² is -L²-Z²-;
L¹ is selected from a bond, -CH₂-, -NH-, or -O-;
L² is selected from a bond or -CH₂-;
Z¹ is 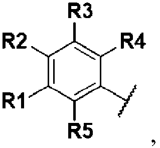 wherein the ring optionally contains 1 or 2 nitrogen heteroatoms;
Z² is selected from a 5- to 10-membered heteroaryl group, wherein one or more hydrogen atoms in Z² are each optionally substituted with halogen, a hydroxy group, a cyano group, a C₂-C₆ ester group, a C₁-C₆ alkyl group, a C₁-C₆ haloalkyl group, a C₁-C₆ alkoxy group, or a C₁-C₆haloalkoxy group independently;
R¹, R² and R⁵ are each independently selected from hydrogen, halogen, a hydroxy group, a cyano group, a nitro group, a C₁-C₆ alkyl group or a C₁-C₆ alkoxy group, and optionally, one or more hydrogen atoms in R¹, R² and R⁵ are each independently substituted with halogen, the hydroxy group, the amino group, the C₁-C₆ alkyl group or the C₃-C₆ cycloalkyl group;
one of R³ and R⁴ is H, and the other is selected from hydrogen, halogen, the hydroxy group, the cyano group, the nitro group, the amino group, the amido group, the carboxy group, the C₁-C₆ alkyl group, the C₃-C₆ cycloalkyl group, -O(CH₂)ₙ₁CR⁶R⁷R⁸, -NH(CH₂)ₙ₁R^{10,} -O(CH₂)ₙ₁R¹¹, -(CH₂)ₙ₁NH(CH₂)ₙ₂R¹², -(CH₂)ₙ₁NHCOR¹³, -(CH₂)ₙ₁NHSO₂R¹⁴, or -(CH₂)ₙ₁R¹⁵, and optionally, one or more hydrogen atoms in R³ and R⁴ are each independently substituted with halogen, the hydroxy group, the amino group, the C₁-C₆ alkyl group, the C₃-C₆ cycloalkyl group, the phenyl group or a hydroxy protecting group;
R⁶ and R⁷ are selected from hydrogen, or, together with the carbon atom to which they are attached, form a C₃-C₆ cycloalkyl group or a C₃-C₆ heterocycloalkyl group;
R⁸ is selected from -NH₂, -NHCOR⁹ or -NHSO₂R⁹, wherein R⁹ is selected from the C₁-C₆ alkyl group or the phenyl group;
R¹⁰, R¹¹, R¹², R¹³ and R¹⁴ are each independently selected from hydrogen, a cyano group, a C₁-C₆ alkyl group, a C₃-C₆ cycloalkyl group, a 3- to 6-membered heterocycloalkyl group, the phenyl group, or the 5- to 6-membered heteroaryl group;
R¹⁵ is selected from a 3- to 7-membered heterocycloalkyl group containing at least one nitrogen atom; and
n₁ and n₂ are selected from 0, 1, 2 or 3.

2. The compound of formula (I) or the isotope-labeled compound, or the optical isomer, geometric isomer, tautomer, or isomer mixture thereof, or the pharmaceutically acceptable salt thereof, or the prodrug thereof, or the metabolite thereof of claim 1, wherein:
Y¹ and Y² jointly form a carbonyl group;
Y³ and Y⁴ are each independently selected from hydrogen, halogen, a cyano group, or a C₁-C₆ alkyl group optionally substituted with halogen or the cyano group; or Y³ and Y⁴, together with a carbon atom to which they are attached, form a C₃-C₆ cycloalkyl group or a 3- to 6-membered heterocycloalkyl group optionally substituted with halogen, a C₁-C₃ alkyl group, a C₁-C₃ haloalkyl group, a C₁-C₃ alkoxy group, a C₁-C₃ haloalkoxy group, the amino group, or a C₁-C₃ alkylamino group;
Y⁵ and Y⁶ are each independently selected from hydrogen, halogen, a cyano group, or a C₁-C₆ alkyl group optionally substituted with halogen or the cyano group; or Y⁵ and Y⁶, together with a carbon atom to which they are attached, form a C₃-C₆ cycloalkyl group or a 3- to 6-membered heterocycloalkyl group optionally substituted with halogen, a C₁-C₃ alkyl group, a C₁-C₃ haloalkyl group, a C₁-C₃ alkoxy group, a C₁-C₃ haloalkoxy group, the amino group, or a C₁-C₃ alkylamino group;
optionally, one of Y⁵ and Y⁶, together with one of Y³ and Y⁴ and the carbon atom to which they are attached, form a C₃-C₆ cycloalkyl group or a phenyl group optionally substituted with halogen or a C₁-C₃ alkyl group; and
preferably, the total amount of rings formed by Y¹, Y², Y³, Y⁴, Y⁵ and Y⁶ is not more than one.

3. The compound of formula (I) or the isotope-labeled compound, or the optical isomer, geometric isomer, tautomer, or isomer mixture thereof, or the pharmaceutically acceptable salt thereof, or the prodrug thereof, or the metabolite thereof of claim 1, wherein:
Z² is selected from 5- to 10-membered heteroaryl groups containing 1 to 3 nitrogen atoms, or preferably, Z² is selected from a pyridyl group, a pyrimidinyl group, a pyrazinyl group, a pyridazinyl group, a pyrrolyl group, a pyrazolyl group, an imidazolyl group, a triazolyl group or a quinolinyl group, wherein one or more hydrogen atoms in Z² are each optionally substituted with halogen, a hydroxy group, a cyano group, a methyl group, an ethyl group, a trifluoromethyl group or a methoxy group.

4. The compound of formula (I) or the isotope-labeled compound, or the optical isomer, geometric isomer, tautomer, or isomer mixture thereof, or the pharmaceutically acceptable salt thereof, or the prodrug thereof, or the metabolite thereof of claim 1, wherein:
one of R³ and R⁴ is H, and the other is selected from any one of the following groups:

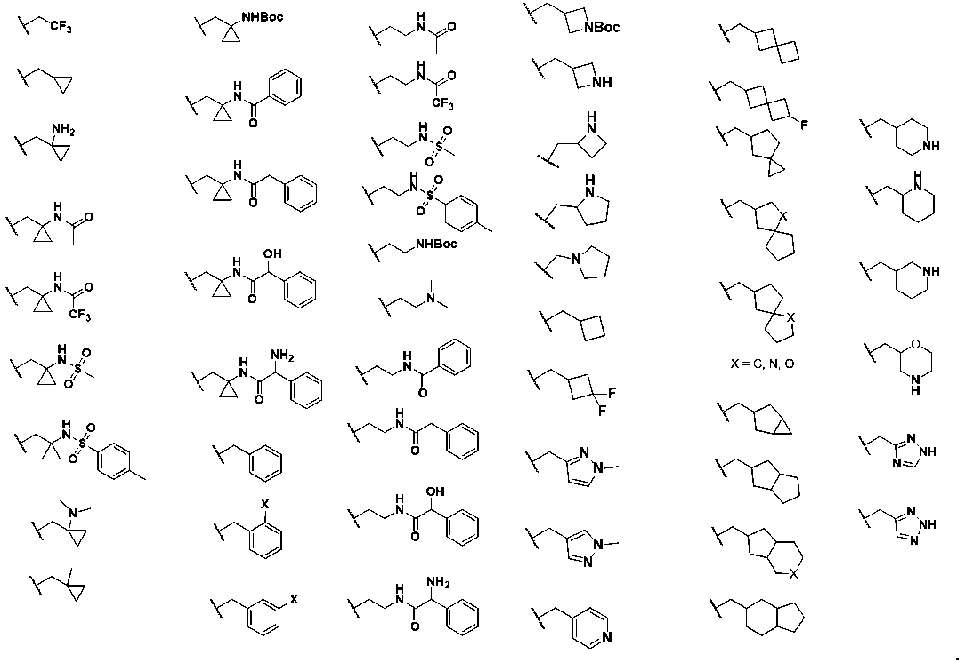

5. The compound of formula (I) or the isotope-labeled compound, or the optical isomer, geometric isomer, tautomer, or isomer mixture thereof, or the pharmaceutically acceptable salt thereof, or the prodrug thereof, or the metabolite thereof of claim 1, wherein:
R¹⁵ is selected from a tetrahydropyrrolinyl group, a hexahydropyridyl group, a morpholinyl group, or a thiamorpholinyl group.

6. The compound of formula (I) or the isotope-labeled compound, or the optical isomer, geometric isomer, tautomer, or isomer mixture thereof, or the pharmaceutically acceptable salt thereof, or the prodrug thereof, or the metabolite thereof of claim 1, wherein:
the halogen is selected from F, Cl, or Br;
the C₁-C₆ alkyl group is selected from a methyl group, an ethyl group, a propyl group, or an isopropyl group;
the C₁-C₆ haloalkyl group is selected from a monofluoromethyl group, a difluoromethyl group, a trifluoromethyl group, a trifluoroethyl group, or a trichloromethyl group;
the C₁-C₆ alkoxy group is selected from a methoxy group, an ethoxy group, or a propoxy group;
the C₁-C₆ haloalkoxy group is selected from a trifluoromethoxy group or a trifluoroethoxy group; and/or
the C₃-C₆ cycloalkyl group is selected from a cyclopropyl group, a cyclobutyl group, a cyclopentyl group, or a cyclohexyl group.

7. The compound of formula (I) or the isotope-labeled compound, or the optical isomer, geometric isomer, tautomer, or isomer mixture thereof, or the pharmaceutically acceptable salt thereof, or the prodrug thereof, or the metabolite thereof of any one of claims 1 to 6, having any one of the following structures:

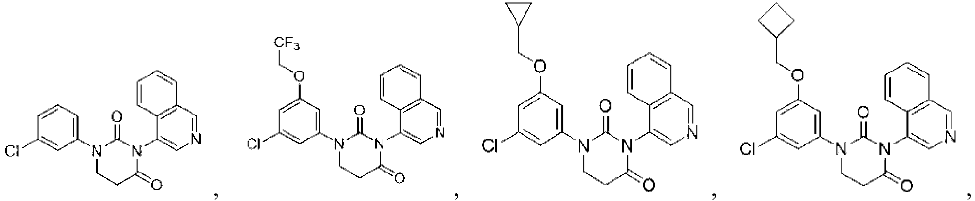

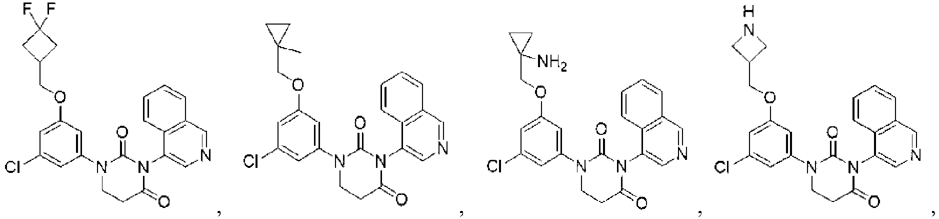

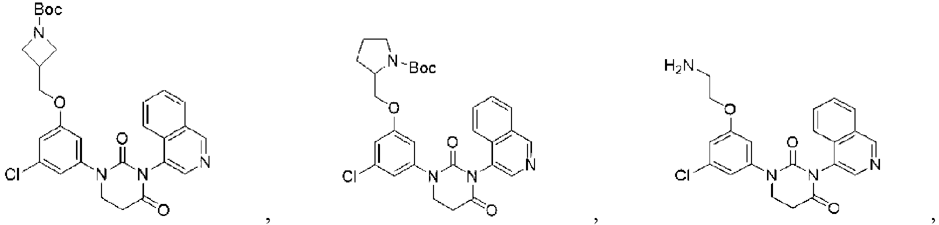

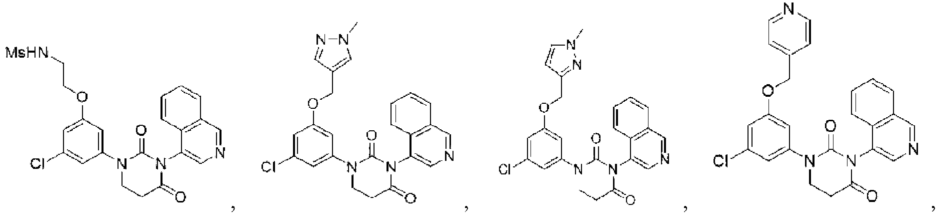

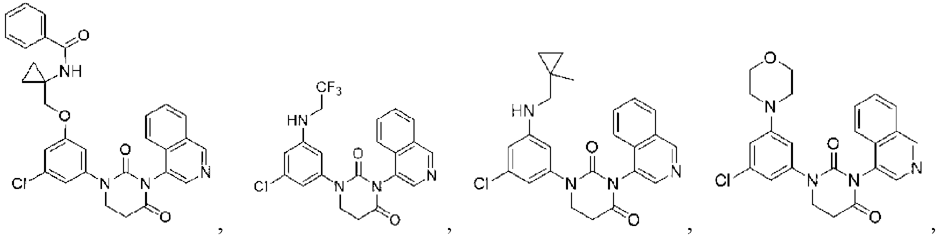

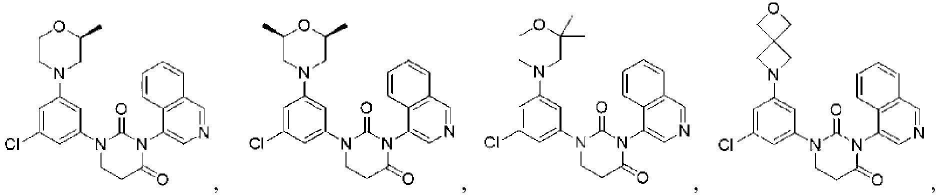

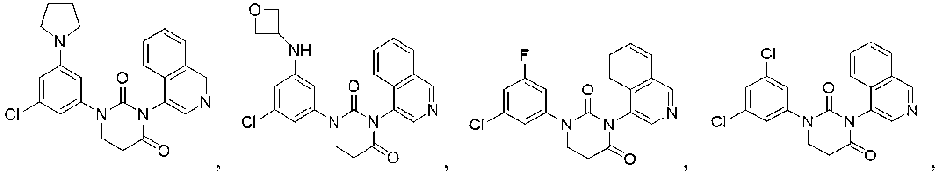

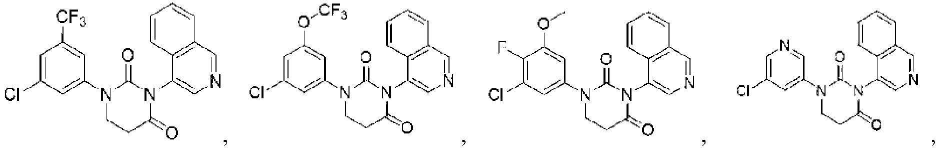

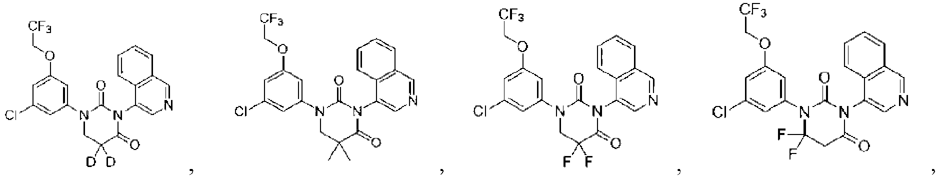

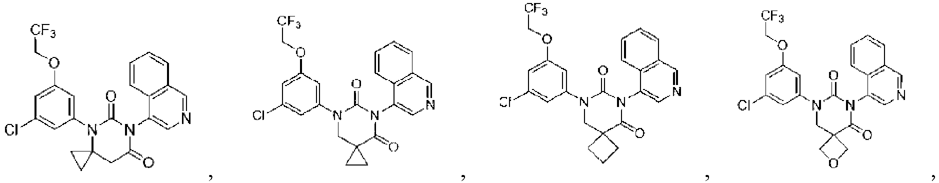

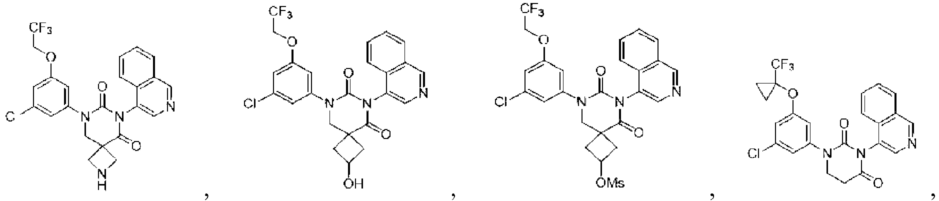

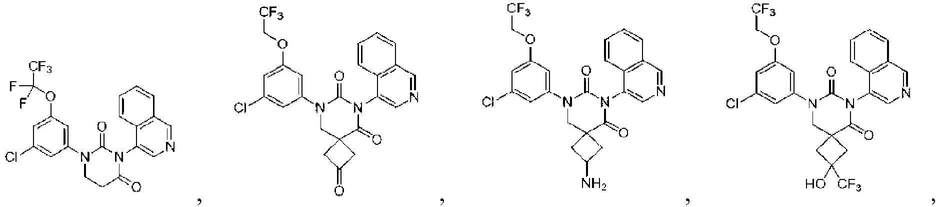

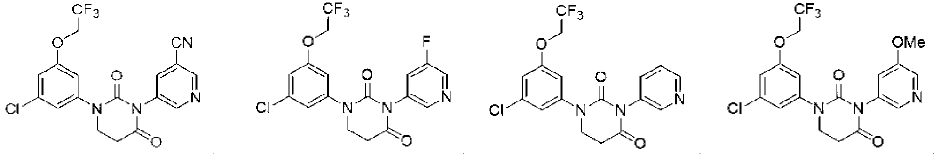

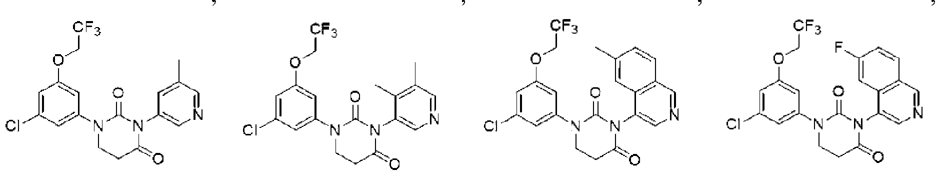

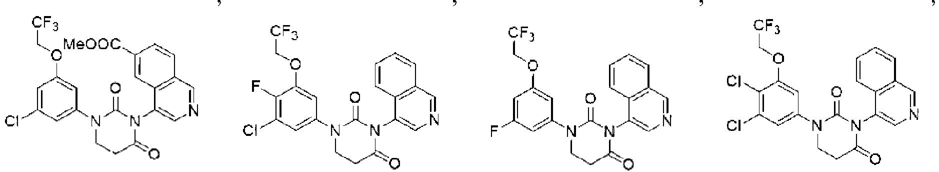

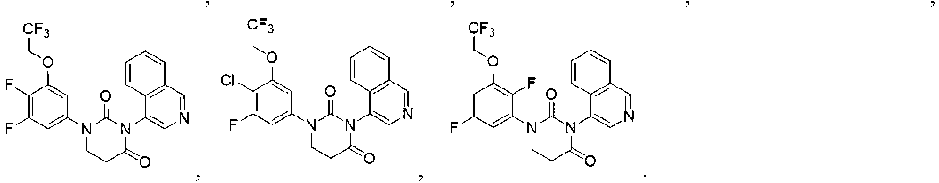

8. A pharmaceutical composition, comprising the compound of formula (I) or the isotope-labeled compound, or the optical isomer, geometric isomer, tautomer, or isomer mixture thereof, or the pharmaceutically acceptable salt thereof, or the prodrug thereof, or the metabolite thereof of any one of claims 1 to 7, and a pharmaceutically acceptable carrier.

9. The pharmaceutical composition of claim 8, wherein a dosage form of the pharmaceutical composition is selected from tablet, granule, powder, syrup, inhalation and injection.

10. Use of the compound of formula (I) or the isotope-labeled compound, or the optical isomer, geometric isomer, tautomer, or isomer mixture thereof, or the pharmaceutically acceptable salt thereof, or the prodrug thereof, or the metabolite thereof of any one of claims 1 to 9 in preparation of a medicament, wherein the medicament is used to treat or prevent a coronavirus infection or a disease or symptom caused by a coronavirus in a subject in need.

11. The use of claim 10, wherein the coronavirus is selected from severe acute respiratory syndrome coronavirus (SARS-CoV), severe acute respiratory syndrome coronavirus 2 (SARS-CoV -2), Middle East respiratory syndrome coronavirus (MERS-CoV), human coronavirus OC43 (HCoV-OC43), murine hepatitis coronavirus (MHV), and a coronavirus that shares more than 85% homology with any one of the foregoing coronaviruses and that has viral activity.

12. The use of claim 10 or 11, wherein the disease or symptom caused by the coronavirus is selected from one or more of a respiratory infection, severe acute respiratory syndrome (SARS), pneumonia (comprising severe pneumonia), gastroenteritis (comprising acute gastroenteritis), cough, fever, shivering, vomiting, headache, chill, shortness of breath, and cytokine storm.
